# EUROPEAN PATENT APPLICATION

(11) **EP 4 286 537 A1**
(43) Date of publication of application: **06.12.2023**
(21) Application number: 22775789.5
(22) Date of filing: 24.03.2022
(51) Int. Cl.: C12Q 1/689, C12N 15/10, C12N 15/11, C12Q 1/686, C12R 1/19, C12R 1/22, C12R 1/445, C12R 1/45

(54) **METHOD AND KIT FOR IDENTIFYING BACTERIA THAT CAUSE SEPSIS**

(30) Priority: 24.03.2021 JP 2021049799
(71) Applicant: Denka Company Limited, Tokyo 103-8338 (JP); Toho University, Tokyo 143-8540 (JP)
(72) Inventor: TATEDA, Kazuhiro, Tokyo 143-8540 (JP); MIYATAKE, Yuya, Tokyo 103-8338 (JP); SAKAI, Kentarou, Tokyo 103-8338 (JP); HIGUCHI, Mizuho, Tokyo 103-8338 (JP); YAMAUCHI, Kazuaki, Tokyo 103-8338 (JP)
(74) Representative: Mewburn Ellis LLP
(86) International application number: PCT/JP2022/014051
(87) International publication number: WO 2022/203008

(57) **Abstract**

Disclosed is a method for identifying causative bacteria of sepsis, the method comprising: a step of performing a PCR method using a sample collected from a subject and a combination of sets of primers each specific to the full-length or a partial region of each of *gyrB* gene of *Escherichia coli, nuc* gene of *Staphylococcus aureus, atlE* gene of *Staphylococcus epidermidis, gyrB* gene of *Klebsiella pneumoniae* and *rpoB* gene of *Enterococcus* spp.; and a step of detecting the presence or absence of amplification of the full-length or the partial region of each of the genes by using a combination of probes each specific to DNA of the full-length or the partial region.

## Description

### Technical Field

The present invention relates to a method and kit for identifying causative bacteria of sepsis.

### Background Art

Sepsis is a secondary symptom resulting from an infection and can be caused by bacterial infections. Severe sepsis leads to respiratory failure, kidney failure, lung failure, and septic shock, increasing a life-threatening risk. For this reason, it is important to quickly identify pathogens of the infections and start treatment.

As to pathogenic factors of sepsis, for example, Patent Literature 1 describes a method for preparing a ligand of adsorbing materials for adsorbing multiple pathogenic factors of sepsis. However, there is no report of a method for identifying a pathogen causing an infection from multiple pathogens, i.e., a causative bacterium for sepsis.

### Citation List

### Patent Literature

Patent Literature 1: U.S. Patent Application Publication No. 2019054227

### Summary of Invention

### Technical Problem

An object of the present invention is to provide a method that can efficiently identify causative bacteria of sepsis and a kit therefor.

### Solution to Problem

The present invention provides, for example, the followings.
[1] A method for identifying causative bacteria of sepsis, the method comprising:
   a step of performing a PCR method using a sample collected from a subject and a combination of sets of primers each specific to the full-length or a partial region of each of the *gyrB* gene of *Escherichia coli,* the *nuc* gene of *Staphylococcus aureus,* the *atlE* gene of *Staphylococcus epidermidis,* the *gyrB* gene of *Klebsiella pneumoniae* and the *rpoB* gene of *Enterococcus* spp. (for example, *Enterococcus faecalis*); and
   a step of detecting the presence or absence of amplification of the full-length or the partial region of each of the genes by using a combination of probes each specific to DNA of the full-length or the partial region.
[2] The method according to [1], wherein
   the set of primers specific to the full-length or the partial region of the *gyrB* gene of *Escherichia coli* comprises: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
   the set of primers specific to the full-length or the partial region of the *nuc* gene of *Staphylococcus aureus* comprises: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
   the set of primers specific to the full-length or the partial region of the *atlE* gene of *Staphylococcus epidermidis* comprises: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
   the set of primers specific to the full-length or the partial region of the *gyrB* gene of *Klebsiella pneumoniae* comprises: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15,
   the set of primers specific to the full-length or the partial region of the *rpoB* gene of *Enterococcus* spp. comprises: one or more primers selected from a primer containing the nucleotide sequence of SEQ ID NO: 26 or a nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29.
[3] The method according to [1] or [2], wherein
   the DNA of the full-length or the partial region of the *gyrB* gene of *Escherichia coli* comprises the nucleotide sequence of SEQ ID NO: 51 or a nucleotide sequence having a sequence identity of 90% or more with the nucleotide sequence of SEQ ID NO: 51,
   the DNA of the full-length or the partial region of the *nuc* gene of *Staphylococcus aureus* comprises the nucleotide sequence of SEQ ID NO: 61 or a nucleotide sequence having a sequence identity of 90% or more with the nucleotide sequence of SEQ ID NO: 61,
   the DNA of the full-length or the partial region of the *atlE* gene of *Staphylococcus epidermidis* comprises the nucleotide sequence of SEQ ID NO: 63 or a nucleotide sequence having a sequence identity of 90% or more with the nucleotide sequence of SEQ ID NO: 63,
   the DNA of the full-length or the partial region of the *gyrB* gene of *Klebsiella pneumoniae* comprises the nucleotide sequence of SEQ ID NO: 53 or a nucleotide sequence having a sequence identity of 90% or more with the nucleotide sequence of SEQ ID NO: 53, and
   the DNA of the full-length or the partial region of the *rpoB* gene of *Enterococcus* spp. comprises the nucleotide sequence of SEQ ID NO: 59 or a nucleotide sequence having a sequence identity of 90% or more with the nucleotide sequence of SEQ ID NO: 59.
[4] The method according to [3], wherein the probes each hybridize with DNA of the full-length or the partial region under stringent conditions.
[5] The method according to any of [1] to [4], wherein
   the probe specific to DNA of the full-length or the partial region of the *gyrB* gene of *Escherichia coli* comprises the nucleotide sequence of SEQ ID NO: 121 or a nucleotide sequence having a sequence identity of 90% or more with the nucleotide sequence of SEQ ID NO: 121,
   the probe specific to DNA of the full-length or the partial region of the *nuc* gene of *Staphylococcus aureus* comprises the nucleotide sequence of SEQ ID NO: 134 or a nucleotide sequence having a sequence identity of 90% or more with the nucleotide sequence of SEQ ID NO: 134,
   the probe specific to DNA of the full-length or the partial region of the *atlE* gene of *Staphylococcus epidermidis* comprises the nucleotide sequence of SEQ ID NO: 136 or a nucleotide sequence having a sequence identity of 90% or more with the nucleotide sequence of SEQ ID NO: 136,
   the probe specific to DNA of the full-length or the partial region of the *gyrB* gene of *Klebsiella pneumoniae* comprises the nucleotide sequence of SEQ ID NO: 124 or a nucleotide sequence having a sequence identity of 90% or more with the nucleotide sequence of SEQ ID NO: 124, and
   the probe specific to DNA of the full-length or the partial region of the *rpoB* gene of *Enterococcus* spp. is selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof.
[6] The method according to any of [1] to [5], wherein the probes contain different identifiers or are bound to the different identifiers on a solid support.
[7] The method according to any of [1] to [6], wherein the DNA amplified by the PCR method contains a label.
[8] The method according to any one of [1] to [7], wherein the sample collected from a subject is blood.
[9] The method according to [8], further comprising a step of culturing the blood.
[10] A kit for identifying causative bacteria of sepsis, the kit comprising:
   a first reagent containing sets of primers for PCR each specific to the full-length or a partial region of each of the *gyrB* gene of *Escherichia coli,* the nuc gene of *Staphylococcus aureus,* the *atlE* gene of *Staphylococcus epidermidis,* the *gyrB* gene of *Klebsiella pneumoniae* and the *rpoB* gene of *Enterococcus* spp.; and
   a second reagent containing a combination of probes each specific to DNA of the full-length or the partial region of each of the genes.
[11] The kit according to [10], wherein
   the set of primers specific to the full-length or the partial region of the *gyrB* gene of *Escherichia coli* comprises: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
   the set of primers specific to the full-length or the partial region of the *nuc* gene of *Staphylococcus aureus* comprises: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
   the set of primers specific to the full-length or the partial region of the *atlE* gene of *Staphylococcus epidermidis* comprises: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
   the set of primers specific to the full-length or the partial region of the *gyrB* gene of *Klebsiella pneumoniae* comprises: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15, and
   the set of primers specific to the full-length or the partial region of the *rpoB* gene of *Enterococcus* spp. comprises: one or more primers selected from a primer containing the nucleotide sequence of SEQ ID NO: 26 or a nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29.
[12] The kit according to [10] or [11], wherein
   the DNA of the full-length or the partial region of the *gyrB* gene of *Escherichia coli* comprises the nucleotide sequence of SEQ ID NO: 51 or a nucleotide sequence having a sequence identity of 90% or more with the nucleotide sequence of SEQ ID NO: 51,
   the DNA of the full-length or the partial region of the *nuc* gene of *Staphylococcus aureus* comprises the nucleotide sequence of SEQ ID NO: 61 or a nucleotide sequence having a sequence identity of 90% or more with the nucleotide sequence of SEQ ID NO: 61,
   the DNA of the full-length or the partial region of the *atlE* gene of *Staphylococcus epidermidis* comprises the nucleotide sequence of SEQ ID NO: 63 or a nucleotide sequence having a sequence identity of 90% or more with the nucleotide sequence of SEQ ID NO: 63,
   the DNA of the full-length or the partial region of the *gyrB* gene of *Klebsiella pneumoniae* comprises the nucleotide sequence of SEQ ID NO: 53 or a nucleotide sequence having a sequence identity of 90% or more with the nucleotide sequence of SEQ ID NO: 53, and
   the DNA of the full-length or the partial region of the *rpoB* gene of *Enterococcus* spp. comprises the nucleotide sequence of SEQ ID NO: 59 or a nucleotide sequence having a sequence identity of 90% or more with the nucleotide sequence of SEQ ID NO: 59.
[13] The kit according to [12], wherein the probes each hybridize with DNA of the full-length or the partial region under stringent conditions.
[14] The kit according to any of [10] to [13], wherein
   the probe specific to DNA of the full-length or the partial region of the *gyrB* gene of *Escherichia coli* comprises the nucleotide sequence of SEQ ID NO: 121 or a nucleotide sequence having a sequence identity of 90% or more with the nucleotide sequence of SEQ ID NO: 121,
   the probe specific to DNA of the full-length or the partial region of the *nuc* gene of *Staphylococcus aureus* comprises the nucleotide sequence of SEQ ID NO: 134 or a nucleotide sequence having a sequence identity of 90% or more with the nucleotide sequence of SEQ ID NO: 134,
   the probe specific to DNA of the full-length or the partial region of the *atlE* gene of *Staphylococcus epidermidis* comprises the nucleotide sequence of SEQ ID NO: 136 or a nucleotide sequence having a sequence identity of 90% or more with the nucleotide sequence of SEQ ID NO: 136,
   the probe specific to DNA of the full-length or the partial region of the *gyrB* gene of *Klebsiella pneumoniae* comprises the nucleotide sequence of SEQ ID NO: 124 or a nucleotide sequence having a sequence identity of 90% or more with the nucleotide sequence of SEQ ID NO: 124, and
   the probe specific to DNA of the full-length or the partial region of the *rpoB* gene of *Enterococcus* spp. is selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof.
[15] The kit according to any of [10] to [14], wherein the probes contained in the second reagent contain different identifiers or are bound to the different identifiers on a solid support.
[16] The kit according to any of [10] to [15], wherein the sets of primers are designed such that DNAs to be amplified by the PCR method using the sets of primers contain labels or the kit further comprises a third reagent for labeling the DNAs to be amplified.

### Advantageous Effects of Invention

According to the present invention, it is possible to simultaneously examine a plurality of causative bacteria of sepsis and efficiently identify causative bacteria of sepsis. Since causative bacteria of sepsis can be efficiently identified, treatment can be quickly chosen.

### Brief Description of Drawings

[Figure 1] The figure is a graph showing the results (amplification curves) of real-time PCR using primer set 1 and primer set 2 in Example 1.
[Figure 2] The figure is a graph showing the results (fluorescence values detected) that the PCR product obtained by using primer set 1 was detected by two types of probes (*E. coli*_531P16 and *E. coli*_526P15) in Example 1.
[Figure 3] The figure is a graph showing the results (amplification curves) that real-time PCR was performed using primer set 1 in Example 2.
[Figure 4] The figure is a graph showing the results (amplification curves) that real-time PCR was performed using primer set 2 in Example 2.
[Figure 5] The figure is a graph showing the results (amplification curves) that real-time PCR was performed using primer set 3 in Example 2.
[Figure 6] The figure is a graph showing the results (fluorescence values detected) that the PCR products obtained by using primer set 1 with *Citrobacter freundii* as a template were detected by three types of probes (C.spp_24P17, C.spp_55P15Y, and C.spp_65P19) in Example 2.

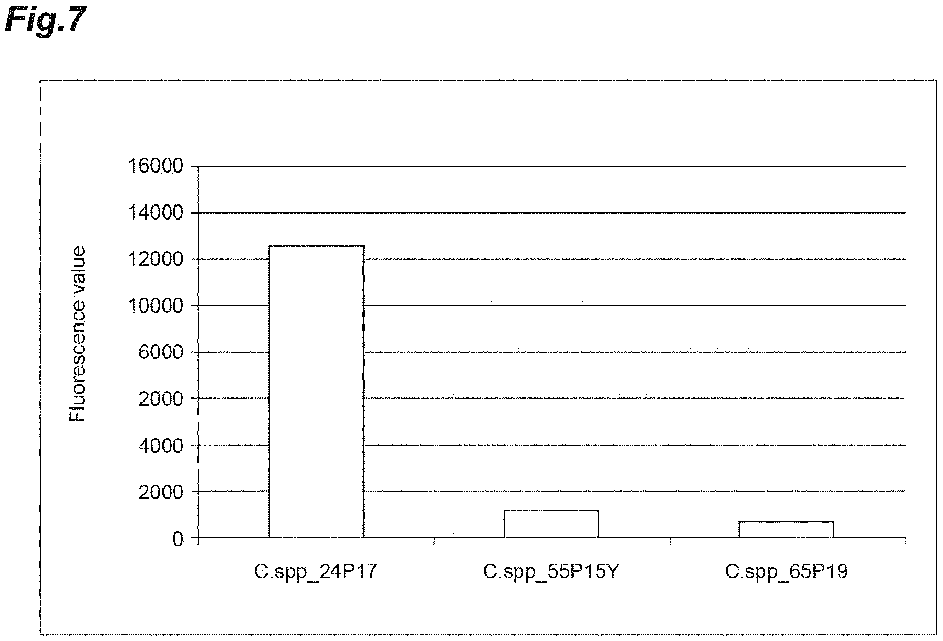

[Figure 7] The figure is a graph showing the results (fluorescence values detected) that the PCR products obtained by using primer set 1 with *Citrobacter koseri* as a template were detected by three types of probes (C.spp_24P17, C.spp_55P15Y, and C.spp_65P19) in Example 2.
[Figure 8] The figure is a graph showing the results (fluorescence values detected) that the reactivity and specificity of probes to PCR products of bacterial species were evaluated in Example 3.
[Figure 9] The figure is a graph showing the results (fluorescence values detected) that the reactivity and specificity of probes to PCR products of bacterial species were evaluated in Example 3.
[Figure 10] The figure is a graph showing the results (fluorescence values detected) that the reactivity and specificity of probes to PCR products of bacterial species were evaluated in Example 3.
[Figure 11] The figure is a graph showing the results (fluorescence values detected) that the reactivity and specificity of probes to PCR products of antimicrobial resistance genes were evaluated in Example 3.
[Figure 12] The figure is a graph showing the results (fluorescence values detected) that the reactivity and specificity of probes to PCR products of antimicrobial resistance genes were evaluated in Example 3.

### Description of Embodiments

Now, embodiments for carrying out the present invention will be more specifically described. Note that, the present invention is not limited to the following embodiments.

### identification method>

The present invention provides, as an embodiment, a method for identifying causative bacteria of sepsis comprising:
a step of performing a PCR method using a sample collected from a subject and a combination of sets of primers each specific to the full-length or a partial region of each of the *gyrB* gene of *Escherichia coli,* the *nuc* gene of *Staphylococcus aureus,* the *atlE* gene of *Staphylococcus epidermidis,* the *gyrB* gene of *Klebsiella pneumoniae* and the *rpoB* gene of *Enterococcus* spp.; and
a step of detecting the presence or absence of amplification of the full-length or the partial region of each of the genes by using a combination of probes each specific to DNA of the full-length or the partial region.

### [Step of performing PCR method]

In an embodiment, the step of performing a PCR method is a step of performing a PCR method using a sample collected from a subject and a combination of sets of primers each specific to the full-length or a partial region of each of the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp.

The subject from which a sample is collected may be, for example, a subject having sepsis or a subject suspected of having sepsis, or a subject having a bacterial infection or a subject suspected of having a bacterial infection. A sample is acceptable as long as it contains, for example, DNA of causative bacteria of sepsis or it is suspected of containing DNA of causative bacteria of sepsis. A subject is acceptable as long as it is, for example, a mammal, a human or a non-human mammal.

The sample collected from a subject may be, for example, bodily fluids such as blood, saliva, urine, and lymph. It is preferable that the sample be blood.

The sample collected from a subject may be, if necessary, cultured, and then, put in use. For example, the method according to the embodiment may further include a step of culturing blood. A method for culturing blood can be performed by a method known in the art.

The step of performing a PCR method may further include a step of preparing a template from a sample collected from a subject.

Examples of the template include a DNA extract. A method for extracting DNA from a sample collected from a subject can be performed by a method known in the art.

In the specification, the set of primers specific to the full-length or a partial region of each of the genes refers to a set of primers specifically binding to the full-length or a partial region of each of the genes as a target and for use in amplifying DNA of the region determined as a target by a polymerase chain reaction (PCR). The set of primers includes at least one type of forward primer and at least one type of reverse primer. The set of primers may be, for example, a combination of a plurality of forward primers and a single reverse primer, a combination of a single forward primer and a plurality of reverse primers, or a combination of a plurality of forward primers and a plurality of reverse primers. Hereinafter, the region determined as a target will be sometimes referred to as, for example, a "target region".

The sequence of each gene determined as a target sequence is available from a public database in which sequence information is registered, such as GenBank distributed by the United States National Center For Biotechnology Information (NCBI). Information such as GenBank IDs of the genes set forth in the specification is listed in Table 1 and Table 2. In the specification, the name of each gene, in principle, represents not only a gene having a predetermined sequence but also, for example, a gene having a naturally-occurring single nucleotide polymorphism.

Examples of the names of strains and GenBank IDs of bacteria set forth in the specification are shown below. The names of genes are examples of target genes and antimicrobial resistance genes set forth in the specification.

**[Table 1]**

| Bacterial species | Name of strain | GenBank ID | Name of target gene |
|---|---|---|---|
| *Acinetobacter baumannii* | strainAb30 | NZ_CP009257.1 | rpoB |
| *Acinetobacter baumannii* | strainNF43 | KR922891.1 | 16S-23S ITS |
| *Citrobacter koseri* | strain_AR_0025 | CP026697.1 | ompA |
| *Enterobacter cloacae* | strain NCTC13405 | NZ_UGIT00000000.1 | rpoS |
| *Escherichia coli* | strain113 | CP028680.1 | gyrB |
| *Klebsiella aerogenes* | strain Ea11791 | QKNB01000001.1 | gyrB |
| *Klebsiella pneumoniae* | strain KPNIH39 | CP014762.1 | gyrB |
| *Klebsiella oxytoca* | strain NCTC11691 | NZ_UGJS01000001.1 | pehX |
| *Klebsiella variicola* | strain MGH-76aedYL | NZ_KK737663.1 | gyrB |
| *Pseudomonas aeruginosa* | strainNCTC13621 | CAADJP010000013.1 | oprL |
| *Proteus mirabilis* | strain ATCC7002 | KN150749.1 | rpoB |
| *Serratia marcescens* | strain 2880STDY5682934 | NZ_FCJR00000000.1 | hasA |
| *Enterococcus faecalis* | strain B15321 | PZZF01000001.1 | rpoB |
| *Listeria monocytogenes* | strain ATCC19118 | AF532293.1 | iap p60 |
| *Staphylococcus aureus* | strainGD1539 | CP019594.2 | nuc |
| *Staphylococcus argenteus* | strain 3688STDY6125066 | FQMM01000001.1 | nuc |
| *Staphylococcus epidermidis* | strain AU23 | LNUS01000001.1 | atIE |
| *Staphylococcus aureus* | KG-18 | AP019543.1 | tuf |
| *Streptococcus pneumoniae* | strain GPSC19 | NZ_LR216069.1 | rnpB |
| | substr. ST1955 | | |
| *Streptococcus pneumoniae* | strain GPSC108 | NZ_LR216028.1 | xisco |
| | substr. ST9487 | | |
| *Streptococcus pyogenes* | strain MGAS7888 | CP031640.1 | gyrB |

**[Table 2]**

| Bacterial species | Name of strain | GenBank ID | Name of antimicrobial resistance gene |
|---|---|---|---|
| *Klebsiella pneumoniae* | VAKPC278_plasmid_ pVAKPC278-138 | NZ_JMSW01000002.1 | Beta-lactamase_KP C |
| *Klebsiella pneumoniae* | strain_JNM10C3 | NZ_CP03D876.1 | Beta-lactamase_N DM |
| *Pseudomonas aeruginosa* | strain197018 | MN510335.1 | Beta-lactamase_IM P85 |
| *Citrobacter freundii* | str._E2614 | NZ_LS992177.1 | Beta-lactamase_VI M |
| *Salmonella enterica* | LC0541/17 | NZ_CP030839.1 | Beta-lactamase_CT X-M1 |
| *Salmonella enterica* | CAS-5 | NG_048968.1 | Beta-lactamase_CT X-M2 |
| *Escherichia coli* | 785-D | NG_049043.1 | Beta-lactamase_CT X-M9 |
| *Escherichia coli* | | AF518567.2 | Beta-lactamase_CT X-M25 |
| *Citrobacter amalonaticus* | Rio-2 | NG_049032.1 | Beta-lactamase_CT X-M8 |
| *Acinetobacter baumannii* | strainTG60155 | NZ_CP036284.1 | Beta-lactamase_OX A-23 |
| *Acinetobacter baumannii* | BAL_204 | KT946773.1 | Beta-lactamase_OX A-40 |
| *Klebsiella pneumoniae* | plasmidpKPoxa-48N2 | NC_021502.1 | Beta-lactamase_OX A-48 |
| *Acinetobacter haemolyticus* | strainTJR01plasmid pAHTJR1 | NZ_CP038010.1 | Beta-lactamase_OX A-58 |
| *Staphylococcus aureus* | isolateTMHS-SA-3125 | KU194302.1 | mecA |
| *Enterococcus faecalis* | plasmidpWZ7140 | NC_019284.1 | vanA |
| *Enterococcus faecium* | strain10_1922 | KT003978.1 | vanB |

A set of specific primers (forward primer and reverse primer) for amplifying the full-length or a partial region of each of the genes as a target sequence can be designed based on the sequence of a target sequence. The primers can be synthesized in accordance with a method known to those skilled in the art. The primers may be, for example, an oligonucleotide of 10 to 50 bases in length, an oligonucleotide of 15 to 30 bases in length or an oligonucleotide of 17 to 25 bases in length. The primers are not necessary to reflect an accurate sequence of a target sequence but necessary to have complementarity sufficient to hybridize with a template and initiate DNA synthesis.

In the specification, the primer may contain a predetermined nucleotide sequence or a nucleotide sequence having a sequence identity of 85% or more, 90% or more, 91% or more, 92% or more, 93% or more, 94% or more, 95% or more, 96% or more, 97% or more, 98% or more, or 99% or more with the predetermined nucleotide sequence. The primer may consist of a predetermined nucleotide sequence or a nucleotide sequence having a sequence identity of 85% or more, 90% or more, 91% or more, 92% or more, 93% or more, 94% or more, 95% or more, 96% or more, 97% or more, 98% or more, or 99% or more with the predetermined nucleotide sequence. The primer may contain a predetermined nucleotide sequence or a nucleotide sequence having addition or deletion of 0 to 5, 1 to 4, 1 to 3, 1 or 2, or 1 nucleotide at the 3' end or 5' end of the predetermined nucleotide sequence.

In the specification, a single-letter code of a nucleotide base is used in accordance with the notation of bases defined in the International Union of Pure and Applied Chemistry (IUPAC) listed in the following Table.

**[Table 3]**

| Base | Cord |
|---|---|
| Adenine | A |
| Cytosine | C |
| Guanine | G |
| Thymine | T |
| Uridine | U |
| Adenine or cytosine | M |
| Adenine or guanine | R |
| Adenine or thymine | w |
| Cytosine or guanine | S |
| Cytosine or thymine | Y |
| Guanine or thymine | K |

A primer may be constituted of bases A, G, C and T or analogs or degenerate bases (M, R, W, S, Y and K). The gene of bacterium can be more suitably amplified by inserting a degenerate base to a predetermined position of the sequence at which the base varies depending on the bacterial strain.

A primer may have a label detectable by a spectroscopic means, a photochemical means, a biochemical means, an immunochemical means, or a chemical means. Examples of the detectable label include biotin that is detected by labeled avidin (for example, fluorescently labeled streptavidin), hapten, fluorescent dyes (for example, fluorescein, Texas red and rhodamine), electron density reagents, enzymes (for example, horseradish peroxidase and alkaline phosphatase) and radioisotopes (for example, ³²P, ³H, ¹⁴C and ¹²⁵I). The label, for example, may be attached to the 5' end, 3' end, or the internal portion of a primer or the label may be attached via a linker. For example, the 5' end of a primer may be labeled with biotin modification, in which case, the 5' end of a primer may be modified with biotin via a linker.

In the step of performing a PCR method, amplified DNA containing a label can be obtained by using a primer having a label. The label can be used also for capturing a primer to immobilize a primer or amplified DNA onto a solid support.

In the embodiment, for simultaneously performing a PCR method using a combination of sets of primers each specific to the full-length or a partial region of each of a plurality of genes, the sets of primers are designed so as to perform an amplification stage of individual genes under analogous or equivalent amplification conditions. If DNA of the full-length or a partial region of a gene targeted by a primer is contained in a sample, the region is amplified.

PCR using a combination of sets of primers each specific to the full-length or a partial region of each of a plurality of genes can be performed by a method known to those skilled in the art. In the PCR, at first, there usually is a denaturation step, and then, a PCR amplification cycle follows. In each PCR amplification cycle, a double-stranded target sequence is denatured (denaturation step); a primer is allowed to be annealed to each strand denatured (annealing step); and the primer is allowed to extend by the action of DNA polymerase (extension step).

Examples of the method for amplifying various genomic regions having different sequences in a single PCR reaction system include a multiplex polymerase chain reaction (Multiplex PCR). The Multiplex PCR refers to a method of simultaneously examining a single sample in a multiplex form in which a plurality of primers for amplifying different target nucleic acids are used in combination.

In the embodiment, for amplifying the regions of the different genes simultaneously in a single PCR reaction system, the sets of primers each specific to the full-length or a partial region of each of the genes are collectively used. For example, a sample collected from a subject is brought into contact with a mixture of sets of primers in conditions suitable for nucleic acid amplification, and then, the sets of primers can each specifically bind to the full-length or a partial region of a bacterial gene, or a bacterial gene and an antimicrobial resistance gene, thereby amplifying the region by DNA polymerase.

A PCR method is performed using a combination of sets of primers targeting a bacterial gene, or a bacterial gene and an antimicrobial resistance gene contained or suspected of being contained in a sample collected from a subject. As a result, a mixture of amplified products can be obtained. If a targeted gene is contained in the sample, DNA obtained by amplifying the full-length or part of a bacterial gene, or bacterial gene and antimicrobial resistance gene will be contained in the amplification reaction mixture.

In the step of performing a PCR method, a mixture containing, for example, a template obtained from a sample and a combination of sets of primers as mentioned above, can be referred to as a reaction mixture.

The reaction mixture may contain a PCR standard reagent. Examples of the PCR standard reagent include Multiplex PCR Assay Kit Ver. 2 (Takara Bio Inc.). The reaction mixture may further contain one or more selected from the group consisting of DNA polymerase, deoxynucleoside triphosphate (dNTP), a magnesium ion, one or more salts, Tris buffer (Tris-HCL), EDTA, glycerol, and a pH buffer.

Examples of the DNA polymerase include taq (*Thermus aquaticus*) polymerase and pfu (*Pyrococcus furiosus*) polymerase.

Examples of the one or more salts include potassium chloride and magnesium chloride. The salt concentration of a buffer for use in PCR may be, for example, 1 mM to 200 mM. If potassium chloride is used, the salt concentration may be 25 mM to 175 mM. If magnesium chloride is used, the salt concentration may be 1 to 4 mM.

Examples of the pH buffer include Tris-pH buffer. The pH value of the pH buffer may be, for example, 7.5 to 9.0 or 8.0 to 9.0.

The concentration of each of the primers (final concentration of the primer in a PCR reaction solution) for use in PCR may be, for example, 0.01 µM to 3 µM, 0.1 µM to 0.3 µM, 0.05 µM to 2.5 µM, or 0.1 µM to 0.4 µM.

The denaturation step in PCR may be performed, for example, at 90°C to 95°C for 30 to 60 seconds or 94°C for 30 seconds.

The annealing step may be performed, for example, at 58 to 64°C for 30 to 65 seconds and 90°C to 95°C for 20 to 40 seconds; at 60 to 62°C for 50 to 60 seconds and 92°C to 95°C for 25 to 35 seconds; or 60°C for 60 seconds and 94°C for 30 seconds. The number of annealing cycles may be, for example, 25 to 50, 30 to 40, or 30.

The extension step may be performed, for example, at 70°C to 75°C for 100 to 700 seconds, 70°C to 75°C for 400 to 700 seconds, or 72°C for 600 seconds.

It is preferable that the combination of the sets of primers to be used in the embodiment be designed such that the denaturation step, annealing step, and extension step of a PCR method can be performed under predetermined temperature cycle conditions.

In the step of performing a PCR method, a combination of sets of primers each specific to the full-length or a partial region of each of the *gyrB* gene of *Escherichia coli,* the *nuc* gene of *Staphylococcus aureus,* the *atlE* gene of *Staphylococcus epidermidis,* the *gyrB* gene of *Klebsiella pneumoniae* and the *rpoB* gene of *Enterococcus* spp. (for example, *Enterococcus faecalis*) is used.

In the step of performing a PCR method, a set of primers specific to the full-length or a partial region of a gene other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp. may be further used.

In the step of performing a PCR method, for example, sets of primers specific to the full-length or a partial region of at least one gene selected from the group consisting of the *rpoB* gene of *Acinetobacter* spp. (for example, *Acinetobacter baumannii*), the *16S-23S ITS* gene of *Acinetobacter baumannii,* the *ompA* gene of *Citrobacter* spp. (for example, *Citrobacter koseri, Citrobacter freundii*), the *rpoS* gene of *Enterobacter* spp. (for example, *Enterobacter cloacae),* the *gyrB* gene of *Klebsiella aerogenes,* the *pehX* gene of *Klebsiella oxytoca,* the *gyrB* gene of *Klebsiella variicola,* the *oprL* gene of *Pseudomonas aeruginosa,* the *rpoB* gene of *Proteus* spp. (for example, *Proteus mirabilis),* the *hasA* gene of *Serratia marcescens,* the *iap p60* gene of *Listeria* spp. (for example, *Listeria monocytogenes),* the nuc gene of *Staphylococcus argenteus,* the *tuf* gene of *Staphylococcus* spp. (for example, *Staphylococcus aureus),* the *rnpB* gene of *Streptococcus* spp. (for example, *Streptococcus pneumoniae),* the *xisco* gene of *Streptococcus pneumoniae,* and the *gyrB* gene *Streptococcus pyogenes* may be further used.

In the step of performing a PCR method, for example, sets of primers specific to the full-length or a partial region of one or more genes selected from the group consisting of antimicrobial resistance genes: the *beta-lactamase_KPC* gene of *Klebsiella pneumoniae,* the *beta-lactamase_NDM* gene of *Klebsiella pneumoniae,* the *beta-lactamase_IMP85* gene of *Pseudomonas aeruginosa,* the *beta-lactamase_VIM* gene of *Citrobacter_freundii,* the *beta-lactamase_CTX-M1* gene of *Salmonella enterica,* the *beta-lactamase_CTX-M2* gene of *Salmonella enterica,* the *beta-lactamase_CTX-M8* gene of *Citrobacter amalonaticus,* the *beta-lactamase_CTX-M25* gene of *Escherichia coli,* the *beta-lactamase_CTX-M9* gene of *Escherichia coli,* the *beta-lactamase_OXA-23* gene of *Acinetobacter baumannii,* the *beta-lactamase_OXA-40* gene of *Acinetobacter baumannii,* the *beta-lactamase_OXA-48* gene *of Klebsiella pneumoniae,* the *beta-lactamase_OXA-58* gene *of Acinetobacter haemolyticus,* the *mecA* gene of *Staphylococcus aureus,* the *vanA* gene of *Enterococcus faecalis,* and the *vanB* gene of *Enterococcus faecium* may be further used.

In the step of performing a PCR method, for example, sets of primers specific to the full-length or a partial region of one or more genes selected from the group consisting of the *rpoB* gene of *Acinetobacter* spp., the *16S-23S ITS* gene of *Acinetobacter baumannii,* the *ompA* gene of *Citrobacter* spp. (for example, *Citrobacter koseri, Citrobacter freundii*), the *rpoS* gene of *Enterobacter* spp. (for example, *Enterobacter cloacae),* the *gyrB* gene of *Klebsiella aerogenes, the pehX* gene of *Klebsiella oxytoca,* the *gyrB* gene of *Klebsiella variicola,* the *oprL* gene of *Pseudomonas aeruginosa,* the *rpoB* gene of *Proteus* spp. (for example, *Proteus mirabilis),* the *hasA* gene of *Serratia marcescens,* the *iap p60* gene of *Listeria* spp., the *nuc* gene of *Staphylococcus argenteus,* the *tuf* gene of *Staphylococcus* spp., the *rnpB* gene of *Streptococcus* spp., the *xisco* gene of *Streptococcus pneumoniae,* the *gyrB* gene of *Streptococcus pyogenes,* the *beta-lactamase_KPC* gene of *Klebsiella pneumoniae,* the *beta-lactamase_NDM* gene of *Klebsiella pneumoniae,* the *beta-lactamase_IMP85* gene of *Pseudomonas aeruginosa,* the *beta-lactamase_VIM* gene of *Citrobacter freundii,* the *beta-lactamase_CTX-M1* gene of *Salmonella enterica,* the *beta-lactamase_CTX-M2* gene of *Salmonella enterica,* the *beta-lactamase_CTX-M8* gene of *Citrobacter amalonaticus,* the *beta-lactamase*_*CTX-M25* gene of *Escherichia coli,* the *beta-lactamase_CTX-M9* gene of *Escherichia coli,* the *beta-lactamase_OXA-23* gene of *Acinetobacter baumannii,* the *beta-lactamase_OXA-40* gene of *Acinetobacter baumannii,* the *beta-lactamase_OXA-48* gene of *Klebsiella pneumoniae,* the *beta-lactamase_OXA-58* gene of *Acinetobacter haemolyticus,* the *mecA* gene of *Staphylococcus aureus,* the *vanA* gene of *Enterococcus faecalis,* and the *vanB* gene of *Enterococcus faecium* may be used.

The bacteria or genes selected from the group of the plurality of genes as mentioned above may be, for example, 2 types or more, 3 types or more, 5 types or more, 7 types or more, 10 types or more, 15 types or more, 20 types or more, 25 types or more, 28 types or more, 30 types or more, or 32 types or more. The bacteria or genes selected from the group of the plurality of genes as mentioned above may be, for example, 37 types or less, 34 types or less, 21 types or less, 16 types or less, or 11 types or less. The bacteria or genes selected from the group of the plurality of genes as mentioned above may be, for example, 2 to 40 types, 5 to 37 types, 10 to 37 types, or 15 to 25 types.

Further use of the sets of primers as mentioned above makes it possible to examine a larger number of causative bacteria of sepsis and antimicrobial resistance genes in combination. Further use of a set of primers for antimicrobial resistance genes makes it possible to identify causative bacteria of sepsis and simultaneously the presence or absence of antimicrobial resistance genes of the bacteria, owing to which, treatment can be quickly chosen. In addition, in consideration of the presence or absence of the resistance of causative bacteria to therapeutic drugs, a suitable treatment and a therapeutic strategy can be selected and determined.

In the step of performing a PCR method, the set of primers specific to the full-length or a partial region of the *gyrB* gene of *Escherichia coli* may contain: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
the set of primers specific to the full-length or a partial region of the *nuc* gene of *Staphylococcus aureus* may contain: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
the set of primers specific to the full-length or a partial region of the *atlE* gene of *Staphylococcus epidermidis* may contain: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
the set of primers specific to the full-length or a partial region of the *gyrB* gene of *Klebsiella pneumoniae* may contain: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15, and
the set of primers specific to the full-length or a partial region of the *rpoB* gene of *Enterococcus* spp. may contain: one or more primers selected from a primer containing the nucleotide sequence of SEQ ID NO: 26 or the nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29.

The use of the sets of primers reduces the interaction among the primers and improves specificity and reactivity when each region is amplified. In addition, the use of the sets of primers improves the amplification efficiency when a PCR method is performed in conditions equivalent in, e.g., temperature and reagent.

In the case where a set of primers specific to the full-length or a partial region of a gene other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp., is further used,
the set of primers specific to the full-length or a partial region of the *rpoB* gene of *Acinetobacter* spp. may contain: a primer containing the nucleotide sequence of SEQ ID NO: 1 or a nucleotide sequence having a sequence identity of 90% or more with the nucleotide sequence of SEQ ID NO: 1; and a primer containing the nucleotide sequence of SEQ ID NO: 2 or a nucleotide sequence having a sequence identity of 90% or more with the nucleotide sequence of SEQ ID NO: 2,
the set of primers specific to the full-length or a partial region of the *16S-23S ITS* gene of *Acinetobacter baumannii* may contain: a primer containing the nucleotide sequence of SEQ ID NO: 3 or a nucleotide sequence having a sequence identity of 90% or more with the nucleotide sequence of SEQ ID NO: 3; and a primer containing the nucleotide sequence of SEQ ID NO: 4 or a nucleotide sequence having a sequence identity of 90% or more with the nucleotide sequence of SEQ ID NO: 4,
the set of primers specific to the full-length or a partial region of the *ompA* gene of *Citrobacter* spp. (for example, *Citrobacter koseri, Citrobacter freundii*) may contain: one or more primers selected from a primer containing the nucleotide sequence of SEQ ID NO: 5 or a nucleotide sequence having a sequence identity of 90% or more with the nucleotide sequence of SEQ ID NO: 5, a primer containing the nucleotide sequence of SEQ ID NO: 6 or a nucleotide sequence having a sequence identity of 90% or more with the nucleotide sequence of SEQ ID NO: 6 and a combination thereof; and a primer containing the nucleotide sequence of SEQ ID NO: 7 or a nucleotide sequence having a sequence identity of 90% or more with the nucleotide sequence of SEQ ID NO: 7,
the set of primers specific to the full-length or a partial region of the *rpoS* gene of *Enterobacter* spp. may contain: a primer containing the nucleotide sequence of SEQ ID NO: 8 or a nucleotide sequence having a sequence identity of 90% or more with the nucleotide sequence of SEQ ID NO: 8; and a primer containing the nucleotide sequence of SEQ ID NO: 9 or a nucleotide sequence having a sequence identity of 90% or more with the nucleotide sequence of SEQ ID NO: 9,
the set of primers specific to the full-length or a partial region of the *gyrB* gene of *Klebsiella aerogenes* may contain: a primer containing the nucleotide sequence of SEQ ID NO: 12 or a nucleotide sequence having a sequence identity of 90% or more with the nucleotide sequence of SEQ ID NO: 12; and a primer containing the nucleotide sequence of SEQ ID NO: 13 or a nucleotide sequence having a sequence identity of 90% or more with the nucleotide sequence of SEQ ID NO: 13,
the set of primers specific to the full-length or a partial region of the *pehX* gene of *Klebsiella oxytoca* may contain: a primer containing the nucleotide sequence of SEQ ID NO: 16 or a nucleotide sequence having a sequence identity of 90% or more with the nucleotide sequence of SEQ ID NO: 16; and a primer containing the nucleotide sequence of SEQ ID NO: 17 or a nucleotide sequence having a sequence identity of 90% or more with the nucleotide sequence of SEQ ID NO: 17,
the set of primers specific to the full-length or a partial region of the *gyrB* gene of *Klebsiella variicola* may contain: a primer containing the nucleotide sequence of SEQ ID NO: 18 or a nucleotide sequence having a sequence identity of 90% or more with the nucleotide sequence of SEQ ID NO: 18; and a primer containing the nucleotide sequence of SEQ ID NO: 19 or a nucleotide sequence having a sequence identity of 90% or more with the nucleotide sequence of SEQ ID NO: 19,
the set of primers specific to the full-length or a partial region of the *oprL* gene of *Pseudomonas aeruginosa* may contain: a primer containing the nucleotide sequence of SEQ ID NO: 20 or a nucleotide sequence having a sequence identity of 90% or more with the nucleotide sequence of SEQ ID NO: 20; and a primer containing the nucleotide sequence of SEQ ID NO: 21 or a nucleotide sequence having a sequence identity of 90% or more with the nucleotide sequence of SEQ ID NO: 21,
the set of primers specific to the full-length or a partial region of the *rpoB* gene of *Proteus* spp. may contain: a primer containing the nucleotide sequence of SEQ ID NO: 22 or a nucleotide sequence having a sequence identity of 90% or more with the nucleotide sequence of SEQ ID NO: 22; and a primer containing the nucleotide sequence of SEQ ID NO: 23 or a nucleotide sequence having a sequence identity of 90% or more with the nucleotide sequence of SEQ ID NO: 23,
the set of primers specific to the full-length or a partial region of the *hasA* gene of *Serratia marcescens* may contain: a primer containing the nucleotide sequence of SEQ ID NO: 24 or a nucleotide sequence having a sequence identity of 90% or more with the nucleotide sequence of SEQ ID NO: 24; and a primer containing the nucleotide sequence of SEQ ID NO: 25 or a nucleotide sequence having a sequence identity of 90% or more with the nucleotide sequence of SEQ ID NO: 25,
the set of primers specific to the full-length or a partial region of the *iap p60* gene of *Listeria* spp. may contain: a primer containing the nucleotide sequence of SEQ ID NO: 30 or a nucleotide sequence having a sequence identity of 90% or more with the nucleotide sequence of SEQ ID NO: 30; and a primer containing the nucleotide sequence of SEQ ID NO: 31 or a nucleotide sequence having a sequence identity of 90% or more with the nucleotide sequence of SEQ ID NO: 31,
the set of primers specific to the full-length or a partial region of the *nuc* gene of *Staphylococcus argenteus* may contain: a primer containing the nucleotide sequence of SEQ ID NO: 35 or a nucleotide sequence having a sequence identity of 90% or more with the nucleotide sequence of SEQ ID NO: 35; and a primer containing the nucleotide sequence of SEQ ID NO: 36 or a nucleotide sequence having a sequence identity of 90% or more with the nucleotide sequence of SEQ ID NO: 36,
the set of primers specific to the full-length or a partial region of the *tuf* gene of *Staphylococcus* spp. may contain: a primer containing the nucleotide sequence of SEQ ID NO: 39 or a nucleotide sequence having a sequence identity of 90% or more with the nucleotide sequence of SEQ ID NO: 39; and a primer containing the nucleotide sequence of SEQ ID NO: 40 or a nucleotide sequence having a sequence identity of 90% or more with the nucleotide sequence of SEQ ID NO: 40,
the set of primers specific to the full-length or a partial region of the *rnpB* gene of *Streptococcus* spp. may contain: a primer containing the nucleotide sequence of SEQ ID NO: 41 or a nucleotide sequence having a sequence identity of 90% or more with the nucleotide sequence of SEQ ID NO: 41; and a primer containing the nucleotide sequence of SEQ ID NO: 42 or a nucleotide sequence having a sequence identity of 90% or more with the nucleotide sequence of SEQ ID NO: 42,
the set of primers specific to the full-length or a partial region of the *xisco* gene of *Streptococcus pneumoniae* may contain: a primer containing the nucleotide sequence of SEQ ID NO: 43 or a nucleotide sequence having a sequence identity of 90% or more with the nucleotide sequence of SEQ ID NO: 43; and a primer containing the nucleotide sequence of SEQ ID NO: 44 or a nucleotide sequence having a sequence identity of 90% or more with the nucleotide sequence of SEQ ID NO: 44,
the set of primers specific to the full-length or a partial region of the *gyrB* gene of *Streptococcus pyogenes* may contain: a primer containing the nucleotide sequence of SEQ ID NO: 45 or a nucleotide sequence having a sequence identity of 90% or more with the nucleotide sequence of SEQ ID NO: 45; and a primer containing the nucleotide sequence of SEQ ID NO: 46 or a nucleotide sequence having a sequence identity of 90% or more with the nucleotide sequence of SEQ ID NO: 46,
the set of primers specific to the full-length or a partial region of the *beta-lactamase_KPC* gene of *Klebsiella pneumoniae* may contain: a primer containing the nucleotide sequence of SEQ ID NO: 68 or a nucleotide sequence having a sequence identity of 90% or more with the nucleotide sequence of SEQ ID NO: 68; and a primer containing the nucleotide sequence of SEQ ID NO: 69 or a nucleotide sequence having a sequence identity of 90% or more with the nucleotide sequence of SEQ ID NO: 69,
the set of primers specific to the full-length or a partial region of the *beta-lactamase_NDM* gene of *Klebsiella pneumoniae* may contain: a primer containing the nucleotide sequence of SEQ ID NO: 70 or a nucleotide sequence having a sequence identity of 90% or more with the nucleotide sequence of SEQ ID NO: 70; and a primer containing the nucleotide sequence of SEQ ID NO: 71 or a nucleotide sequence having a sequence identity of 90% or more with the nucleotide sequence of SEQ ID NO: 71,
the set of primers specific to the full-length or a partial region of the *beta-lactamase_IMP85* gene of *Pseudomonas aeruginosa* may contain: a primer containing the nucleotide sequence of SEQ ID NO: 72 or a nucleotide sequence having a sequence identity of 90% or more with the nucleotide sequence of SEQ ID NO: 72; and a primer containing the nucleotide sequence of SEQ ID NO: 73 or a nucleotide sequence having a sequence identity of 90% or more with the nucleotide sequence of SEQ ID NO: 73,
the set of primers specific to the full-length or a partial region of the *beta-lactamase_VIM* gene of *Citrobacter freundii* may contain: a primer containing the nucleotide sequence of SEQ ID NO: 74 or a nucleotide sequence having a sequence identity of 90% or more with the nucleotide sequence of SEQ ID NO: 74; and a primer containing the nucleotide sequence of SEQ ID NO: 75 or a nucleotide sequence having a sequence identity of 90% or more with the nucleotide sequence of SEQ ID NO: 75,
the set of primers specific to the full-length or a partial region of the *beta-lactamase_CTX-M1* gene of *Salmonella enterica* may contain: a primer containing the nucleotide sequence of SEQ ID NO: 76 or a nucleotide sequence having a sequence identity of 90% or more with the nucleotide sequence of SEQ ID NO: 76; and a primer containing the nucleotide sequence of SEQ ID NO: 77 or a nucleotide sequence having a sequence identity of 90% or more with the nucleotide sequence of SEQ ID NO: 77,
the set of primers specific to the full-length or a partial region of the *beta-lactamase_CTX-M2* gene of *Salmonella enterica* may contain: a primer containing the nucleotide sequence of SEQ ID NO: 78 or a nucleotide sequence having a sequence identity of 90% or more with the nucleotide sequence of SEQ ID NO: 78; and a primer containing the nucleotide sequence of SEQ ID NO: 79 or a nucleotide sequence having a sequence identity of 90% or more with the nucleotide sequence of SEQ ID NO: 79,
the set of primers specific to the full-length or a partial region of the *beta-lactamase_CTX-M8* gene of *Citrobacter amalonaticus* may contain: a primer containing the nucleotide sequence of SEQ ID NO: 80 or a nucleotide sequence having a sequence identity of 90% or more with the nucleotide sequence of SEQ ID NO: 80; and a primer containing the nucleotide sequence of SEQ ID NO: 81 or a nucleotide sequence having a sequence identity of 90% or more with the nucleotide sequence of SEQ ID NO: 81,
the set of primers specific to the full-length or a partial region of the *beta-lactamase_CTX-M25* gene of *Escherichia coli* may contain: a primer containing the nucleotide sequence of SEQ ID NO: 82 or a nucleotide sequence having a sequence identity of 90% or more with the nucleotide sequence of SEQ ID NO: 82; and a primer containing the nucleotide sequence of SEQ ID NO: 83 or a nucleotide sequence having a sequence identity of 90% or more with the nucleotide sequence of SEQ ID NO: 83,
the set of primers specific to the full-length or a partial region of the *beta-lactamase_CTX-M9* gene of *Escherichia coli* may contain: a primer containing the nucleotide sequence of SEQ ID NO: 84 or a nucleotide sequence having a sequence identity of 90% or more with the nucleotide sequence of SEQ ID NO: 84; and a primer containing the nucleotide sequence of SEQ ID NO: 85 or a nucleotide sequence having a sequence identity of 90% or more with the nucleotide sequence of SEQ ID NO: 85,
the set of primers specific to the full-length or a partial region of the *beta-lactamase_OXA-23* gene of *Acinetobacter baumannii* may contain: a primer containing the nucleotide sequence of SEQ ID NO: 86 or a nucleotide sequence having a sequence identity of 90% or more with the nucleotide sequence of SEQ ID NO: 86; and a primer containing the nucleotide sequence of SEQ ID NO: 87 or a nucleotide sequence having a sequence identity of 90% or more with the nucleotide sequence of SEQ ID NO: 87,
the set of primers specific to the full-length or a partial region of the *beta-lactamase_OXA-40* gene of *Acinetobacter baumannii* may contain: a primer containing the nucleotide sequence of SEQ ID NO: 88 or a nucleotide sequence having a sequence identity of 90% or more with the nucleotide sequence of SEQ ID NO: 88; and a primer containing the nucleotide sequence of SEQ ID NO: 89 or a nucleotide sequence having a sequence identity of 90% or more with the nucleotide sequence of SEQ ID NO: 89,
the set of primers specific to the full-length or a partial region of the *beta-lactamase_OXA-48* gene of *Klebsiella pneumoniae* may contain: a primer containing the nucleotide sequence of SEQ ID NO: 90 or a nucleotide sequence having a sequence identity of 90% or more with the nucleotide sequence of SEQ ID NO: 90; and a primer containing the nucleotide sequence of SEQ ID NO: 91 or a nucleotide sequence having a sequence identity of 90% or more with the nucleotide sequence of SEQ ID NO: 91,
the set of primers specific to the full-length or a partial region of the *beta-lactamase_OXA-58* gene of *Acinetobacter haemolyticus* may contain: a primer containing the nucleotide sequence of SEQ ID NO: 92 or a nucleotide sequence having a sequence identity of 90% or more with the nucleotide sequence of SEQ ID NO: 92; and a primer containing the nucleotide sequence of SEQ ID NO: 93 or a nucleotide sequence having a sequence identity of 90% or more with the nucleotide sequence of SEQ ID NO: 93,
the set of primers specific to the full-length or a partial region of the *mecA* gene of *Staphylococcus aureus* may contain: a primer containing the nucleotide sequence of SEQ ID NO: 94 or a nucleotide sequence having a sequence identity of 90% or more with the nucleotide sequence of SEQ ID NO: 94; and a primer containing the nucleotide sequence of SEQ ID NO: 95 or a nucleotide sequence having a sequence identity of 90% or more with the nucleotide sequence of SEQ ID NO: 95,
the set of primers specific to the full-length or a partial region of the *vanA* gene of *Enterococcus faecalis* may contain: a primer containing the nucleotide sequence of SEQ ID NO: 96 or a nucleotide sequence having a sequence identity of 90% or more with the nucleotide sequence of SEQ ID NO: 96; and a primer containing the nucleotide sequence of SEQ ID NO: 97 or a nucleotide sequence having a sequence identity of 90% or more with the nucleotide sequence of SEQ ID NO: 97, and
the set of primers specific to the full-length or a partial region of the *vanB* gene of *Enterococcus faecium* may contain: a primer containing the nucleotide sequence of SEQ ID NO: 98 or a nucleotide sequence having a sequence identity of 90% or more with the nucleotide sequence of SEQ ID NO: 98; and a primer containing the nucleotide sequence of SEQ ID NO: 99 or a nucleotide sequence having a sequence identity of 90% or more with the nucleotide sequence of SEQ ID NO: 99.

The further use of sets of primers as mentioned above makes it possible to examine a larger number of causative bacteria of sepsis or causative bacterial genes and antimicrobial resistance genes. The use of the sets of primers reduces the interaction among the primers and improves specificity and reactivity when a PCR method is performed. In addition, the use of the sets of primers improves the amplification efficiency when a PCR method is performed in conditions equivalent in, e.g., temperature and reagent.

In the embodiment, the DNA of the full-length or a partial region (target region) of each gene and/or amplified DNA may be, for example, 50 to 300 bases in length, or 100 to 200 bases in length.

In the specification, DNA of a target region of each gene and/or amplified DNA may have a predetermined nucleotide sequence or a nucleotide sequence having a sequence identity of 85% or more, 90% or more, 91% or more, 92% or more, 93% or more, 94% or more, 95% or more, 96% or more, 97% or more, 98% or more, or 99% or more with the predetermined nucleotide sequence. DNA of the gene amplified may consist of a predetermined nucleotide sequence or a nucleotide sequence having a sequence identity of 85% or more, 90% or more, 91% or more, 92% or more, 93% or more, 94% or more, 95% or more, 96% or more, 97% or more, 98% or more, or 99% or more with the predetermined nucleotide sequence. DNA of the gene amplified may contain a predetermined nucleotide sequence or a nucleotide sequence containing 0 to 5, 1 to 4, 1 to 3, 1 or 2, or 1 nucleotide addition or deletion at the 3' end or 5' end of the predetermined nucleotide sequence.

DNA of a target region of each gene and/or amplified DNA may have a label detectable by a spectroscopic means, a photochemical means, a biochemical means, an immunochemical means, or a chemical means. Examples of the detectable label include biotin that is detected by, for example, labeled streptavidin, hapten, fluorescent dyes (for example, fluorescein, Texas red, and rhodamine), electron density reagents, enzymes (for example, horseradish peroxidase and alkaline phosphatase) and radioisotopes (for example, ³²P, ³H, ¹⁴C, and ¹²⁵I). The label, for example, may be attached to the 5' end, 3' end, or the internal portion of DNA of a gene amplified or the label may be attached via a linker. For example, the 5' end of DNA of a gene amplified may be labeled with biotin modification, in which case, the 5' end of a primer may be modified with biotin via a linker. Owing to the amplification of DNA using a primer having a label as mentioned above, it is possible to obtain amplified DNA containing a label.

The DNA of the full-length or a partial region of the *gyrB* gene of *Escherichia coli* may contain the nucleotide sequence of SEQ ID NO: 51 or a nucleotide sequence having a sequence identity of 90% or more with the nucleotide sequence of SEQ ID NO: 51,
the DNA of the full-length or a partial region of the *nuc* gene of *Staphylococcus aureus* may contain the nucleotide sequence of SEQ ID NO: 61 or a nucleotide sequence having a sequence identity of 90% or more with the nucleotide sequence of SEQ ID NO: 61,
the DNA of the full-length or a partial region of the *atlE* gene of *Staphylococcus epidermidis* may contain the nucleotide sequence of SEQ ID NO: 63 or a nucleotide sequence having a sequence identity of 90% or more with the nucleotide sequence of SEQ ID NO: 63,
the DNA of the full-length or a partial region of the *gyrB* gene of *Klebsiella pneumoniae* may contain the nucleotide sequence of SEQ ID NO: 53 or a nucleotide sequence having a sequence identity of 90% or more with the nucleotide sequence of SEQ ID NO: 53, and
the DNA of the full-length or a partial region of the *rpoB* gene of *Enterococcus* spp. may contain the nucleotide sequence of SEQ ID NO: 59 or a nucleotide sequence having a sequence identity of 90% or more with the nucleotide sequence of SEQ ID NO: 59.

The use of DNAs of the full-length or the partial region reduces the interaction among the DNAs and improves specificity and reactivity in detecting the presence or absence of amplification of each region.

In the case where any of sets of primers each specific to the full-length or a partial region of each of genes other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae* and the *rpoB* gene of *Enterococcus* spp. is further used,
DNA of the full-length or a partial region of the *rpoB* gene of *Acinetobacter* spp. may contain the nucleotide sequence of SEQ ID NO: 47 or a nucleotide sequence having a sequence identity of 90% or more with the nucleotide sequence of SEQ ID NO: 47,
DNA of the full-length or a partial region of the *16S-23S ITS* gene of *Acinetobacter baumannii* may contain the nucleotide sequence of SEQ ID NO: 48 or a nucleotide sequence having a sequence identity of 90% or more with the nucleotide sequence of SEQ ID NO: 48,
DNA of the full-length or a partial region of the *ompA* gene of *Citrobacter* spp. may contain the nucleotide sequence of SEQ ID NO: 49 or a nucleotide sequence having a sequence identity of 90% or more with the nucleotide sequence of SEQ ID NO: 49,
DNA of the full-length or a partial region of the *rpoS* gene of *Enterobacter* spp. may contain the nucleotide sequence of SEQ ID NO: 50 or a nucleotide sequence having a sequence identity of 90% or more with the nucleotide sequence of SEQ ID NO: 50,
DNA of the full-length or a partial region of the *gyrB* gene of *Klebsiella aerogenes* may contain the nucleotide sequence of SEQ ID NO: 52 or a nucleotide sequence having a sequence identity of 90% or more with the nucleotide sequence of SEQ ID NO: 52,
DNA of the full-length or a partial region of the *pehX* gene of *Klebsiella oxytoca* may contain the nucleotide sequence of SEQ ID NO: 54 or a nucleotide sequence having a sequence identity of 90% or more with the nucleotide sequence of SEQ ID NO: 54,
DNA of the full-length or a partial region of the *gyrB* gene of *Klebsiella variicola* may contain the nucleotide sequence of SEQ ID NO: 55 or a nucleotide sequence having a sequence identity of 90% or more with the nucleotide sequence of SEQ ID NO: 55,
DNA of the full-length or a partial region of the *oprL* gene of *Pseudomonas aeruginosa* may contain the nucleotide sequence of SEQ ID NO: 56 or a nucleotide sequence having a sequence identity of 90% or more with the nucleotide sequence of SEQ ID NO: 56,
DNA of the full-length or a partial region of the *rpoB* gene of *Proteus* spp. may contain the nucleotide sequence of SEQ ID NO: 57 or a nucleotide sequence having a sequence identity of 90% or more with the nucleotide sequence of SEQ ID NO: 57,
DNA of the full-length or a partial region of the *hasA* gene of *Serratia marcescens* may contain the nucleotide sequence of SEQ ID NO: 58 or a nucleotide sequence having a sequence identity of 90% or more with the nucleotide sequence of SEQ ID NO: 58,
DNA of the full-length or a partial region of the *iap p60* gene of *Listeria* spp. may contain the nucleotide sequence of SEQ ID NO: 60 or a nucleotide sequence having a sequence identity of 90% or more with the nucleotide sequence of SEQ ID NO: 60,
DNA of the full-length or a partial region of the *nuc* gene of *Staphylococcus argenteus* may contain the nucleotide sequence of SEQ ID NO: 62 or a nucleotide sequence having a sequence identity of 90% or more with the nucleotide sequence of SEQ ID NO: 62,
DNA of the full-length or a partial region of the *tuf* gene of *Staphylococcus* spp. may contain the nucleotide sequence of SEQ ID NO: 64 or a nucleotide sequence having a sequence identity of 90% or more with the nucleotide sequence of SEQ ID NO: 64,
DNA of the full-length or a partial region of the *rnpB* gene of *Streptococcus* spp. may contain the nucleotide sequence of SEQ ID NO: 65 or a nucleotide sequence having a sequence identity of 90% or more with the nucleotide sequence of SEQ ID NO: 65,
DNA of the full-length or a partial region of the *xisco* gene of *Streptococcus pneumoniae* may contain the nucleotide sequence of SEQ ID NO: 66 or a nucleotide sequence having a sequence identity of 90% or more with the nucleotide sequence of SEQ ID NO: 66,
DNA of the full-length or a partial region of the *gyrB* gene of *Streptococcus pyogenes* may contain the nucleotide sequence of SEQ ID NO: 67 or a nucleotide sequence having a sequence identity of 90% or more with the nucleotide sequence of SEQ ID NO: 67,
DNA of the full-length or a partial region of the *beta-lactamase_KPC* gene of *Klebsiella pneumoniae* may contain the nucleotide sequence of SEQ ID NO: 100 or a nucleotide sequence having a sequence identity of 90% or more with the nucleotide sequence of SEQ ID NO: 100,
DNA of the full-length or a partial region of the *beta-lactamase_NDM* gene of *Klebsiella pneumoniae* may contain the nucleotide sequence of SEQ ID NO: 101 or a nucleotide sequence having a sequence identity of 90% or more with the nucleotide sequence of SEQ ID NO: 101,
DNA of the full-length or a partial region of the *beta-lactamase_IMP85* gene of *Pseudomonas aeruginosa* may contain the nucleotide sequence of SEQ ID NO: 102 or a nucleotide sequence having a sequence identity of 90% or more with the nucleotide sequence of SEQ ID NO: 102,
DNA of the full-length or a partial region of the *beta-lactamase_VIM* gene of *Citrobacter freundii* may contain the nucleotide sequence of SEQ ID NO: 103 or a nucleotide sequence having a sequence identity of 90% or more with the nucleotide sequence of SEQ ID NO: 103,
DNA of the full-length or a partial region of the *beta-lactamase_CTX-M1* gene of *Salmonella enterica* may contain the nucleotide sequence of SEQ ID NO: 104 or a nucleotide sequence having a sequence identity of 90% or more with the nucleotide sequence of SEQ ID NO: 104,
DNA of the full-length or a partial region of the *beta-lactamase_CTX-M2* gene of *Salmonella enterica* may contain the nucleotide sequence of SEQ ID NO: 105 or a nucleotide sequence having a sequence identity of 90% or more with the nucleotide sequence of SEQ ID NO: 105,
DNA of the full-length or a partial region of the *beta-lactamase_CTX-M8* gene of *Citrobacter amalonaticus* may contain the nucleotide sequence of SEQ ID NO: 106 or a nucleotide sequence having a sequence identity of 90% or more with the nucleotide sequence of SEQ ID NO: 106,
DNA of the full-length or a partial region of the *beta-lactamase_ CTX-M25* gene of *Escherichia coli* may contain the nucleotide sequence of SEQ ID NO: 107 or a nucleotide sequence having a sequence identity of 90% or more with the nucleotide sequence of SEQ ID NO: 107,
DNA of the full-length or a partial region of the *beta-lactamase_ CTX-M9* gene of *Escherichia coli* may contain the nucleotide sequence of SEQ ID NO: 108 or a nucleotide sequence having a sequence identity of 90% or more with the nucleotide sequence of SEQ ID NO: 108,
DNA of the full-length or a partial region of the *beta-lactamase_OXA-23* gene of *Acinetobacter baumannii* may contain the nucleotide sequence of SEQ ID NO: 109 or a nucleotide sequence having a sequence identity of 90% or more with the nucleotide sequence of SEQ ID NO: 109,
DNA of the full-length or a partial region of the *beta-lactamase_OXA-40* gene of *Acinetobacter baumannii* may contain the nucleotide sequence of SEQ ID NO: 110 or a nucleotide sequence having a sequence identity of 90% or more with the nucleotide sequence of SEQ ID NO: 110,
DNA of the full-length or a partial region of the *beta-lactamase_OXA-48* gene of *Klebsiella pneumoniae* may contain the nucleotide sequence of SEQ ID NO: 111 or a nucleotide sequence having a sequence identity of 90% or more with the nucleotide sequence of SEQ ID NO: 111,
DNA of the full-length or a partial region of the *beta-lactamase_OXA-58* gene of *Acinetobacter haemolyticus* may contain the nucleotide sequence of SEQ ID NO: 112 or a nucleotide sequence having a sequence identity of 90% or more with the nucleotide sequence of SEQ ID NO: 112,
DNA of the full-length or a partial region of the *mecA* gene of *Staphylococcus aureus* may contain the nucleotide sequence of SEQ ID NO: 113 or a nucleotide sequence having a sequence identity of 90% or more with the nucleotide sequence of SEQ ID NO: 113,
DNA of the full-length or a partial region of the *vanA* gene of *Enterococcus faecalis* may contain the nucleotide sequence of SEQ ID NO: 114 or a nucleotide sequence having a sequence identity of 90% or more with the nucleotide sequence of SEQ ID NO: 114, and
DNA of the full-length or a partial region of the *vanB* gene of *Enterococcus faecium* may contain the nucleotide sequence of SEQ ID NO: 115 or a nucleotide sequence having a sequence identity of 90% or more with the nucleotide sequence of SEQ ID NO: 115.

The use of DNAs of the full-length or the partial region reduces the interaction among the DNAs and improves specificity and reactivity in detecting the presence or absence of amplification of each region.

### [Detection step]

In an embodiment, the detection step is a step of detecting the presence or absence of amplification of the full-length or a partial region of each of the genes by using probes each specific to DNA of the full-length or the partial region.

The probe specific to DNA of the full-length or a partial region (target region) of each gene can be designed based on the DNA sequence of a target region as mentioned above. The probe can be synthesized by a method known to those skilled in the art. The probe may be, for example, an oligonucleotide of 10 to 30 bases, or an oligonucleotide of 15 to 20 bases in length. The primers are not necessary to reflect an accurate sequence of a target sequence but necessary to have complementarity sufficient to hybridize with at least part (for example, a sequence of an interior region) of DNA of the target region and detect the part.

In the specification, a probe may contain a predetermined nucleotide sequence or a nucleotide sequence having a sequence identity of 85% or more, 90% or more, 91% or more, 92% or more, 93% or more, 94% or more, 95% or more, 96% or more, 97% or more, 98% or more, or 99% or more with the predetermined nucleotide sequence. The probe may consist of a predetermined nucleotide sequence or a nucleotide sequence having a sequence identity of 85% or more, 90% or more, 91% or more, 92% or more, 93% or more, 94% or more, 95% or more, 96% or more, 97% or more, 98% or more, or 99% or more with the predetermined nucleotide sequence. The probe may contain a predetermined nucleotide sequence or a nucleotide sequence containing 0 to 5, 1 to 4, 1 to 3, 1 to 2, or 1 nucleotide addition or deletion at the 3' end or 5' end of the predetermined nucleotide sequence.

The probe may be constituted of bases A, G, C, and T or analogs or degenerate bases (M, R, W, S, Y, and K). The hybridization with DNA of a target region can be more suitably made by inserting a degenerate base to a predetermined position of the sequence at which the base varies depending on the bacterial strain.

The probe may have a label detectable by a spectroscopic means, a photochemical means, a biochemical means, an immunochemical means, or a chemical means. Examples of the detectable label include biotin that is detected by labeled avidin, hapten, fluorescent dyes (for example, fluorescein, Texas red, and rhodamine), electron density reagents, enzymes (for example, horseradish peroxidase and alkaline phosphatase) and radioisotopes (for example, ³²P, ³H, ¹⁴C, and ¹²⁵I). The label, for example, may be attached to the 5' end, 3' end, or the internal portion of a probe or the label may be attached via a linker. For example, the 5' end of a probe may be labeled with biotin modification, in which case, the 5' end of the probe may be modified with biotin via a linker.

Owing to the hybridization of amplified DNA with a probe having a label, amplified DNA can be detected by use of the label. A label can be used to capture a probe in order to immobilize the probe onto a solid support.

In the embodiment, to simultaneously detect the presence or absence of amplifications of DNAs of target regions of individual genes, probes are designed such that DNAs of the individual genes can be detected in similar or equivalent conditions.

A probe may be hybridized with amplified DNA, for example, by mixing them, or alternatively, a probe may be immobilized onto a solid support and hybridized with amplified DNA. Examples of the solid support include a substrate, an array, magnetic beads, a column, and colored beads. The system to immobilize the probes onto a solid support may be in a multiplex format. Amplified DNAs may be captured by the probes immobilized onto a solid support.

In the case where probes are immobilized onto a solid support, probes containing, for example, mutually distinguishable, different identifiers (for example, ID) may be immobilized or probes may be immobilized onto respective identifiers of a solid support including distinguishable identifiers. In other words, probes may contain different identifiers or probes may be bound to different identifiers on a solid support. If probes are immobilized on a solid support, amplified DNAs can specifically bind to the corresponding probes to capture the amplified DNAs. The amplified DNA captured is labeled and can be detected based on the label. For example, if a fluorescent label is attached, amplified DNA can be detected by measuring fluorescence value. Examples of a device for measuring the values of fluorescence, which is emitted when probes bind to amplified DNAs through identification by an identifier, include a fluorometer, 100 Fluorescent Analyzer (PlexBio Co., Ltd.).

The method to detect the presence or absence of amplification of DNA of a target region of each gene using a probe can be performed in accordance with a method known to those skilled in the art, for example, as follows.

A PCR product (containing amplified DNA if a sample contains DNA of a target region) obtained in a step of performing a PCR method is denatured with heat (for example, 95°C for 5 minutes, and thereafter, rapidly cooled to 4°C) and hybridized with probes. The heat denaturation may be performed by heating at, e.g., 90°C to 95°C for 3 to 10 minutes, followed by rapid cooling to 0 to 5°C, or by heating at 95°C for 5 minutes followed by rapid cooling to 4°C. Hybridization may be performed by incubation at, e.g., 35°C to 39°C for 10 to 30 minutes or at 37°C for 20 minutes. The probes may be acceptable as long as they hybridize separately with DNAs of target regions under stringent conditions. The "stringent conditions" in the specification refer to conditions where a complementary strand of a nucleotide chain having a homology to the sequence of a target region preferentially hybridizes with the sequence of a target region but a complementary strand of a nucleotide chain not having a homology does not substantially hybridize with the sequence of a target region. The stringent conditions are determined in a sequence-dependent manner and vary depending on various situations. The longer the sequence, the higher temperature at which the sequence specifically hybridizes. Examples of the stringent conditions include conditions in which incubation is performed with, for example, 50% formamide, 5 × SSC (150 mM sodium chloride, 15 mM trisodium citrate, 10 mM sodium phosphate, 1 mM ethylenediaminetetraacetate, pH 7.2), 5 × Denhardt's solution, 0.1% SDS, 10% dextran sulfate and 100 µg/mL of denatured salmon sperm DNA at 42°C, and thereafter, a filter is washed in 0.2 × SSC at 42°C.

After completion of hybridization, for example, detection can be made by use of a label of amplified DNA and/or probes. Detection can be appropriately made depending on the type of label. Alternatively, detection can be made, after completion of hybridization, by labeling amplified DNA modified with biotin with labeled avidin (for example, Streptavidin-Phycoerythrin (PlexBio Co., Ltd.)). The labeling can be appropriately performed in accordance with the type of label, for example, by incubation at 35°C to 39°C for 10 to 30 minutes or 37°C for 10 minutes.

In the detection step of the embodiment, the presence or absence of amplifications of DNAs of target regions is detected simultaneously by use of probes specific to individual DNAs. The method for simultaneously detecting the presence or absence of amplifications of DNAs of target regions by use of probes specific to the individual DNAs can be performed in accordance with a method known to those skilled in the art.

Examples of a method for simultaneously detecting the presence or absence of amplifications of DNAs of different target regions include multiplex PCR assay. Accordingly, the method according to the embodiment can be performed in accordance with multiplex PCR assay. The detection step may include hybridizing probes which are immobilized onto a solid substrate in a multiplex format and specific to the corresponding DNAs of target regions, with amplified DNAs of target regions in a step of performing a PCR method (for example, by using an amplification reaction mixture), and simultaneously detecting the amplified DNAs of target regions hybridized.

In the embodiment, in order to simultaneously detect the presence or absence of amplifications of DNAs of different target regions in a single reaction system (for example, hybridization reaction system), probes specific to the corresponding DNAs of target regions of individual genes as mentioned above are collectively used. For example, if a combination of amplified DNAs (for example, mixture) is brought into contact with a combination of probes (for example, mixture) in conditions suitable for a hybridization reaction, probes can specifically bind to at least part of the corresponding amplified DNAs of individual genes, and detection can be made, for example, by real-time PCR detection (for example, TaqMan probe, molecular label) based on a label of amplified DNA, a label of probes or labeling following amplified DNA captured, or by solid-support hybridization (for example, nucleic-acid microarray hybridization and bead-base capturing).

The presence or absence of amplifications of DNAs of target regions of bacterial target genes or bacterial target genes and antimicrobial resistance genes can be appropriately detected depending on the detection method. For example, whether or not DNA of a target region was amplified may be determined in comparison with a control sample not containing bacterial DNA or it may be determined with reference to a standard value. When the presence or absence of amplification is determined with reference to a standard value previously determined, for example, if a value exceeds the standard value, it may be determined that DNA of a target region of a bacterial target gene or a bacterial target gene and an antimicrobial resistance gene was detected. The standard value may be set for each gene or in common for all genes. The standard value may be set based on a value of a control sample not containing bacterial DNA, based on a value of a sample containing bacterial DNA, or through comparison between a value of a control sample and a value of a sample containing bacterial DNA. Alternatively, a cut-off value may be used for appropriately determining the presence or absence of the amplification of DNA of a target region of each gene. A cut-off value may be set for each gene or in common for all genes. The cut-off value can be set using, for example, the ROC curve, that is, a value at which a desired sensitivity and specificity can be obtained may be selected as the cut-off value. For example, if a cut-off value for a probe exhibiting some cross-reactivity is set to be slightly higher, accuracy in determination can be improved.

Based on DNA of a target region the amplification of which was confirmed, causative bacteria, or causative bacteria, and an antimicrobial resistance gene of the bacteria present in the sample of a subject can be identified. For example, if the amplification of DNA of a target region of a gene of a predetermined bacterium is detected, the bacterium may be identified as a causative bacterium.

In the detection step, a probe specific to DNA of the full-length or a partial region of the *gyrB* gene of *Escherichia coli* may contain the nucleotide sequence of SEQ ID NO: 121 or a nucleotide sequence having a sequence identity of 90% or more with the nucleotide sequence of SEQ ID NO: 121,
a probe specific to DNA of the full-length or a partial region of the *nuc* gene of *Staphylococcus aureus* may contain the nucleotide sequence of SEQ ID NO: 134 or a nucleotide sequence having a sequence identity of 90% or more with the nucleotide sequence of SEQ ID NO: 134,
a probe specific to DNA of the full-length or a partial region of the *atlE* gene of *Staphylococcus epidermidis* may contain the nucleotide sequence of SEQ ID NO: 136 or a nucleotide sequence having a sequence identity of 90% or more with the nucleotide sequence of SEQ ID NO: 136,
a probe specific to DNA of the full-length or a partial region of the *gyrB* gene of *Klebsiella pneumoniae* may contain the nucleotide sequence of SEQ ID NO: 124 or a nucleotide sequence having a sequence identity of 90% or more with the nucleotide sequence of SEQ ID NO: 124, and
a probe specific to DNA of the full-length or a partial region of the *rpoB* gene of *Enterococcus* spp. may be selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof.

The use of probes as mentioned above reduces the interaction among the probes and improves specificity and reactivity in detecting the presence or absence of amplification of each region. And simultaneously, the use of probes as mentioned above improves the hybridization efficiency when the hybridization reactions are performed in conditions equivalent in, e.g., temperature and reagent.

In the case where a probe specific to DNA of the full-length or a partial region of a gene other than the *gyrB* gene of *Escherichia coli,* the *nuc* gene of *Staphylococcus* aureus, the *atlE* gene of *Staphylococcus epidermidis*, the *gyrB* gene of *Klebsiella pneumoniae* and the *rpoB* gene of *Enterococcus* spp., is further used,
the probe specific to DNA of the full-length or a partial region of the *rpoB* gene of *Acinetobacter* spp. may be selected from a probe containing the nucleotide sequence of SEQ ID NO: 116 or a nucleotide sequence having a sequence identity of 90% or more with the nucleotide sequence of SEQ ID NO: 116, a probe containing the nucleotide sequence of SEQ ID NO: 117 or a nucleotide sequence having a sequence identity of 90% or more with the nucleotide sequence of SEQ ID NO: 117 and a combination thereof,
the probe specific to DNA of the full-length or a partial region of the *16S-23S ITS* gene of *Acinetobacter baumannii* may contain the nucleotide sequence of SEQ ID NO: 118 or a nucleotide sequence having a sequence identity of 90% or more with the nucleotide sequence of SEQ ID NO: 118,
the probe specific to DNA of the full-length or a partial region of the *ompa* gene of *Citrobacter* spp. may contain the nucleotide sequence of SEQ ID NO: 119 or a nucleotide sequence having a sequence identity of 90% or more with the nucleotide sequence of SEQ ID NO: 119,
the probe specific to DNA of the full-length or a partial region of the *rpoS* gene of *Enterobacter* spp. may contain the nucleotide sequence of SEQ ID NO: 120 or a nucleotide sequence having a sequence identity of 90% or more with the nucleotide sequence of SEQ ID NO: 120,
the probe specific to DNA of the full-length or a partial region of the *gyrB* gene of *Klebsiella aerogenes* may contain the nucleotide sequence of SEQ ID NO: 122 or a nucleotide sequence having a sequence identity of 90% or more with the nucleotide sequence of SEQ ID NO: 122,
the probe specific to DNA of the full-length or a partial region of the *pehX* gene of *Klebsiella oxytoca* may contain the nucleotide sequence of SEQ ID NO: 123 or a nucleotide sequence having a sequence identity of 90% or more with the nucleotide sequence of SEQ ID NO: 123,
the probe specific to DNA of the full-length or a partial region of the *gyrB* gene of *Klebsiella variicola* may contain the nucleotide sequence of SEQ ID NO: 125 or a nucleotide sequence having a sequence identity of 90% or more with the nucleotide sequence of SEQ ID NO: 125,
the probe specific to DNA of the full-length or a partial region of the *oprL* gene of *Pseudomonas aeruginosa* may contain the nucleotide sequence of SEQ ID NO: 126 or a nucleotide sequence having a sequence identity of 90% or more with the nucleotide sequence of SEQ ID NO: 126,
the probe specific to DNA of the full-length or a partial region of the *rpoB* gene of *Proteus* spp. may contain the nucleotide sequence of SEQ ID NO: 127 or a nucleotide sequence having a sequence identity of 90% or more with the nucleotide sequence of SEQ ID NO: 127,
the probe specific to DNA of the full-length or a partial region of the *hasA* gene of *Serratia marcescens* may contain the nucleotide sequence of SEQ ID NO: 128 or a nucleotide sequence having a sequence identity of 90% or more with the nucleotide sequence of SEQ ID NO: 128,
the probe specific to DNA of the full-length or a partial region of the *iap p60* gene of *Listeria* spp. may be selected from a probe containing the nucleotide sequence of SEQ ID NO: 132 or a nucleotide sequence having a sequence identity of 90% or more with the nucleotide sequence of SEQ ID NO: 132, a probe containing the nucleotide sequence of SEQ ID NO: 133 or a nucleotide sequence having a sequence identity of 90% or more with the nucleotide sequence of SEQ ID NO: 133 and a combination thereof,
the probe specific to DNA of the full-length or a partial region of the *nuc* gene of *Staphylococcus argenteus* may contain the nucleotide sequence of SEQ ID NO: 135 or a nucleotide sequence having a sequence identity of 90% or more with the nucleotide sequence of SEQ ID NO: 135,
the probe specific to DNA of the full-length or a partial region of the *tuf* gene of *Staphylococcus* spp. may contain the nucleotide sequence of SEQ ID NO: 137 or a nucleotide sequence having a sequence identity of 90% or more with the nucleotide sequence of SEQ ID NO: 137,
the probe specific to DNA of the full-length or a partial region of the *rnpB* gene of *Streptococcus* spp. may contain the nucleotide sequence of SEQ ID NO: 140 or a nucleotide sequence having a sequence identity of 90% or more with the nucleotide sequence of SEQ ID NO: 140,
the probe specific to DNA of the full-length or a partial region of the *xisco* gene of *Streptococcus pneumoniae* may contain the nucleotide sequence of SEQ ID NO: 138 or a nucleotide sequence having a sequence identity of 90% or more with the nucleotide sequence of SEQ ID NO: 138,
the probe specific to DNA of the full-length or a partial region of the *gyrB* gene of *Streptococcus pyogenes* may contain the nucleotide sequence of SEQ ID NO: 139 or a nucleotide sequence having a sequence identity of 90% or more with the nucleotide sequence of SEQ ID NO: 139,
the probe specific to DNA of the full-length or a partial region of the *beta-lactamase_KPC* gene of *Klebsiella pneumoniae* may contain the nucleotide sequence of SEQ ID NO: 142 or a nucleotide sequence having a sequence identity of 90% or more with the nucleotide sequence of SEQ ID NO: 142,
the probe specific to DNA of the full-length or a partial region of the *beta-lactamase_NDM* gene of *Klebsiella pneumoniae* may contain the nucleotide sequence of SEQ ID NO: 141 or a nucleotide sequence having a sequence identity of 90% or more with the nucleotide sequence of SEQ ID NO: 141,
the probe specific to DNA of the full-length or a partial region of the *beta-lactamase_IMP85* gene of *Pseudomonas aeruginosa* may contain the nucleotide sequence of SEQ ID NO: 144 or a nucleotide sequence having a sequence identity of 90% or more with the nucleotide sequence of SEQ ID NO: 144,
the probe specific to DNA of the full-length or a partial region of the *beta-lactamase_VIM* gene of *Citrobacter freundii* may contain the nucleotide sequence of SEQ ID NO: 143 or a nucleotide sequence having a sequence identity of 90% or more with the nucleotide sequence of SEQ ID NO: 143,
the probe specific to DNA of the full-length or a partial region of the *beta-lactamase_CTX-M1* gene of *Salmonella enterica* may contain the nucleotide sequence of SEQ ID NO: 149 or a nucleotide sequence having a sequence identity of 90% or more with the nucleotide sequence of SEQ ID NO: 149,
the probe specific to DNA of the full-length or a partial region of the *beta-lactamase_CTX-M2* gene of *Salmonella enterica* may contain the nucleotide sequence of SEQ ID NO: 150 or a nucleotide sequence having a sequence identity of 90% or more with the nucleotide sequence of SEQ ID NO: 150,
the probe specific to DNA of the full-length or a partial region of the *beta-lactamase_CTX-M8* gene of *Citrobacter amalonaticus* may contain the nucleotide sequence of SEQ ID NO: 151 or a nucleotide sequence having a sequence identity of 90% or more with the nucleotide sequence of SEQ ID NO: 151,
the probe specific to DNA of the full-length or a partial region of the *beta-lactamase*_*CTX-M25* gene of *Escherichia coli* may contain the nucleotide sequence of SEQ ID NO: 153 or a nucleotide sequence having a sequence identity of 90% or more with the nucleotide sequence of SEQ ID NO: 153,
the probe specific to DNA of the full-length or a partial region of the *beta-lactamase_CTX-M9* gene of *Escherichia coli* may contain the nucleotide sequence of SEQ ID NO: 152 or a nucleotide sequence having a sequence identity of 90% or more with the nucleotide sequence of SEQ ID NO: 152,
the probe specific to DNA of the full-length or a partial region of the *beta-lactamase_OXA-23* gene of *Acinetobacter baumannii* may contain the nucleotide sequence of SEQ ID NO: 145 or a nucleotide sequence having a sequence identity of 90% or more with the nucleotide sequence of SEQ ID NO: 145,
the probe specific to DNA of the full-length or a partial region of the *beta-lactamase_OXA-40* gene of *Acinetobacter baumannii* may contain the nucleotide sequence of SEQ ID NO: 146 or a nucleotide sequence having a sequence identity of 90% or more with the nucleotide sequence of SEQ ID NO: 146,
the probe specific to DNA of the full-length or a partial region of the *beta-lactamase_OXA-48* gene of *Klebsiella pneumoniae* may contain the nucleotide sequence of SEQ ID NO: 147 or a nucleotide sequence having a sequence identity of 90% or more with the nucleotide sequence of SEQ ID NO: 147,
the probe specific to DNA of the full-length or a partial region of the *beta-lactamase-OXA-58* gene of *Acinetobacter haemolyticus* may contain the nucleotide sequence of SEQ ID NO: 148 or a nucleotide sequence having a sequence identity of 90% or more with the nucleotide sequence of SEQ ID NO: 148,
the probe specific to DNA of the full-length or a partial region of the *mecA* gene of *Staphylococcus aureus* may contain the nucleotide sequence of SEQ ID NO: 154 or a nucleotide sequence having a sequence identity of 90% or more with the nucleotide sequence of SEQ ID NO: 154,
the probe specific to DNA of the full-length or a partial region of the *vanA* gene of *Enterococcus faecalis* may contain the nucleotide sequence of SEQ ID NO: 155 or a nucleotide sequence having a sequence identity of 90% or more with the nucleotide sequence of SEQ ID NO: 155, and
the probe specific to DNA of the full-length or a partial region of the *vanB* gene of *Enterococcus faecium* may contain the nucleotide sequence of SEQ ID NO: 156 or a nucleotide sequence having a sequence identity of 90% or more with the nucleotide sequence of SEQ ID NO: 156.

The further use of probes as mentioned above makes it possible to examine a larger number of causative bacteria of sepsis in combination with antimicrobial resistance genes.

The use of probes as mentioned above reduces the interaction among the probes and improves specificity and reactivity in detecting the presence or absence of amplification of each region. In addition, the use of probes as mentioned above improves the hybridization efficiency when the hybridization reactions are performed in conditions equivalent in, e.g., temperature and reagent.

The present invention also provides, as an embodiment, a method for identifying causative bacteria of sepsis and/or antimicrobial resistance genes, including:
a step of performing a PCR method using a sample collected from a subject and a combination of sets of primers each specific to the full-length or a partial region of each of two types or more bacterial genes and/or antimicrobial resistance genes selected from the group consisting of: the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* the *rpoB* gene of *Enterococcus* spp. (for example, *Enterococcus faecalis*), the *rpoB* gene of *Acinetobacter* spp. (for example, *Acinetobacter baumannii,* the *16S-23S ITS* gene of *Acinetobacter baumannii,* the *ompA* gene of *Citrobacter* spp. (for example, *Citrobacter koseri, Citrobacter freundii*), the *rpoS* gene of *Enterobacter cloacae*, the *gyrB* gene of *Klebsiella aerogenes,* the *pehX* gene of *Klebsiella oxytoca,* the *gyrB* gene of *Klebsiella variicola*, the *oprL* gene of *Pseudomonas aeruginosa,* the *rpoB* gene of *Proteus* spp. (for example, *Proteus mirabilis*), the *hasA* gene of *Serratia marcescens,* the *iap p60* gene of *Listeria* spp. (for example, *Listeria monocytogenes*), the *nuc* gene of *Staphylococcus argenteus,* the *tuf* gene of *Staphylococcus* spp. (for example, *Staphylococcus aureus*), the *rnpB* gene of *Streptococcus* spp. (for example, *Streptococcus pneumoniae*), the *xisco* gene of *Streptococcus pneumoniae,* the *gyrB* gene of *Streptococcus pyogenes,* the *beta-lactamase_KPC* gene of *Klebsiella pneumoniae,* the *beta-lactamase_NDM* gene of *Klebsiella pneumoniae,* the *beta-lactamase_IMP85* gene of *Pseudomonas aeruginosa,* the *beta-lactamase_VIM gene* of *Citrobacter freundii,* the *beta-lactamase_CTX-M1* gene of *Salmonella enterica,* the *beta-lactamase_CTX-M2 gene* of *Salmonella enterica,* the *beta-lactamase_CTX-M8 gene* of *Citrobacter amalonaticus,* the *beta-lactamase_CTX-M25* gene of *Escherichia coli,* the *beta-lactamase_CTX-M9 gene* of *Escherichia coli,* the *beta-lactamase_OXA-23 gene* of *Acinetobacter baumannii,* the *beta-lactamase_OXA-40 gene* of *Acinetobacter baumannii,* the *beta-lactamase_OXA-48 gene* of *Klebsiella pneumoniae,* the *beta-lactamase_OXA-58* gene *of Acinetobacter haemolyticus,* the *mecA* gene of *Staphylococcus aureus,* the *vanA* gene of *Enterococcus faecalis,* and the *vanB* gene of *Enterococcus faecium*; and
a step of detecting the presence or absence of amplification of DNA of the full-length or a partial region of each of the genes by using a combination of probes each specific to DNA of the full-length or the partial region.

The bacteria or genes selected from the group of the plurality of genes as mentioned above may be, for example, 5 types or more, 10 types or more, 15 types or more, 20 types or more, 25 types or more, 28 types or more, 30 types or more, 32 types or more, 35 types or more, or 37 types. The genes of bacteria selected from the group of the plurality of genes as mentioned above may be, for example, 3 types or more, 5 types or more, 7 types or more, 10 types or more, 12 types or more, 15 types or more, 17 types or more, 19 types or more, or 21 types or more. The antimicrobial resistance genes selected from the group of the plurality of genes as mentioned above may be, for example, 3 types or more, 5 types or more, 7 types or more, 10 types or more, 12 types or more, 14 types or more, or 16 types or more. The bacteria or genes selected from the group of the plurality of genes as mentioned above may be, for example, 37 types or less, 34 types or less, 21 types or less, 16 types or less, or 11 types or less. The bacteria or genes selected from the group of the plurality of genes as mentioned above may be, for example, 2 to 40 types, 5 to 37 types, 10 to 37 types, or 15 to 25 types.

In the step of performing a PCR method, the set of primers specific to the full-length or a partial region of the *gyrB* gene of *Escherichia coli* may contain: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
the set of primers specific to the full-length or a partial region of the *nuc* gene of *Staphylococcus aureus* may contain: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
the set of primers specific to the full-length or a partial region of the *atlE* gene of *Staphylococcus epidermidis* may contain: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
the set of primers specific to the full-length or a partial region of the *gyrB* gene of *Klebsiella pneumoniae* may contain: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15,
the set of primers specific to the full-length or a partial region of the *rpoB* gene of *Enterococcus* spp. may contain: one or more primers selected from a primer containing the nucleotide sequence of SEQ ID NO: 26 or the nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29,
the set of primers specific to the full-length or a partial region of the *rpoB* gene of *Acinetobacter* spp. may contain: a primer containing the nucleotide sequence of SEQ ID NO: 1 or a nucleotide sequence having a sequence identity of 90% or more with the nucleotide sequence of SEQ ID NO: 1; and a primer containing the nucleotide sequence of SEQ ID NO: 2 or a nucleotide sequence having a sequence identity of 90% or more with the nucleotide sequence of SEQ ID NO: 2,
the set of primers specific to the full-length or a partial region of the *16S-23S ITS* gene of *Acinetobacter baumannii* may contain: a primer containing the nucleotide sequence of SEQ ID NO: 3 or a nucleotide sequence having a sequence identity of 90% or more with the nucleotide sequence of SEQ ID NO: 3; and a primer containing the nucleotide sequence of SEQ ID NO: 4 or a nucleotide sequence having a sequence identity of 90% or more with the nucleotide sequence of SEQ ID NO: 4,
the set of primers specific to the full-length or a partial region of the *ompa* gene of *Citrobacter* spp. may contain: one or more primers selected from a primer containing the nucleotide sequence of SEQ ID NO: 5 or a nucleotide sequence having a sequence identity of 90% or more with the nucleotide sequence of SEQ ID NO: 5, a primer containing the nucleotide sequence of SEQ ID NO: 6 or a nucleotide sequence having a sequence identity of 90% or more with the nucleotide sequence of SEQ ID NO: 6 and a combination thereof; and a primer containing the nucleotide sequence of SEQ ID NO: 7 or a nucleotide sequence having a sequence identity of 90% or more with the nucleotide sequence of SEQ ID NO: 7,
the set of primers specific to the full-length or a partial region of the *rpoS* gene of *Enterobacter* spp. may contain: a primer containing the nucleotide sequence of SEQ ID NO: 8 or a nucleotide sequence having a sequence identity of 90% or more with the nucleotide sequence of SEQ ID NO: 8; and a primer containing the nucleotide sequence of SEQ ID NO: 9 or a nucleotide sequence having a sequence identity of 90% or more with the nucleotide sequence of SEQ ID NO: 9,
the set of primers specific to the full-length or a partial region of the *gyrB* gene of *Klebsiella aerogenes* may contain: a primer containing the nucleotide sequence of SEQ ID NO: 12 or a nucleotide sequence having a sequence identity of 90% or more with the nucleotide sequence of SEQ ID NO: 12; and a primer containing the nucleotide sequence of SEQ ID NO: 13 or a nucleotide sequence having a sequence identity of 90% or more with the nucleotide sequence of SEQ ID NO: 13,
the set of primers specific to the full-length or a partial region of the *pehX* gene of *Klebsiella oxytoca* may contain: a primer containing the nucleotide sequence of SEQ ID NO: 16 or a nucleotide sequence having a sequence identity of 90% or more with the nucleotide sequence of SEQ ID NO: 16; and a primer containing the nucleotide sequence of SEQ ID NO: 17 or a nucleotide sequence having a sequence identity of 90% or more with the nucleotide sequence of SEQ ID NO: 17,
the set of primers specific to the full-length or a partial region of the *gyrB* gene of *Klebsiella variicola* may contain: a primer containing the nucleotide sequence of SEQ ID NO: 18 or a nucleotide sequence having a sequence identity of 90% or more with the nucleotide sequence of SEQ ID NO: 18; and a primer containing the nucleotide sequence of SEQ ID NO: 19 or a nucleotide sequence having a sequence identity of 90% or more with the nucleotide sequence of SEQ ID NO: 19,
the set of primers specific to the full-length or a partial region of the *oprL* gene of *Pseudomonas aeruginosa* may contain: a primer containing the nucleotide sequence of SEQ ID NO: 20 or a nucleotide sequence having a sequence identity of 90% or more with the nucleotide sequence of SEQ ID NO: 20; and a primer containing the nucleotide sequence of SEQ ID NO: 21 or a nucleotide sequence having a sequence identity of 90% or more with the nucleotide sequence of SEQ ID NO: 21,
the set of primers specific to the full-length or a partial region of the *rpoB* gene of *Proteus* spp. may contain: a primer containing the nucleotide sequence of SEQ ID NO: 22 or a nucleotide sequence having a sequence identity of 90% or more with the nucleotide sequence of SEQ ID NO: 22; and a primer containing the nucleotide sequence of SEQ ID NO: 23 or a nucleotide sequence having a sequence identity of 90% or more with the nucleotide sequence of SEQ ID NO: 23,
the set of primers specific to the full-length or a partial region of the *hasA* gene of *Serratia marcescens* may contain: a primer containing the nucleotide sequence of SEQ ID NO: 24 or a nucleotide sequence having a sequence identity of 90% or more with the nucleotide sequence of SEQ ID NO: 24; and a primer containing the nucleotide sequence of SEQ ID NO: 25 or a nucleotide sequence having a sequence identity of 90% or more with the nucleotide sequence of SEQ ID NO: 25,
the set of primers specific to the full-length or a partial region of the *iap p60* gene of *Listeria* spp. may contain: a primer containing the nucleotide sequence of SEQ ID NO: 30 or a nucleotide sequence having a sequence identity of 90% or more with the nucleotide sequence of SEQ ID NO: 30; and a primer containing the nucleotide sequence of SEQ ID NO: 31 or a nucleotide sequence having a sequence identity of 90% or more with the nucleotide sequence of SEQ ID NO: 31,
the set of primers specific to the full-length or a partial region of the *nuc* gene of *Staphylococcus argenteus* may contain: a primer containing the nucleotide sequence of SEQ ID NO: 35 or a nucleotide sequence having a sequence identity of 90% or more with the nucleotide sequence of SEQ ID NO: 35; and a primer containing the nucleotide sequence of SEQ ID NO: 36 or a nucleotide sequence having a sequence identity of 90% or more with the nucleotide sequence of SEQ ID NO: 36,
the set of primers specific to the full-length or a partial region of the *tuf* gene of *Staphylococcus* spp. may contain: a primer containing the nucleotide sequence of SEQ ID NO: 39 or a nucleotide sequence having a sequence identity of 90% or more with the nucleotide sequence of SEQ ID NO: 39; and a primer containing the nucleotide sequence of SEQ ID NO: 40 or a nucleotide sequence having a sequence identity of 90% or more with the nucleotide sequence of SEQ ID NO: 40,
the set of primers specific to the full-length or a partial region of the *rnpB* gene of *Streptococcus* spp. may contain: a primer containing the nucleotide sequence of SEQ ID NO: 41 or a nucleotide sequence having a sequence identity of 90% or more with the nucleotide sequence of SEQ ID NO: 41; and a primer containing the nucleotide sequence of SEQ ID NO: 42 or a nucleotide sequence having a sequence identity of 90% or more with the nucleotide sequence of SEQ ID NO: 42,
the set of primers specific to the full-length or a partial region of the *xisco* gene of *Streptococcus pneumoniae* may contain: a primer containing the nucleotide sequence of SEQ ID NO: 43 or a nucleotide sequence having a sequence identity of 90% or more with the nucleotide sequence of SEQ ID NO: 43; and a primer containing the nucleotide sequence of SEQ ID NO: 44 or a nucleotide sequence having a sequence identity of 90% or more with the nucleotide sequence of SEQ ID NO: 44,
the set of primers specific to the full-length or a partial region of the *gyrB* gene of *Streptococcus pyogenes* may contain: a primer containing the nucleotide sequence of SEQ ID NO: 45 or a nucleotide sequence having a sequence identity of 90% or more with the nucleotide sequence of SEQ ID NO: 45; and a primer containing the nucleotide sequence of SEQ ID NO: 46 or a nucleotide sequence having a sequence identity of 90% or more with the nucleotide sequence of SEQ ID NO: 46,
the set of primers specific to the full-length or a partial region of the *beta-lactamase_KPC* gene of *Klebsiella pneumoniae* may contain: a primer containing the nucleotide sequence of SEQ ID NO: 68 or a nucleotide sequence having a sequence identity of 90% or more with the nucleotide sequence of SEQ ID NO: 68; and a primer containing the nucleotide sequence of SEQ ID NO: 69 or a nucleotide sequence having a sequence identity of 90% or more with the nucleotide sequence of SEQ ID NO: 69,
the set of primers specific to the full-length or a partial region of the *beta-lactamase_NDM* gene of *Klebsiella pneumoniae* may contain: a primer containing the nucleotide sequence of SEQ ID NO: 70 or a nucleotide sequence having a sequence identity of 90% or more with the nucleotide sequence of SEQ ID NO: 70; and a primer containing the nucleotide sequence of SEQ ID NO: 71 or a nucleotide sequence having a sequence identity of 90% or more with the nucleotide sequence of SEQ ID NO: 71,
the set of primers specific to the full-length or a partial region of the *beta-lactamase_IMP85* gene of *Pseudomonas aeruginosa* may contain: a primer containing the nucleotide sequence of SEQ ID NO: 72 or a nucleotide sequence having a sequence identity of 90% or more with the nucleotide sequence of SEQ ID NO: 72; and a primer containing the nucleotide sequence of SEQ ID NO: 73 or a nucleotide sequence having a sequence identity of 90% or more with the nucleotide sequence of SEQ ID NO: 73,
the set of primers specific to the full-length or a partial region of the *beta-lactamase_VIM* gene of *Citrobacter freundii* may contain: a primer containing the nucleotide sequence of SEQ ID NO: 74 or a nucleotide sequence having a sequence identity of 90% or more with the nucleotide sequence of SEQ ID NO: 74; and a primer containing the nucleotide sequence of SEQ ID NO: 75 or a nucleotide sequence having a sequence identity of 90% or more with the nucleotide sequence of SEQ ID NO: 75,
the set of primers specific to the full-length or a partial region of the *beta-lactamase_CTX-M1* gene of *Salmonella enterica* may contain: a primer containing the nucleotide sequence of SEQ ID NO: 76 or a nucleotide sequence having a sequence identity of 90% or more with the nucleotide sequence of SEQ ID NO: 76; and a primer containing the nucleotide sequence of SEQ ID NO: 77 or a nucleotide sequence having a sequence identity of 90% or more with the nucleotide sequence of SEQ ID NO: 77,
the set of primers specific to the full-length or a partial region of the *beta-lactamase_CTX-M2* gene of *Salmonella enterica* may contain: a primer containing the nucleotide sequence of SEQ ID NO: 78 or a nucleotide sequence having a sequence identity of 90% or more with the nucleotide sequence of SEQ ID NO: 78; and a primer containing the nucleotide sequence of SEQ ID NO: 79 or a nucleotide sequence having a sequence identity of 90% or more with the nucleotide sequence of SEQ ID NO: 79,
the set of primers specific to the full-length or a partial region of the *beta-lactamase_CTX-M8* gene of *Citrobacter amalonaticus* may contain: a primer containing the nucleotide sequence of SEQ ID NO: 80 or a nucleotide sequence having a sequence identity of 90% or more with the nucleotide sequence of SEQ ID NO: 80; and a primer containing the nucleotide sequence of SEQ ID NO: 81 or a nucleotide sequence having a sequence identity of 90% or more with the nucleotide sequence of SEQ ID NO: 81,
the set of primers specific to the full-length or a partial region of the *beta-lactamase_CTX-M25* gene of *Escherichia coli* may contain: a primer containing the nucleotide sequence of SEQ ID NO: 82 or a nucleotide sequence having a sequence identity of 90% or more with the nucleotide sequence of SEQ ID NO: 82; and a primer containing the nucleotide sequence of SEQ ID NO: 83 or a nucleotide sequence having a sequence identity of 90% or more with the nucleotide sequence of SEQ ID NO: 83,
the set of primers specific to the full-length or a partial region of the *beta-lactamase_CTX-M9* gene of *Escherichia coli* may contain: a primer containing the nucleotide sequence of SEQ ID NO: 84 or a nucleotide sequence having a sequence identity of 90% or more with the nucleotide sequence of SEQ ID NO: 84; and a primer containing the nucleotide sequence of SEQ ID NO: 85 or a nucleotide sequence having a sequence identity of 90% or more with the nucleotide sequence of SEQ ID NO: 85,
the set of primers specific to the full-length or a partial region of the *beta-lactamase_OXA-23* gene of *Acinetobacter baumannii* may contain: a primer containing the nucleotide sequence of SEQ ID NO: 86 or a nucleotide sequence having a sequence identity of 90% or more with the nucleotide sequence of SEQ ID NO: 86; and a primer containing the nucleotide sequence of SEQ ID NO: 87 or a nucleotide sequence having a sequence identity of 90% or more with the nucleotide sequence of SEQ ID NO: 87,
the set of primers specific to the full-length or a partial region of the *beta-lactamase_OXA-40* gene of *Acinetobacter baumannii* may contain: a primer containing the nucleotide sequence of SEQ ID NO: 88 or a nucleotide sequence having a sequence identity of 90% or more with the nucleotide sequence of SEQ ID NO: 88; and a primer containing the nucleotide sequence of SEQ ID NO: 89 or a nucleotide sequence having a sequence identity of 90% or more with the nucleotide sequence of SEQ ID NO: 89,
the set of primers specific to the full-length or a partial region of the *beta-lactamase_OXA-48* gene of *Klebsiella pneumoniae* may contain: a primer containing the nucleotide sequence of SEQ ID NO: 90 or a nucleotide sequence having a sequence identity of 90% or more with the nucleotide sequence of SEQ ID NO: 90; and a primer containing the nucleotide sequence of SEQ ID NO: 91 or a nucleotide sequence having a sequence identity of 90% or more with the nucleotide sequence of SEQ ID NO: 91,
the set of primers specific to the full-length or a partial region of the *beta-lactamase_OXA-58* gene of *Acinetobacter haemolyticus* may contain: a primer containing the nucleotide sequence of SEQ ID NO: 92 or a nucleotide sequence having a sequence identity of 90% or more with the nucleotide sequence of SEQ ID NO: 92; and a primer containing the nucleotide sequence of SEQ ID NO: 93 or a nucleotide sequence having a sequence identity of 90% or more with the nucleotide sequence of SEQ ID NO: 93,
the set of primers specific to the full-length or a partial region of the *mecA* gene of *Staphylococcus aureus* may contain: a primer containing the nucleotide sequence of SEQ ID NO: 94 or a nucleotide sequence having a sequence identity of 90% or more with the nucleotide sequence of SEQ ID NO: 94; and a primer containing the nucleotide sequence of SEQ ID NO: 95 or a nucleotide sequence having a sequence identity of 90% or more with the nucleotide sequence of SEQ ID NO: 95,
the set of primers specific to the full-length or a partial region of the *vanA* gene of *Enterococcus faecalis* may contain: a primer containing the nucleotide sequence of SEQ ID NO: 96 or a nucleotide sequence having a sequence identity of 90% or more with the nucleotide sequence of SEQ ID NO: 96; and a primer containing the nucleotide sequence of SEQ ID NO: 97 or a nucleotide sequence having a sequence identity of 90% or more with the nucleotide sequence of SEQ ID NO: 97, and
the set of primers specific to the full-length or a partial region of the *vanB* gene of *Enterococcus faecium* may contain: a primer containing the nucleotide sequence of SEQ ID NO: 98 or a nucleotide sequence having a sequence identity of 90% or more with the nucleotide sequence of SEQ ID NO: 98; and a primer containing the nucleotide sequence of SEQ ID NO: 99 or a nucleotide sequence having a sequence identity of 90% or more with the nucleotide sequence of SEQ ID NO: 99.

The use of a combination of the set of primers as mentioned above improves specificity and reactivity in performing a PCR method. In addition, the use of a combination of the set of primers as mentioned above improves the amplification efficiency when a PCR method is performed in conditions equivalent in, e.g., temperature and reagent.

In the step of performing a PCR method, the DNA of the full-length or a partial region of the *gyrB* gene of *Escherichia coli* may contain the nucleotide sequence of SEQ ID NO: 51 or a nucleotide sequence having a sequence identity of 90% or more with the nucleotide sequence of SEQ ID NO: 51,
the DNA of the full-length or a partial region of the *nuc* gene of *Staphylococcus aureus* may contain the nucleotide sequence of SEQ ID NO: 61 or a nucleotide sequence having a sequence identity of 90% or more with the nucleotide sequence of SEQ ID NO: 61,
the DNA of the full-length or a partial region of the *atlE* gene of *Staphylococcus epidermidis* may contain the nucleotide sequence of SEQ ID NO: 63 or a nucleotide sequence having a sequence identity of 90% or more with the nucleotide sequence of SEQ ID NO: 63,
the DNA of the full-length or a partial region of the *gyrB* gene of *Klebsiella pneumoniae* may contain the nucleotide sequence of SEQ ID NO: 53 or a nucleotide sequence having a sequence identity of 90% or more with the nucleotide sequence of SEQ ID NO: 53,
the DNA of the full-length or a partial region of the *rpoB* gene of *Enterococcus* spp. may contain the nucleotide sequence of SEQ ID NO: 59 or a nucleotide sequence having a sequence identity of 90% or more with the nucleotide sequence of SEQ ID NO: 59,
the DNA of the full-length or a partial region of the *rpoB* gene of *Acinetobacter* spp. may contain the nucleotide sequence of SEQ ID NO: 47 or a nucleotide sequence having a sequence identity of 90% or more with the nucleotide sequence of SEQ ID NO: 47,
the DNA of the full-length or a partial region of the *16S-23S ITS* gene of *Acinetobacter baumannii* may contain the nucleotide sequence of SEQ ID NO: 48 or a nucleotide sequence having a sequence identity of 90% or more with the nucleotide sequence of SEQ ID NO: 48,
the DNA of the full-length or a partial region of the *ompa* gene of *Citrobacter* spp. may contain the nucleotide sequence of SEQ ID NO: 49 or a nucleotide sequence having a sequence identity of 90% or more with the nucleotide sequence of SEQ ID NO: 49,
the DNA of the full-length or a partial region of the *rpoS* gene of *Enterobacter* spp. may contain the nucleotide sequence of SEQ ID NO: 50 or a nucleotide sequence having a sequence identity of 90% or more with the nucleotide sequence of SEQ ID NO: 50,
the DNA of the full-length or a partial region of the *gyrB* gene of *Klebsiella aerogenes* may contain the nucleotide sequence of SEQ ID NO: 52 or a nucleotide sequence having a sequence identity of 90% or more with the nucleotide sequence of SEQ ID NO: 52,
the DNA of the full-length or a partial region of the *pehX* gene of *Klebsiella oxytoca* may contain the nucleotide sequence of SEQ ID NO: 54 or a nucleotide sequence having a sequence identity of 90% or more with the nucleotide sequence of SEQ ID NO: 54,
the DNA of the full-length or a partial region of the *gyrB* gene of *Klebsiella variicola* may contain the nucleotide sequence of SEQ ID NO: 55 or a nucleotide sequence having a sequence identity of 90% or more with the nucleotide sequence of SEQ ID NO: 55,
the DNA of the full-length or a partial region of the *oprL* gene of *Pseudomonas aeruginosa* may contain the nucleotide sequence of SEQ ID NO: 56 or a nucleotide sequence having a sequence identity of 90% or more with the nucleotide sequence of SEQ ID NO: 56,
the DNA of the full-length or a partial region of the *rpoB* gene of *Proteus* spp. may contain the nucleotide sequence of SEQ ID NO: 57 or a nucleotide sequence having a sequence identity of 90% or more with the nucleotide sequence of SEQ ID NO: 57,
the DNA of the full-length or a partial region of the *hasA* gene of *Serratia marcescens* may contain the nucleotide sequence of SEQ ID NO: 58 or a nucleotide sequence having a sequence identity of 90% or more with the nucleotide sequence of SEQ ID NO: 58,
the DNA of the full-length or a partial region of the *iap p60* gene of *Listeria* spp. may contain the nucleotide sequence of SEQ ID NO: 60 or a nucleotide sequence having a sequence identity of 90% or more with the nucleotide sequence of SEQ ID NO: 60,
the DNA of the full-length or a partial region of the *nuc* gene of *Staphylococcus argenteus* may contain the nucleotide sequence of SEQ ID NO: 62 or a nucleotide sequence having a sequence identity of 90% or more with the nucleotide sequence of SEQ ID NO: 62,
the DNA of the full-length or a partial region of the *tuf* gene of *Staphylococcus* spp. may contain the nucleotide sequence of SEQ ID NO: 64 or a nucleotide sequence having a sequence identity of 90% or more with the nucleotide sequence of SEQ ID NO: 64,
the DNA of the full-length or a partial region of the *rnpB* gene of *Streptococcus* spp. may contain the nucleotide sequence of SEQ ID NO: 65 or a nucleotide sequence having a sequence identity of 90% or more with the nucleotide sequence of SEQ ID NO: 65,
the DNA of the full-length or a partial region of the *xisco* gene of *Streptococcus pneumoniae* may contain the nucleotide sequence of SEQ ID NO: 66 or a nucleotide sequence having a sequence identity of 90% or more with the nucleotide sequence of SEQ ID NO: 66,
the DNA of the full-length or a partial region of the *gyrB* gene of *Streptococcus pyogenes* may contain the nucleotide sequence of SEQ ID NO: 67 or a nucleotide sequence having a sequence identity of 90% or more with the nucleotide sequence of SEQ ID NO: 67,
the DNA of the full-length or a partial region of the *beta-lactamase_KPC* gene of *Klebsiella pneumoniae* may contain the nucleotide sequence of SEQ ID NO: 100 or a nucleotide sequence having a sequence identity of 90% or more with the nucleotide sequence of SEQ ID NO: 100,
the DNA of the full-length or a partial region of the *beta-lactamase_NDM* gene of *Klebsiella pneumoniae* may contain the nucleotide sequence of SEQ ID NO: 101 or a nucleotide sequence having a sequence identity of 90% or more with the nucleotide sequence of SEQ ID NO: 101,
the DNA of the full-length or a partial region of the *beta-lactamase_IMP85* gene of *Pseudomonas aeruginosa* may contain the nucleotide sequence of SEQ ID NO: 102 or a nucleotide sequence having a sequence identity of 90% or more with the nucleotide sequence of SEQ ID NO: 102,
the DNA of the full-length or a partial region of the *beta-lactamase_VIM* gene of *Citrobacter freundii* may contain the nucleotide sequence of SEQ ID NO: 103 or a nucleotide sequence having a sequence identity of 90% or more with the nucleotide sequence of SEQ ID NO: 103,
the DNA of the full-length or a partial region of the *beta-lactamase_CTX-M1* gene of *Salmonella enterica* may contain the nucleotide sequence of SEQ ID NO: 104 or a nucleotide sequence having a sequence identity of 90% or more with the nucleotide sequence of SEQ ID NO: 104,
the DNA of the full-length or a partial region of the *beta-lactamase_CTX-M2* gene of *Salmonella enterica* may contain the nucleotide sequence of SEQ ID NO: 105 or a nucleotide sequence having a sequence identity of 90% or more with the nucleotide sequence of SEQ ID NO: 105,
the DNA of the full-length or a partial region of the *beta-lactamase_CTX-M8* gene of *Citrobacter amalonaticus* may contain the nucleotide sequence of SEQ ID NO: 106 or a nucleotide sequence having a sequence identity of 90% or more with the nucleotide sequence of SEQ ID NO: 106,
the DNA of the full-length or a partial region of the *beta-lactamase*_*CTX-M25* gene of *Escherichia coli* may contain the nucleotide sequence of SEQ ID NO: 107 or a nucleotide sequence having a sequence identity of 90% or more with the nucleotide sequence of SEQ ID NO: 107,
the DNA of the full-length or a partial region of the *beta-lactamase_CTX-M9* gene of *Escherichia coli* may contain the nucleotide sequence of SEQ ID NO: 108 or a nucleotide sequence having a sequence identity of 90% or more with the nucleotide sequence of SEQ ID NO: 108,
the DNA of the full-length or a partial region of the *beta-lactamase_OXA-23* gene of *Acinetobacter baumannii* may contain the nucleotide sequence of SEQ ID NO: 109 or a nucleotide sequence having a sequence identity of 90% or more with the nucleotide sequence of SEQ ID NO: 109,
the amplified DNA of the full-length or a partial region of the *beta-lactamase_OXA-40* gene of *Acinetobacter baumannii* may contain the nucleotide sequence of SEQ ID NO: 110 or a nucleotide sequence having a sequence identity of 90% or more with the nucleotide sequence of SEQ ID NO: 110,
the amplified DNA of the full-length or a partial region of the *beta-lactamase_OXA-48* gene of *Klebsiella pneumoniae* may contain the nucleotide sequence of SEQ ID NO: 111 or a nucleotide sequence having a sequence identity of 90% or more with the nucleotide sequence of SEQ ID NO: 111,
the DNA of the full-length or a partial region of the *beta-lactamase-OXA-58* gene of *Acinetobacter haemolyticus* may contain the nucleotide sequence of SEQ ID NO: 112 or a nucleotide sequence having a sequence identity of 90% or more with the nucleotide sequence of SEQ ID NO: 112,
the DNA of the full-length or a partial region of the *mecA* gene of *Staphylococcus aureus* may contain the nucleotide sequence of SEQ ID NO: 113 or a nucleotide sequence having a sequence identity of 90% or more with the nucleotide sequence of SEQ ID NO: 113,
the DNA of the full-length or a partial region of the *vanA* gene of *Enterococcus faecalis* may contain the nucleotide sequence of SEQ ID NO: 114 or a nucleotide sequence having a sequence identity of 90% or more with the nucleotide sequence of SEQ ID NO: 114, and
the DNA of the full-length or a partial region of the *vanB* gene of *Enterococcus faecium* may contain the nucleotide sequence of SEQ ID NO: 115 or a nucleotide sequence having a sequence identity of 90% or more with the nucleotide sequence of SEQ ID NO: 115.

The use of DNAs of the full-length or the partial region reduces the interaction among the DNAs and improves specificity and reactivity in detecting the presence or absence of amplification of each region.

In the identification step, the probe specific to amplified DNA of the full-length or a partial region of the *gyrB* gene of *Escherichia coli* may contain the nucleotide sequence of SEQ ID NO: 121 or a nucleotide sequence having a sequence identity of 90% or more with the nucleotide sequence of SEQ ID NO: 121,
the probe specific to DNA of the full-length or a partial region of the *nuc* gene of *Staphylococcus aureus* may contain the nucleotide sequence of SEQ ID NO: 134 or a nucleotide sequence having a sequence identity of 90% or more with the nucleotide sequence of SEQ ID NO: 134,
the probe specific to DNA of the full-length or a partial region of the *atlE* gene of *Staphylococcus epidermidis* may contain the nucleotide sequence of SEQ ID NO: 136 or a nucleotide sequence having a sequence identity of 90% or more with the nucleotide sequence of SEQ ID NO: 136,
the probe specific to DNA of the full-length or a partial region of the *gyrB* gene of *Klebsiella pneumoniae* may contain the nucleotide sequence of SEQ ID NO: 124 or a nucleotide sequence having a sequence identity of 90% or more with the nucleotide sequence of SEQ ID NO: 124,
the probe specific to DNA of the full-length or the partial region of the *rpoB* gene of *Enterococcus* spp. may be selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof,
the probe specific to DNA of the full-length or a partial region of the *rpoB* gene of *Acinetobacter* spp. may be selected from a probe containing the nucleotide sequence of SEQ ID NO: 116 or a nucleotide sequence having a sequence identity of 90% or more with the nucleotide sequence of SEQ ID NO: 116, a probe containing the nucleotide sequence of SEQ ID NO: 117 or a nucleotide sequence having a sequence identity of 90% or more with the nucleotide sequence of SEQ ID NO: 117 and a combination thereof,
the probe specific to DNA of the full-length or a partial region of the *16S-23S ITS* gene of *Acinetobacter baumannii* may contain the nucleotide sequence of SEQ ID NO: 118 or a nucleotide sequence having a sequence identity of 90% or more with the nucleotide sequence of SEQ ID NO: 118,
the probe specific to DNA of the full-length or a partial region of the *ompa* gene of *Citrobacter* spp. may contain the nucleotide sequence of SEQ ID NO: 119 or a nucleotide sequence having a sequence identity of 90% or more with the nucleotide sequence of SEQ ID NO: 119,
the probe specific to DNA of the full-length or a partial region of the *rpoS* gene of *Enterobacter* spp. may contain the nucleotide sequence of SEQ ID NO: 120 or a nucleotide sequence having a sequence identity of 90% or more with the nucleotide sequence of SEQ ID NO: 120,
the probe specific to DNA of the full-length or a partial region of the *gyrB* gene of *Klebsiella aerogenes* may contain the nucleotide sequence of SEQ ID NO: 122 or a nucleotide sequence having a sequence identity of 90% or more with the nucleotide sequence of SEQ ID NO: 122,
the probe specific to DNA of the full-length or a partial region of the *pehX* gene of *Klebsiella oxytoca* may contain the nucleotide sequence of SEQ ID NO: 123 or a nucleotide sequence having a sequence identity of 90% or more with the nucleotide sequence of SEQ ID NO: 123,
the probe specific to DNA of the full-length or a partial region of the *gyrB* gene of *Klebsiella variicola* may contain the nucleotide sequence of SEQ ID NO: 125 or a nucleotide sequence having a sequence identity of 90% or more with the nucleotide sequence of SEQ ID NO: 125,
the probe specific to DNA of the full-length or a partial region of the *oprL* gene of *Pseudomonas aeruginosa* may contain the nucleotide sequence of SEQ ID NO: 126 or a nucleotide sequence having a sequence identity of 90% or more with the nucleotide sequence of SEQ ID NO: 126,
the probe specific to DNA of the full-length or a partial region of the *rpoB* gene of *Proteus* spp. may contain the nucleotide sequence of SEQ ID NO: 127 or a nucleotide sequence having a sequence identity of 90% or more with the nucleotide sequence of SEQ ID NO: 127,
the probe specific to DNA of the full-length or a partial region of the *hasA* gene of *Serratia marcescens* may contain the nucleotide sequence of SEQ ID NO: 128 or a nucleotide sequence having a sequence identity of 90% or more with the nucleotide sequence of SEQ ID NO: 128,
the probe specific to DNA of the full-length or a partial region of the *iap p60* gene of *Listeria* spp. may be selected from a probe containing the nucleotide sequence of SEQ ID NO: 132 or a nucleotide sequence having a sequence identity of 90% or more with the nucleotide sequence of SEQ ID NO: 132, a probe containing the nucleotide sequence of SEQ ID NO: 133 or a nucleotide sequence having a sequence identity of 90% or more with the nucleotide sequence of SEQ ID NO: 133 and a combination thereof,
the probe specific to DNA of the full-length or a partial region of the *nuc* gene of *Staphylococcus argenteus* may contain the nucleotide sequence of SEQ ID NO: 135 or a nucleotide sequence having a sequence identity of 90% or more with the nucleotide sequence of SEQ ID NO: 135,
the probe specific to DNA of the full-length or a partial region of the *tuf* gene of *Staphylococcus* spp. may contain the nucleotide sequence of SEQ ID NO: 137 or a nucleotide sequence having a sequence identity of 90% or more with the nucleotide sequence of SEQ ID NO: 137,
the probe specific to DNA of the full-length or a partial region of the *rnpB* gene of *Streptococcus* spp. may contain the nucleotide sequence of SEQ ID NO: 140 or a nucleotide sequence having a sequence identity of 90% or more with the nucleotide sequence of SEQ ID NO: 140,
the probe specific to DNA of the full-length or a partial region of the *xisco* gene of *Streptococcus pneumoniae* may contain the nucleotide sequence of SEQ ID NO: 138 or a nucleotide sequence having a sequence identity of 90% or more with the nucleotide sequence of SEQ ID NO: 138,
the probe specific to DNA of the full-length or a partial region of the *gyrB* gene of *Streptococcus pyogenes* may contain the nucleotide sequence of SEQ ID NO: 139 or a nucleotide sequence having a sequence identity of 90% or more with the nucleotide sequence of SEQ ID NO: 139,
the probe specific to DNA of the full-length or a partial region of the *beta-lactamase_KPC* gene of *Klebsiella pneumoniae* may contain the nucleotide sequence of SEQ ID NO: 142 or a nucleotide sequence having a sequence identity of 90% or more with the nucleotide sequence of SEQ ID NO: 142,
the probe specific to DNA of the full-length or a partial region of the *beta-lactamase_NDM* gene of *Klebsiella pneumoniae* may contain the nucleotide sequence of SEQ ID NO: 141 or a nucleotide sequence having a sequence identity of 90% or more with the nucleotide sequence of SEQ ID NO: 141,
the probe specific to DNA of the full-length or a partial region of the *beta-lactamase_IMP85* gene of *Pseudomonas aeruginosa* may contain the nucleotide sequence of SEQ ID NO: 144 or a nucleotide sequence having a sequence identity of 90% or more with the nucleotide sequence of SEQ ID NO: 144,
the probe specific to DNA of the full-length or a partial region of the *beta-lactamase_VIM* gene of *Citrobacter freundii* may contain the nucleotide sequence of SEQ ID NO: 143 or a nucleotide sequence having a sequence identity of 90% or more with the nucleotide sequence of SEQ ID NO: 143,
the probe specific to DNA of the full-length or a partial region of the *beta-lactamase_CTX-M1* gene of *Salmonella enterica* may contain the nucleotide sequence of SEQ ID NO: 149 or a nucleotide sequence having a sequence identity of 90% or more with the nucleotide sequence of SEQ ID NO: 149,
the probe specific to DNA of the full-length or a partial region of the *beta-lactamase_CTX-M2* gene of *Salmonella enterica* may contain the nucleotide sequence of SEQ ID NO: 150 or a nucleotide sequence having a sequence identity of 90% or more with the nucleotide sequence of SEQ ID NO: 150,
the probe specific to DNA of the full-length or a partial region of the *beta-lactamase_CTX-M8* gene of *Citrobacter amalonaticus* may contain the nucleotide sequence of SEQ ID NO: 151 or a nucleotide sequence having a sequence identity of 90% or more with the nucleotide sequence of SEQ ID NO: 151,
the probe specific to DNA of the full-length or a partial region of the *beta-lactamase*_*CTX-M25* gene of *Escherichia coli* may contain the nucleotide sequence of SEQ ID NO: 153 or a nucleotide sequence having a sequence identity of 90% or more with the nucleotide sequence of SEQ ID NO: 153,
the probe specific to DNA of the full-length or a partial region of the *beta-lactamase_CTX-M9* gene of *Escherichia coli* may contain the nucleotide sequence of SEQ ID NO: 152 or a nucleotide sequence having a sequence identity of 90% or more with the nucleotide sequence of SEQ ID NO: 152,
the probe specific to DNA of the full-length or a partial region of the *beta-lactamase_OXA-23* gene of *Acinetobacter baumannii* may contain the nucleotide sequence of SEQ ID NO: 145 or a nucleotide sequence having a sequence identity of 90% or more with the nucleotide sequence of SEQ ID NO: 145,
the probe specific to DNA of the full-length or a partial region of the *beta-lactamase_OXA-40* gene of *Acinetobacter baumannii* may contain the nucleotide sequence of SEQ ID NO: 146 or a nucleotide sequence having a sequence identity of 90% or more with the nucleotide sequence of SEQ ID NO: 146,
the probe specific to DNA of the full-length or a partial region of the *beta-lactamase_OXA-48* gene of *Klebsiella pneumoniae* may contain the nucleotide sequence of SEQ ID NO: 147 or a nucleotide sequence having a sequence identity of 90% or more with the nucleotide sequence of SEQ ID NO: 147,
the probe specific to DNA of the full-length or a partial region of the *beta-lactamase-OXA-58* gene of *Acinetobacter haemolyticus* may contain the nucleotide sequence of SEQ ID NO: 148 or a nucleotide sequence having a sequence identity of 90% or more with the nucleotide sequence of SEQ ID NO: 148,
the probe specific to DNA of the full-length or a partial region of the *mecA* gene of *Staphylococcus aureus* may contain the nucleotide sequence of SEQ ID NO: 154 or a nucleotide sequence having a sequence identity of 90% or more with the nucleotide sequence of SEQ ID NO: 154,
the probe specific to DNA of the full-length or a partial region of the *vanA* gene of *Enterococcus faecalis* may contain the nucleotide sequence of SEQ ID NO: 155 or a nucleotide sequence having a sequence identity of 90% or more with the nucleotide sequence of SEQ ID NO: 155, and
the probe specific to DNA of the full-length or a partial region of the *vanB* gene of *Enterococcus faecium* may contain the nucleotide sequence of SEQ ID NO: 156 or a nucleotide sequence having a sequence identity of 90% or more with the nucleotide sequence of SEQ ID NO: 156.

The use of probes as mentioned above reduces the interaction among the probes and improves the specificity and reactivity in detecting the presence or absence of amplification of each region. And simultaneously, the use of probes as mentioned above improves the hybridization efficiency when the hybridization reactions are performed in conditions equivalent in, e.g., temperature and reagent.

As specific elements in the method according to the embodiment, the specific elements as mentioned above are applicable without limit.

### <Kit>

The present invention provides, as an embodiment, a kit for identifying causative bacteria of sepsis, comprising:
a first reagent containing sets of primers for PCR each specific to the full-length or a partial region of each of the *gyrB* gene of *Escherichia coli,* the nuc gene of *Staphylococcus aureus,* the *atlE* gene of *Staphylococcus epidermidis,* the *gyrB* gene of *Klebsiella pneumoniae* and the *rpoB* gene of *Enterococcus* spp.; and
a second reagent containing a combination of probes each specific to DNA of the full-length or the partial region of each of the genes.

The step of performing a PCR method according to <Identification method> above can be performed by using the first reagent. The first reagent may further contain a set of primers specific to the full-length or a partial region of a gene other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp.

The first reagent may further contain, for example, sets of primers specific to the full-length or a partial region of one or more genes selected from the group consisting of the *rpoB* gene of *Acinetobacter* spp., *16S-23S ITS* gene of *Acinetobacter baumannii,* the *ompA* gene of *Citrobacter* spp., the *rpoS* gene of *Enterobacter* spp., the *gyrB* gene of *Klebsiella aerogenes,* the *pehX* gene of *Klebsiella oxytoca,* the *gyrB* gene of *Klebsiella variicola,* the *oprL* gene of *Pseudomonas aeruginosa,* the *rpoB* gene of *Proteus* spp., the *hasA* gene of *Serratia marcescens,* the *iap p60* gene of *Listeria* spp., the *nuc* gene of *Staphylococcus argenteus,* the *tuf* gene of *Staphylococcus* spp., the *rnpB* gene of *Streptococcus* spp., the *xisco* gene of *Streptococcus pneumoniae,* and the *gyrB* gene of *Streptococcus pyogenes.*

The first reagent may further contain, for example, sets of primers specific to the full-length or a partial region of one or more genes as antimicrobial resistance genes, selected from the group consisting of the *beta-lactamase_KPC* gene of *Klebsiella pneumoniae,* the *beta-lactamase_NDM* gene of *Klebsiella pneumoniae,* the *beta-lactamase_IMP85* gene of *Pseudomonas aeruginosa,* the *beta-lactamase_VIM* gene of *Citrobacter freundii,* the *beta-lactamase_CTX-M1* gene of *Salmonella enterica,* the *beta-lactamase_CTX-M2* gene of *Salmonella enterica,* the *beta-lactamase_CTX-M8* gene of *Citrobacter amalonaticus,* the *beta-lactamase*_*CTX-M25* gene of *Escherichia coli,* the *beta-lactamase_CTX-M9* gene of *Escherichia coli,* the *beta-lactamase_OXA-23* gene of *Acinetobacter baumannii, beta-lactamase_OXA-40* gene of *Acinetobacter baumannii,* the *beta-lactamase_OXA-48* gene of *Klebsiella pneumoniae,* the *beta-lactamase_OXA-58* gene of *Acinetobacter haemolyticus,* the *mecA* gene of *Staphylococcus aureus, vanA* gene of *Enterococcus faecalis* and the *vanB* gene of *Enterococcus faecium.*

The first reagent may contain, for example, sets of primers specific to the full-length or a partial region of one or more genes selected from the group consisting of the *rpoB* gene of *Acinetobacter* spp., the *16S-23S ITS* gene of *Acinetobacter baumannii,* the *ompA* gene of *Citrobacter* spp., the *rpoS* gene of *Enterobacter* spp., the *gyrB* gene of *Klebsiella aerogenes,* the *pehX* gene of *Klebsiella oxytoca,* the *gyrB* gene of *Klebsiella variicola,* the *oprL* gene of *Pseudomonas aeruginosa,* the *rpoB* gene of *Proteus* spp., the *hasA* gene of *Serratia marcescens,* the *iap p60* gene of *Listeria* spp., the *nuc* gene of *Staphylococcus argenteus,* the *tuf* gene of *Staphylococcus* spp., the *rnpB* gene of *Streptococcus* spp., the *xisco* gene of *Streptococcus pneumoniae,* the *gyrB* gene of *Streptococcus pyogenes,* the *beta-lactamase_KPC* gene of *Klebsiella pneumoniae,* the *beta-lactamase _NDM* gene of *Klebsiella pneumoniae,* the *beta-lactamase_IMP85* gene of *Pseudomonas aeruginosa,* the *beta-lactamase_VIM* gene of *Citrobacter freundii,* the *beta-lactamase_CTX-M1* gene of *Salmonella enterica,* the *beta-lactamase_CTX-M2* gene of *Salmonella enterica,* the *beta-lactamase_CTX-M8* gene of *Citrobacter amalonaticus,* the *beta-lactamase*_*CTX-M25* gene of *Escherichia coli,* the *beta-lactamase_CTX-M9* gene of *Escherichia coli,* the *beta-lactamase_OXA-23* gene of *Acinetobacter baumannii, beta-lactamase_OXA-40* gene *of Acinetobacter baumannii,* the *beta-lactamase_OXA-48* gene of *Klebsiella pneumoniae,* the *beta-lactamase_OXA-58* gene of *Acinetobacter haemolyticus,* the *mecA* gene of *Staphylococcus aureus,* the *vanA* gene *of Enterococcus faecalis,* and the *vanB* gene of *Enterococcus faecium.*

The bacteria or genes selected from the group of the plurality of genes as mentioned above may be, for example, 2 types or more, 3 types or more, 5 types or more, 7 types or more, 10 types or more, 15 types or more, 20 types or more, 25 types or more, 28 types or more, 30 types or more, or 32 types or more. The bacteria or genes selected from the group of the plurality of genes as mentioned above may be, for example, 37 types or less, 34 types or less, 21 types or less, 16 types or less, or 11 types or less. The bacteria or genes selected from the group of the plurality of genes as mentioned above may be, for example, 2 to 40 types, 5 to 37 types, 10 to 37 types, or 15 to 25 types.

In the first reagent, for example, the set of primers specific to the full-length or a partial region of the *gyrB* gene of *Escherichia coli* may contain: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
the set of primers specific to the full-length or a partial region of the *nuc* gene of *Staphylococcus aureus* may contain: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
the set of primers specific to the full-length or a partial region of the *atlE* gene of *Staphylococcus epidermidis* may contain: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
the set of primers specific to the full-length or a partial region of the *gyrB* gene of *Klebsiella pneumoniae* may contain: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15, and
the set of primers specific to the full-length or a partial region of the *rpoB* gene of *Enterococcus* spp. may contain: one or more primers selected from a primer containing the nucleotide sequence of SEQ ID NO: 26 or a nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29.

The use of sets of primers as mentioned above reduces the interaction among the primers and improves specificity and reactivity in amplifying each region. And simultaneously, the use of sets of primers as mentioned above improves the amplification efficiency when amplification is performed in conditions equivalent in, e.g., temperature and reagent.

The first reagent may further contain a set of primers specific to the full-length or a partial region of a gene other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp. Each of the primers is the same as defined in <Identification method>.

The DNA of the full-length or a partial region of the *gyrB* gene of *Escherichia coli* may contain the nucleotide sequence of SEQ ID NO: 51 or a nucleotide sequence having a sequence identity of 90% or more with the nucleotide sequence of SEQ ID NO: 51,
the DNA of the full-length or a partial region of the *nuc* gene of *Staphylococcus aureus* may contain the nucleotide sequence of SEQ ID NO: 61 or a nucleotide sequence having a sequence identity of 90% or more with the nucleotide sequence of SEQ ID NO: 61,
the DNA of the full-length or a partial region of the *atlE* gene of *Staphylococcus epidermidis* may contain the nucleotide sequence of SEQ ID NO: 63 or a nucleotide sequence having a sequence identity of 90% or more with the nucleotide sequence of SEQ ID NO: 63,
the DNA of the full-length or a partial region of the *gyrB* gene of *Klebsiella pneumoniae* may contain the nucleotide sequence of SEQ ID NO: 53 or a nucleotide sequence having a sequence identity of 90% or more with the nucleotide sequence of SEQ ID NO: 53, and
the DNA of the full-length or a partial region of the *rpoB* gene of *Enterococcus* spp. may contain the nucleotide sequence of SEQ ID NO: 59 or a nucleotide sequence having a sequence identity of 90% or more with the nucleotide sequence of SEQ ID NO: 59.

The use of DNAs of the full-length or the partial region reduces the interaction among the DNAs and improves specificity and reactivity in detecting presence or absence of amplification of each region.

In the case where the full-length or a partial region of a gene other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp. is subjected to a PCR method using the first reagent, the DNA of the full-length or a partial region of each gene is the same as defined in <Identification method>.

The second reagent contains probes each specific to DNA of the full-length or the partial region of each of the genes. The detection step according to <Identification method> above can be carried out by using the second reagent. To detect the presence or absence of amplification of the full-length or a partial region of each of a plurality of genes, probes are designed so as to detect DNAs of individual genes in similar or equivalent conditions. The probes each may hybridize with amplified DNA under stringent conditions. The stringent conditions are the same as defined in the <Identification method>. The second reagent may contain a combination of probes, for example, as a mixture or in an immobilized form onto a solid support. Examples of the solid support include a substrate, an array, magnetic beads, a column, and colored beads. A combination of probes may be immobilized onto a solid support in a multiplex format.

In the case where the probes contained in the second reagent are immobilized onto a solid support, the solid support may be acceptable as long as probes containing, for example, mutually distinguishable, different identifiers (for example, ID) are immobilized or it contains distinguishable identifiers on which probes are immobilized. In other words, probes may contain different identifiers or probes may be bound to different identifiers on a solid support.

In the second reagent, the probe specific to DNA of the full-length or a partial region of the *gyrB* gene of *Escherichia coli* may contain the nucleotide sequence of SEQ ID NO: 121 or a nucleotide sequence having a sequence identity of 90% or more with the nucleotide sequence of SEQ ID NO: 121,
the probe specific to DNA of the full-length or a partial region of the *nuc* gene of *Staphylococcus aureus* may contain the nucleotide sequence of SEQ ID NO: 134 or a nucleotide sequence having a sequence identity of 90% or more with the nucleotide sequence of SEQ ID NO: 134,
the probe specific to DNA of the full-length or a partial region of the *atlE* gene of *Staphylococcus epidermidis* may contain the nucleotide sequence of SEQ ID NO: 136 or a nucleotide sequence having a sequence identity of 90% or more with the nucleotide sequence of SEQ ID NO: 136,
the probe specific to DNA of the full-length or a partial region of the *gyrB* gene of *Klebsiella pneumoniae* may contain the nucleotide sequence of SEQ ID NO: 124 or a nucleotide sequence having a sequence identity of 90% or more with the nucleotide sequence of SEQ ID NO: 124, and
the probe specific to DNA of the full-length or a partial region of the *rpoB* gene of *Enterococcus* spp. may be selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof.

The use of probes as mentioned above reduces the interaction among the probes and improves specificity and reactivity in detecting individual regions. And simultaneously, the use of probes as mentioned above improves the hybridization efficiency when the hybridization reactions are performed in conditions equivalent in, e.g., temperature and reagent.

The second reagent may further contain a probe specific to DNA of the full-length or a partial region of a gene other than the *gyrB* gene of *Escherichia coli,* the *nuc* gene of *Staphylococcus aureus,* the *atlE* gene of *Staphylococcus epidermidis,* the *gyrB* gene of *Klebsiella pneumoniae,* and the *rpoB* gene of *Enterococcus* spp. Each of the probes is the same as defined in <Identification method>.

The kit according to the embodiment may contain a set of primers designed such that the DNA amplified by a PCR method using the set of primers contains a label. The kit may further contain a third reagent for labeling the amplified DNA. The primers designed such that the amplified DNA contains a label are the same as concretely mentioned in <Identification method>, and since the amplified DNA containing a label can be obtained by performing a PCR method using, for example, primers having the label, the primers may be designed so as to have a label at the 5' end and 3' end or internal portion. For example, the 5' end of the primers may be modified with biotin. The labeling of the amplified DNA is the same as concretely mentioned in <Identification method>, and for example, labeled avidin may be contained as the third reagent in order to label amplified DNA modified with biotin.

The kit according to the embodiment may further contain a means for collecting a sample from a subject. The means for collecting a sample varies depending on the type of sample, and examples of the means include a blood collection means (such as a blood collection kit) if the sample is blood and a urine collection container if the sample is urea.

The kit according to the embodiment may further contain an preparative agent for processing a sample collected from a control. Examples of the preparative agent include an agent causing cell lysis and an agent for extracting DNA.

The kit according to the embodiment may further contain a PCR standard reagent. The kit may contain one or more elements selected from the group consisting of DNA polymerase, deoxynucleoside triphosphates (dNTPs), a magnesium ion, one or more salts and a pH buffer. These reagents may be contained in the first reagent together with primers.

The kit according to the embodiment may further contain a labeling reagent for labeling amplified DNA and/or probes. Examples of the labeling reagent include labeled avidin.

The kit according to the embodiment may further contain a control (for example, a control sample) for use in comparison of detection results.

The kit according to the embodiment is used for identifying causative bacteria of sepsis and may further contain an instruction as to how to identify causative bacteria of sepsis or an instruction as to how to use reagents contained in the kit.

As other specific elements of the kit according to the embodiment, such as primers, a target region of each gene and probes, the specific elements set forth in, e.g., <Identification method> above, are applicable without limit.

The present invention also provides, as an embodiment, a kit for identifying causative bacteria of sepsis and/or antimicrobial resistance genes, comprising:
a first reagent containing sets of primers each specific to the full-length or a partial region of each of two types or more bacterial genes and/or antimicrobial resistance genes selected from the group consisting of the *gyrB* gene of *Escherichia coli,* the nuc gene of *Staphylococcus aureus,* the *atlE* gene of *Staphylococcus epidermidis,* the *gyrB* gene of *Klebsiella pneumoniae* and the *rpoB* gene of *Enterococcus* spp., the *rpoB* gene of *Acinetobacter* spp., the *16S-23S ITS* gene of *Acinetobacter baumannii,* the *ompA* gene of *Citrobacter* spp., the *rpoS* gene of *Enterobacter* spp., the *gyrB* gene of *Klebsiella aerogenes,* the *pehX* gene of *Klebsiella oxytoca,* the *gyrB* gene of *Klebsiella variicola,* the *oprL* gene of *Pseudomonas aeruginosa,* the *rpoB* gene of *Proteus* spp., the *hasA* gene of *Serratia marcescens,* the *iap p60* gene of *Listeria* spp., the *nuc* gene of *Staphylococcus argenteus,* the *tuf* gene of *Staphylococcus* spp., the *rnpB* gene of *Streptococcus* spp., the *xisco* gene of *Streptococcus pneumoniae,* the *gyrB* gene of *Streptococcus pyogenes,* the *beta-lactamase_KPC* gene of *Klebsiella pneumoniae,* the *beta-lactamase_NDM* gene of *Klebsiella pneumoniae,* the *beta-lactamase_IMP85* gene of *Pseudomonas aeruginosa,* the *beta-lactamase_VIM* gene of *Citrobacter freundii,* the *beta-lactamase_CTX-M1* gene of *Salmonella enterica,* the *beta-lactamase_CTX-M2* gene of *Salmonella enterica,* the *beta-lactamase_CTX-M8* gene of *Citrobacter amalonaticus,* the *beta-lactamase_CTX-M25* gene of *Escherichia coli,* the *beta-lactamase_CTX-M9* gene of *Escherichia coli,* the *beta-lactamase_OXA-23* gene of *Acinetobacter baumannii,* the *beta-lactamase_OXA-40* gene of *Acinetobacter baumannii,* the *beta-lactamase_OXA-48* gene of *Klebsiella pneumoniae,* the *beta-lactamase_OXA-58* gene of *Acinetobacter haemolyticus,* the *mecA* gene of *Staphylococcus aureus,* the *vanA* gene of *Enterococcus faecalis,* and the *vanB* gene of *Enterococcus faecium*; and
a second reagent containing a combination of probes each specific to DNA of the full-lengths or the partial region as mentioned above.

The bacteria or genes selected from the group of the plurality of genes as mentioned above may be, for example, 5 types or more, 10 types or more, 15 types or more, 20 types or more, 25 types or more, 28 types or more, 30 types or more, 32 types or more, 35 types or more, or 37 types. The bacterial genes selected from the group of the plurality of genes as mentioned above may be, for example, 3 types or more, 5 types or more, 7 types or more, 10 types or more, 12 types or more, 15 types or more, 17 types or more, 19 types or more, or 21 types or more. The antimicrobial resistance genes selected from the group of the plurality of genes as mentioned above may be, for example, 3 types or more, 5 types or more, 7 types or more, 10 types or more, 12 types or more, 14 types or more, or 16 types or more. The bacteria or genes selected from the group of the plurality of genes as mentioned above may be, for example, 37 types or less, 34 types or less, 21 types or less, 16 types or less, or 11 types or less. The bacteria or genes selected from the group of the plurality of genes as mentioned above may be, for example, 2 to 40 types, 5 to 37 types, 10 to 37 types, or 15 to 25 types.

As specific elements in the kit according to the embodiment, the specific elements mentioned above are applicable without limit.

### <Diagnostic method, method for assisting diagnosis and method for treatment of sepsis>

The present invention also provides, as an embodiment, a method for diagnosing sepsis or a method for assisting diagnosis thereof including identifying causative bacteria of sepsis. The method for identifying causative bacteria of sepsis is the same as defined in

### <Identification method>.

For example, the method for diagnosing sepsis according to the embodiment may include
an amplification step of performing a PCR method using a sample collected from a subject and a combination of sets of primers each specific to the full-length or a partial region of each of the *gyrB* gene of *Escherichia coli,* the *nuc* gene of *Staphylococcus aureus,* the *atlE* gene of *Staphylococcus epidermidis,* the *gyrB* gene of *Klebsiella pneumoniae* and the *rpoB* gene of *Enterococcus* spp.,
a step of detecting the presence or absence of amplification of the full-length or the partial region by using a combination of probes each specific to DNA of the full-length or the partial region, and
a step of diagnosing the subject as having sepsis or being suspected of having sepsis based on the identification of causative bacteria.

Also, for example, the method for assisting diagnosis of sepsis according to the embodiment may include
an amplification step of performing a PCR method using a sample collected from a subject and a combination of sets of primers each specific to the full-length or a partial region of each of the *gyrB* gene of *Escherichia coli,* the *nuc* gene of *Staphylococcus aureus,* the *atlE* gene of *Staphylococcus epidermidis,* the *gyrB* gene of *Klebsiella pneumoniae* and the *rpoB* gene of *Enterococcus* spp.,
a step of detecting the presence or absence of amplification of the full-length or the partial region by using a combination of probes each specific to DNA of the full-length or the partial region, and
a step of providing an indication that the subject has sepsis or is suspected of having sepsis based on the identification of causative bacteria.

Based on the identification of causative bacteria of sepsis, it is also possible to diagnose that the subject has sepsis or is suspected of having sepsis caused by the bacteria identified or to provide an indication that the subject has sepsis or is suspected of having sepsis caused by the bacteria identified for assisting diagnosis.

The diagnostic method or method for assisting diagnosis according to the embodiment can be performed *in vitro.*

The present invention also provides, as an embodiment, a method for treating sepsis, including identifying causative bacteria of sepsis. The method for identifying causative bacteria of sepsis is the same as defined in <Identification method>.

The treatment method of the embodiment may include a step of treating sepsis of a subject, who was diagnosed as having sepsis or being suspected of having sepsis or provided with an indication of sepsis, by the diagnostic method or method for assisting diagnosis mentioned above. The method for treatment may include a step of selecting an appropriate treatment based on the causative bacteria identified. If an antimicrobial resistance gene is also identified, a step of selecting an appropriate treatment based on causative bacteria and antimicrobial resistance genes identified may be included.

As the specific elements of the diagnostic method and method for assisting diagnosis and method of treatment according to the embodiment, specific elements as mentioned above are applicable without limit.

### Examples

### [Example 1 Development of primer and probe for detection of Escherichia coli]

Primers and probes for detecting bacterial species contained in the sepsis panel were developed for each bacterial species. A method for developing primers and probes for detecting genomic DNA of *Escherichia coli* used as a representative example will be more specifically set forth. Note that, the detection method according to Example 1 includes a step of amplifying a target gene of bacterial genomic DNA by PCR and a step of detecting a PCR product amplified by probes on a substrate.

### 1. Development and selection of primer

To amplify a predetermined region of the gene (*gyrB*) of *Escherichia coli* genomic DNA, two types of primer sets were designed as candidates. PCR reaction solutions were prepared separately using primer sets 1 and 2 listed in the following Table and *Escherichia coli*-derived genomic DNA, and subjected to amplification by real-time PCR using Light Cycler (registered trademark) 480 II (Roche). The reagent used for amplification was Takara multiplex assay kit Ver2 (Takara Bio Inc.), and as the intercalator dye, 20xEvaGreen (biotium) was used. Each primer was added so as to obtain a final concentration of 0.2 µM; 2xMultiplex PCR Buffer contained in Takara multiplex assay kit Ver2 was prepared to be 1x; and Multiplex PCR Enzyme Mix was used at a unit of 1 U, and then, *Escherichia coli* genomic DNA was mixed as a template to prepare a PCR reaction solution.

**[Table 4]**

| Primer set | Name of primer | Sequence (5' to 3') | SEQ ID NO: |
|---|---|---|---|
| 1 | E.coli_468F19 | GGTTACCGGCGAGACTGAA | 10 |
| | E.coli_567R18 | GACAACTCACGCAGACGT | 11 |
| 2 | E.coli_1969F17 | CAAAACCTGTTCGAGCC | 157 |
| | E.coli_2093R18 | CTTCTTCCAGCAAGCCAC | 158 |

An amplification reaction was carried out in the following conditions:
Denaturation step: at 94°C for 30 seconds
Annealing step: at 60°C for 60 seconds → at 94°C for 30 seconds (Number of cycles: 40)
Extension step: at 72°C for 600 seconds

The composition of the PCR reaction solution is listed in the following Table.

**[Table 5]**

| Composition of the PCR reaction solution | | |
|---|---|---|
| PCR mix | Amount used/uL | Final concentration |
| Multiplex PCR Enzyme Mix | 0.1 | 1 U |
| 2xMultiplex PCR Buffer | 10 | 1x |
| 20 uM Forward primer | 0.2 | 0.2 uM |
| 20 uM Reverse primer | 0.2 | 0.2 uM |
| Template | 2 | - |
| 20xEvaGreen | 1 | 1x |
| dH₂O | 6.5 | |
| total | 20 | |

### 2. Results

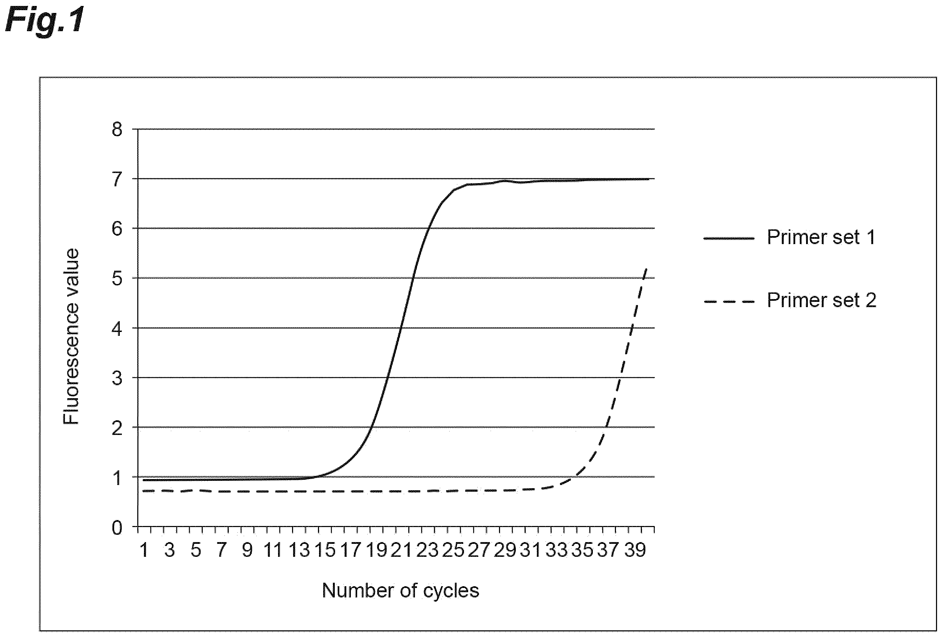

The results of amplification by primer sets 1 and 2 are shown by the amplification curves in Figure 1, respectively. As a result of a review using real-time PCR, it was found that the amplification by primer set 1 for an *Escherichia coli* template increases early, as shown by the amplification curve. From this, it was demonstrated that primer set 1 can amplify the target gene of *Escherichia coli.* The amplification by primer set 2 for an *Escherichia coli* template increased but late and did not reach a plateau, as shown by the amplification curve, and therefore, sufficient amplification of a PCR product was not observed. From this, it was demonstrated that in primer set 2, the function of the primers to selectively amplify target genes of *Escherichia coli* is insufficient. Based on the results, primer set 1 (*E. coli_*468F19/*E. coli_*567R18) was employed as the primers for identifying *Escherichia coli.*

### 3. Development and selection of probe

With respect to the probes for use in a step of detecting amplified PCR products by probes on a substrate, probes for use in identifying causative bacteria of sepsis were determined by evaluating a plurality of candidate probes as follows.

In the real-time PCR review, it was demonstrated that using primer set 1, a PCR product specific to a target gene of *Escherichia coli* can be obtained by performing PCR with *Escherichia coli* used as a template. As the probes for detecting the PCR product, E. *coli_53lP16* and *E*. *coli*_526P15 were designed.

Using primer set 1, amplification by PCR was performed with *Escherichia coli* used as a template, and then, a hybridization reaction with probes on a substrate, labeling and fluorescence measurement were performed. In this case, as a reverse primer, a primer modified with biotin at the 5' end side via a linker was used. The SEQ ID Nos and sequences of candidate probes are listed in the following Table. The measurement procedure is shown in the following section, Overview of detection step.

**[Table 6]**

| Name of probe | Sequence (5' to 3') | SEQ ID NO: |
|---|---|---|
| E.coli_531P16 | TTTTTTTTTCAATGTGACCGAGTTC | 121 |
| E.coli_526P15 | TTTTTTTTTTTTCACCAATGTGACC | 159 |

### Overview of detection step

### (1) PCR amplification reaction

A PCR amplification reaction was performed using the genomic DNA of *Escherichia coli* as a template in the following conditions.
Denaturation step: at 94°C for 30 seconds
Annealing step: at 60°C for 60 seconds → at 94°C for 30 seconds (Number of cycles: 30)
Extension step: at 72°C for 600 seconds

The composition of the PCR reaction solution is listed in the following Table.

**[Table 7]**

| Composition of the PCR reaction solution | | |
|---|---|---|
| PCR mix | Amount used/uL | Final concentration |
| Multiplex PCR Enzyme Mix | 0.1 | 1 U |
| 2×Multiplex PCR Buffer | 10 | 1x |
| 20 uM Forward primer | 0.2 | 0.2 uM |
| 20 uM Reverse primer | 0.2 | 0.2 uM |
| Template | 5 | - |
| dH₂O | 4.5 | |
| total | 20 | |

### (2) Thermal denaturation of PCR product

PCR products were thermally denatured in the following conditions before they were exposed to probes immobilized on a substrate.

Thermal denaturation conditions: at 95°C for 5 minutes, followed by rapid cooling to 4°C.

### (3) Hybridization reaction of probes on a substrate with PCR product, and labeling

Hybridization reactions of individual probes (see Table 6) on a substrate with PCR products were performed. The reaction solution contains Saline-sodium-phosphate-EDTA buffer (SSPE-buffer). The PCR products captured by probes immobilized onto a substrate were modified with biotin at the 5' end side and biotin was labeled with Streptavidin-Phycoerythrin (PlexBio Co., Ltd.). Hybridization reactions on a substrate and labeling were carried out by use of IntelliPlex 1000 πcode Processor, which is a device of PlexBio Co., Ltd., in the following conditions:
Hybridization conditions: incubation at 37°C for 20 minutes
Labeling conditions: incubation at 37°C for 10 minutes

### (4) Detection of labeled PCR product on substrate

Distinguishable IDs were provided onto each substrate and predetermined probes are immobilized to individual IDs. PCR products are captured by the corresponding probes on the substrate and labeled. The labeled PCR products on the substrate emit fluorescence, the values of which can be measured by a detector. The fluorescence values were measured by a fluorometer (100 Fluorescent Analyzer, PlexBio Co., Ltd.). Since 100 Fluorescent Analyzer of PlexBio Co., Ltd. can identify IDs of a substrate and measure a fluorescence value per substrate, the fluorescence value of a substrate having predetermined probes immobilized thereon can be measured to evaluate the reactivity of probes to PCR products.

### 4. Results

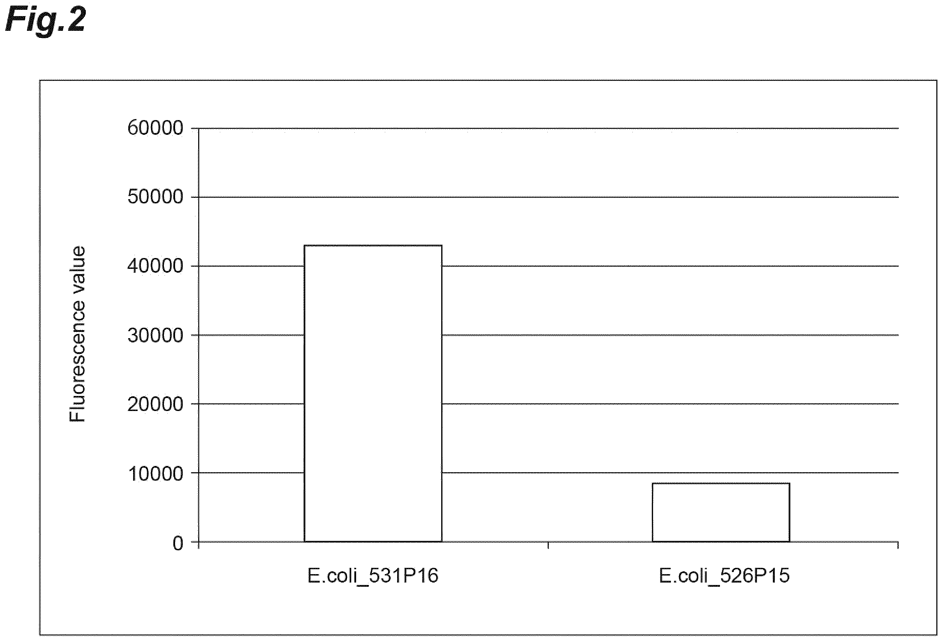

PCR products, which were obtained by using primer set 1 (*E. coli_*468F19/*E*. *coli_*567R18) and *Escherichia coli* genomic DNA as a template, were evaluated by two types of probes (*E. coli_53lP16* and *E*. *coli_*526P15) and the evaluation results are shown in Figure 2. *E. coli*_526P15 had a fluorescence value of 9000 or less while *E. coli*_531P16 showed a fluorescence value of 40000 or more. From the results, it was found that *a* PCR product of *Escherichia coli* can be detected with significance when *E. coli_*531P16 was used.

From the results, it was demonstrated that *Escherichia coli* can be detected by using primer set 1 and *E*. *coli*_531P16 as a probe.

### [Example 2 Development of primer and probe for detection of Citrobacter spp.]

A method for developing primers and probes for detecting genomic DNA of *Citrobacter* spp. used as another representative example will be more specifically set forth. The detection method of Example 2, similar to Example 1, includes a step of amplifying a target gene of bacterial genomic DNA by PCR and a step of detecting a PCR product amplified by probes on a substrate.

### 1. Development and selection of primer

To amplify a predetermined region of the DNA gene (*ompA*) of *Citrobacter* spp., three primer sets were designed as candidates. PCR reaction solutions were prepared using primer sets 1, 2 and 3 listed in the following Table and *Citrobacter freundii*-derived genomic DNA and *Citrobacter koseri-derived* genomic DNA each as a template, and subjected to real-time PCR amplification using Light Cycler (registered trademark) 480 II (Roche). The reagent used for amplification was Takara multiplex assay kit Ver2 (Takara Bio Inc.). As an intercalator dye, 20xEvaGreen (company: biotium) was used. Each primer was added so as to obtain a final concentration of 0.2 µM; genomic DNA used as a template was added in a concentration of 1 µg/L; 2xMultiplex PCR Buffer contained in Takara multiplex assay kit Ver2 was prepared to be 1×; Multiplex PCR Enzyme Mix was used at a unit of 1 U; and template DNAs were mixed to prepare a PCR reaction solution.

**[Table 8]**

| Primer set | Name of primer | Sequence (5' to 3') | SEQ ID NO: |
|---|---|---|---|
| 1 | C. spp_449F17 | GCTGCTTCTTCCTGCTG | 5 |
| | C. spp_551 R19 | GTCTGGAATACCAGTGGAC | 7 |
| 2 | C. spp_443F18S | CAGCTTSTTCCTGCTGAC | 6 |
| | C. spp_551R19 | GTCTGGAATACCAGTGGAC | 7 |
| 3 | C. spp_568F18 | TCYGATAGCCAGCCGATG | 160 |
| | C. spp(3)_670R18 | CATTGCGTGGGTCAGTTT | 161 |

An amplification reaction was performed in the following conditions:
Denaturation step: at 94°C for 30 seconds
Annealing step: at 60°C for 60 seconds → at 94°C for 30 seconds (Number of cycles: 40 times)
Extension step: at 72°C for 600 seconds

The composition of the PCR reaction solution is listed in the following Table.

**[Table 9]**

| Composition of the PCR reaction solution | | |
|---|---|---|
| PCR mix | Amount used/uL | Final concentration |
| Multiplex PCR Enzyme Mix | 0.1 | 1 U |
| 2×Multiplex PCR Buffer | 10 | 1× |
| 20 uM Forward primer | 0.2 | 0.2 uM |
| 20 uM Reverse primer | 0.2 | 0.2 uM |
| Template | 2 | - |
| 20×EvaGreen | 1 | 1x |
| dH₂O | 6.3 | |
| total | 20 | |

### 2. Results

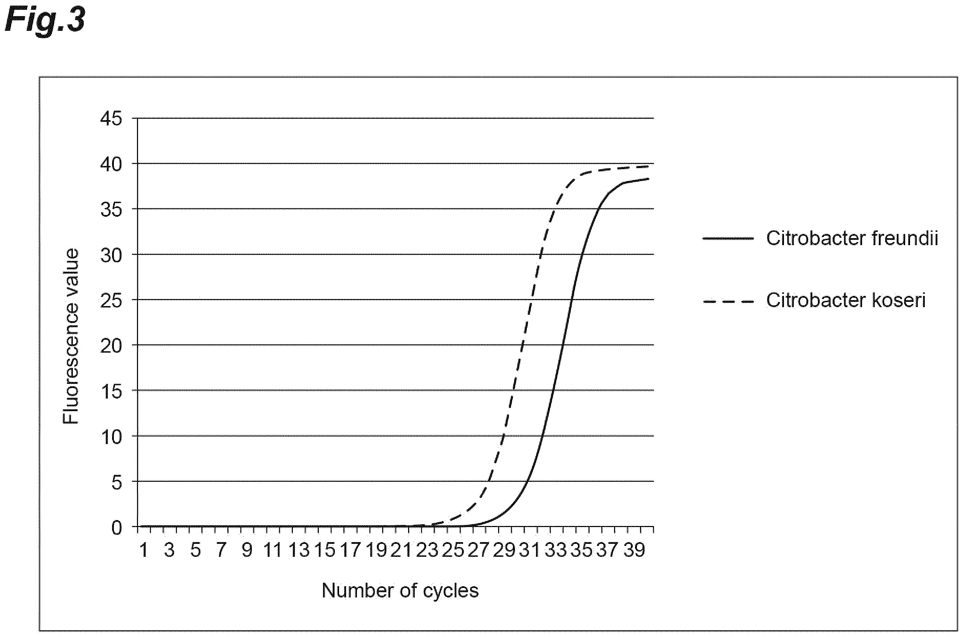

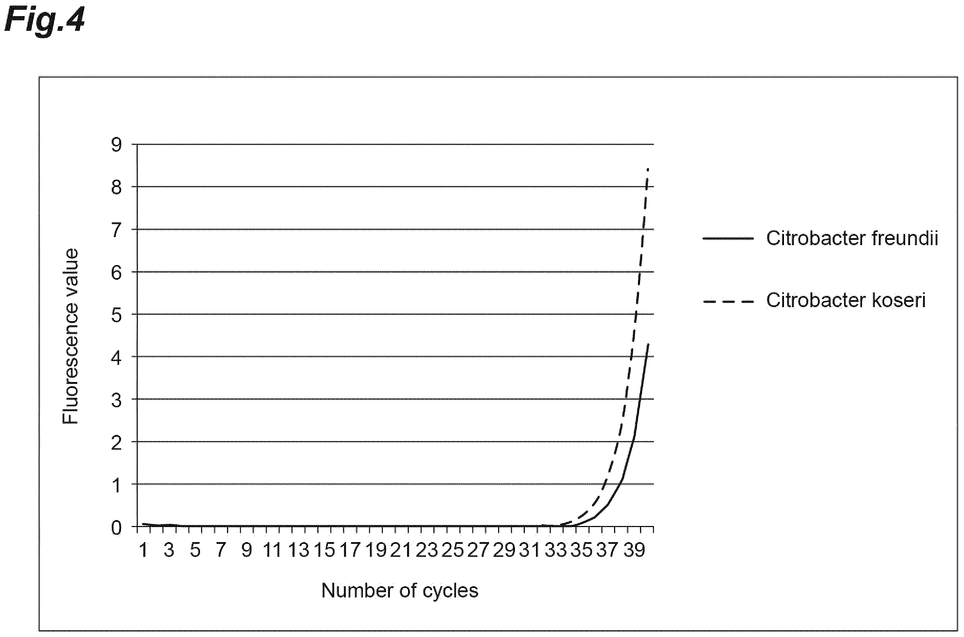

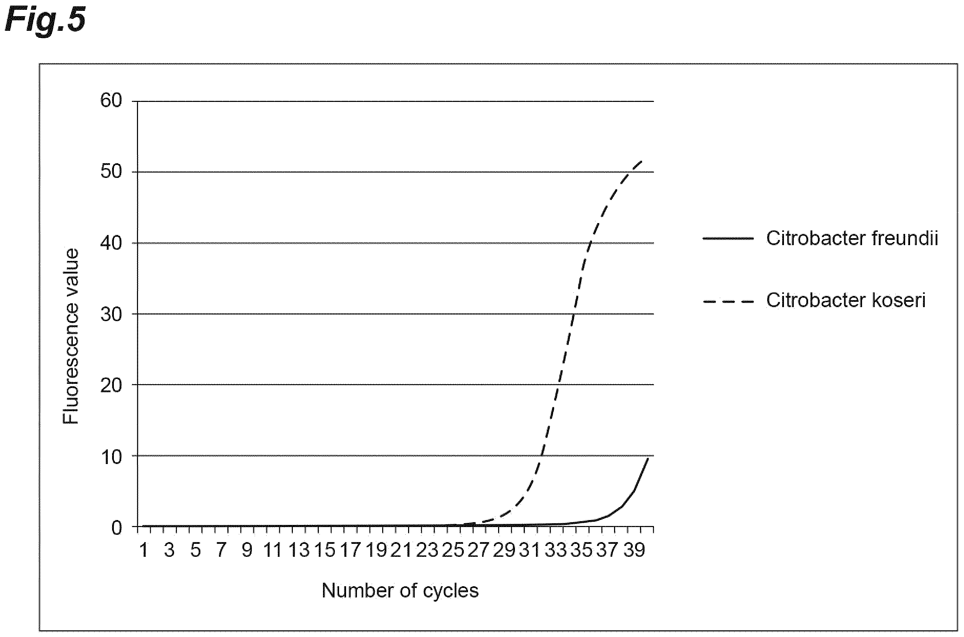

The amplification results of primer sets 1, 2 and 3 mentioned above used for respective templates are shown by amplification curves in Figures 3 to 5, respectively. As a result of a review using real-time PCR, it was found that amplification by primer set 1 for templates of *Citrobacter freundii* and *Citrobacter koseri* increases early, as shown by amplification curves. Since primer set 1 matches with target genes of *Citrobacter braakii, Citrobacter youngae, Citrobacter werkmanii* in consideration of sequence design, it was found that primer set 1 can amplify target genes of *Citrobacter* spp. Amplification by primer set 2 for *Citrobacter koseri* and *Citrobacter freundii* increased but relatively late and did not reach a plateau, as shown by the amplification curves. Amplification by primer set 3 increases selectively for *Citrobacter koseri* used as a template but sufficient amplification of a PCR product was not observed for *Citrobacter freundii* used as a template, as shown by the amplification curves. From this, it was demonstrated that in primer set 3, the function of the primers to selectively amplify target genes of *Citrobacter* spp. is insufficient.

The degree of binding of primer set 2 to any sequences of target genes of *Citrobacter freundii* and *Citrobacter koseri* was low but primer set 2 was designed such that it has a high degree of binding to any sequences of target genes of *Citrobacter* spp. except *Citrobacter freundii* and *Citrobacter koseri.* More specifically, owing to the use of primer set 1 and 2 together in a reaction system, any target genes of *Citrobacter* spp. can be comprehensively and selectively amplified. Three primers (C. spp._449F17, C. spp._443F18, C. spp._551R19) were employed for detecting the genomic DNA of *Citrobacter* spp.

### 3. Development and selection of probe

With respect to the probes for use in a step of detecting amplified PCR products by probes on a substrate, probes for use in identifying causative bacteria of sepsis were determined by evaluating a plurality of candidate probes as follows.

In the real-time PCR review, it was demonstrated that PCR products specific to *Citrobacter* spp can be obtained by performing PCR with *Citrobacter freundii* and *Citrobacter koseri* as templates. As the probes for detecting the PCR products, C. spp_24P17R, C. spp_55P15Y and C. spp_65P19R were designed.

Using Primer set 1, amplification by PCR with *Citrobacter freundii* and *Citrobacter koseri* as templates, and then, hybridization reaction with probes on a substrate, labeling, and fluorescence measurement were performed. In this case, as a reverse primer, a primer modified with biotin at the 5' end side via a linker was used. The SEQ ID NO: and sequence of candidate probes are listed in the following Table. The measurement procedure is shown in the following section, Overview of detection step.

**[Table 10]**

| Name of probe | Sequence (5' to 3') | SEQ ID NO: |
|---|---|---|
| C. spp_24P17R | TTTTTTTTGAAACGGTARGAAACAC | 119 |
| C. spp_55P15Y | TTTTTTTTTTCCGTTATCYGGACGA | 162 |
| C. spp_65P19R | TTTTTTGACGACCRCCAACGGTGTT | 163 |

### Overview of detection step

### (1) PCR amplification reaction

A PCR amplification reaction was performed using the genomic DNAs of *Citrobacter freundii* and *Citrobacter koseri* as templates in the following conditions.
Denaturation step: at 94°C for 30 seconds
Annealing step: at 60°C for 60 seconds → at 94°C for 30 seconds (Number of cycles: 30)
Extension step: at 72°C for 600 seconds

**[Table 11]**

| Composition of the PCR reaction solution | | |
|---|---|---|
| PCR mix | Amount used/uL | Final concentration |
| Multiplex PCR Enzyme Mix | 0.1 | 1 U |
| 2×Multiplex PCR Buffer | 10 | 1× |
| 20 uM Forward primer | 0.2 | 0.2 uM |
| 20 uM Reverse primer | 0.2 | 0.2 uM |
| Template | 5 | - |
| dH₂O | 4.5 | |
| total | 20 | |

### (2) Thermal denaturation of PCR product

PCR products were thermally denatured in the following conditions before they were exposed to probes immobilized on a substrate.

Thermal denaturation conditions: at 95°C for 5 minutes, followed by rapid cooling to 4°C.

### (3) Hybridization reaction of probes on a substrate with PCR product, and labeling

Hybridization reactions of individual probes (see Table 10) immobilized onto a substrate with PCR products were performed. The reaction solution contains Saline-sodium-phosphate-EDTA buffer (SSPE-buffer). The PCR products captured by probes on a substrate were modified with biotin at the 5' end side and biotin was labeled with Streptavidin-Phycoerythrin (PlexBio Co., Ltd.). Hybridization reactions on a substrate and labeling were carried out by use of IntelliPlex 1000 πcode Processor, which is a device of PlexBio Co., Ltd., in the following conditions:
Hybridization conditions: incubation at 37°C for 20 minutes
Labeling conditions: incubation at 37°C for 10 minutes

### (4) Detection of labeled PCR product on substrate

Distinguishable IDs were provided onto each substrate and predetermined probes are immobilized to individual IDs. PCR products are captured by the corresponding probes on the substrate and labeled. The labeled PCR products on the substrate emit fluorescence, the values of which can be measured by a detector. Fluorescence values were measured by a fluorometer (100 Fluorescent Analyzer, PlexBio Co., Ltd.) in the same manner as in Example 1.

### 4. Results

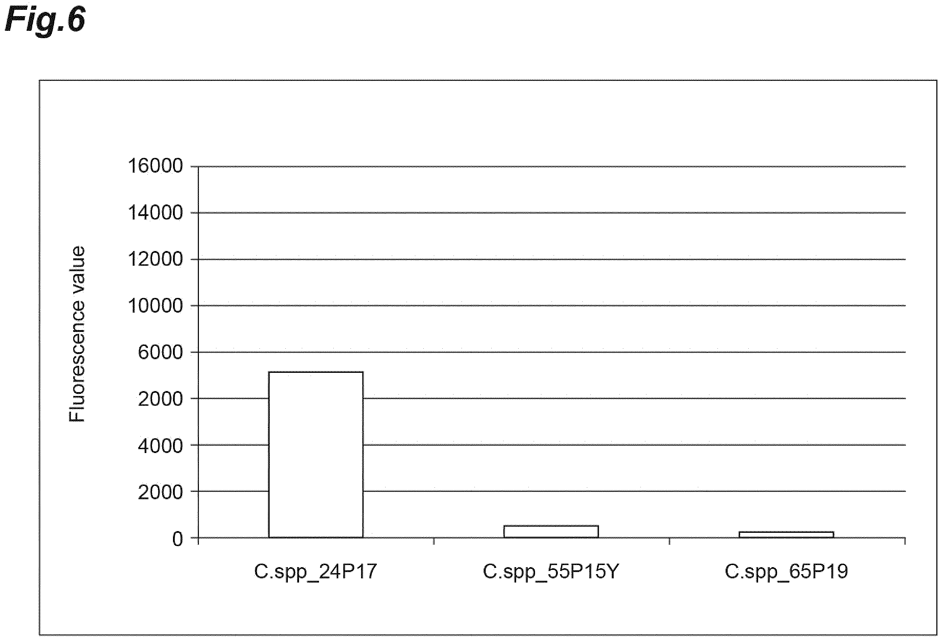

PCR products, which were obtained by using primer set 1 (C. spp_449F17R/C. spp_551R19R) and *Citrobacter freundii* and *Citrobacter koseri* genomic DNAs as templates, were evaluated by three probes (C. spp_24P17R, C. spp_55P15Y and C. spp_65P19R) and the evaluation results are shown in Figure 6. In *Citrobacter freundii* and *Citrobacter koseri,* C. spp_55P15Y and C. spp_65P19R had fluorescence values of 2000 or less while C. spp_24P17R showed a fluorescence value of 6000 or more. Note that, in the cases where any one of the probes was used with water as a template, fluorescence values were not obtained. From this, it was demonstrated that PCR products of *Citrobacter* spp. can be detected with significance when C. spp_24P17R was used.

From the results, it was found that *Citrobacter* spp. can be detected by using primer set 1 and C. spp_24P17R as a probe.

### [Example 3 Performance evaluation of primer and probe in multiplex system]

Based on the above review, *E*. *coli_*468F19/*E. coli_567R18* were selected as primers for detecting the genomic DNA of *Escherichia coli.* As a probe, probe *E. coli_*531P16 was selected based on the evaluation of reactivity. Further, C. spp_449F17, C. spp_443F18, and C. spp_551R19 were selected as primers for detecting the genomic DNAs of *Citrobacter* spp. As a probe, probe C. spp_24P17R was selected based on the evaluation of reactivity. With respect to bacterial species other than *Escherichia coli* and *Citrobacter* spp., primers and probes were developed in the same procedure. Primers and probes for simultaneously detecting bacterial antimicrobial resistance genes (AMR) were developed in the same procedure.

Primer sets selected for detecting target genes of bacteria and the sequences of the DNAs to be amplified by the primer sets are listed in Table 12.

**[Table 12]**

| Bacteria species | Name of target gene | Name of primer | Primer sequence (5' to 3') | SEQ ID NO: | DNA sequence to be amplified (biotinylated ReV chain notation) 5' to 3' | SEQ ID NO: |
|---|---|---|---|---|---|---|
| *Acinetobacter spp.* | rpoB | A.spp_3923 F21 | CAGCATTGCC AGAATAACTT TC | 1 | GATCCGTAAGAATTTTG GTAAATTGCCCCAAGTA ATGGATGCACCGTACTT ATTATCGATTCAGGTCG ATTCGTACAGAACATTCT TGCAAGATGGCAAATCA CCAAAAAACCGCGAAGA TATCGGTCTCCAAGCCG CATTTCGTTCAGTTTTTC CTATCGAAAGTTATTCTG GCAATGCTG | 47 |
| | | A.spp_4056 R25Y | GATYCGTAAG AATTTTGGTA AATTG | 2 | | |
| *Acinetobacter baumanni.* | 16S-23S ITS | A.bau_373F 23 | TGATCTGACG AAGACACATT AAC | 3 | ATTTCAGTTTAGAGCACT GTGCACTTAAGCACCGT ACAGCTTCAATCTAAATT CACATACCAAAACGCTT GATTCAGTTAATTTGCTA GTTCTCAATTCATCTAGA TTAATTGCTTAACCTAAA CTCTTGAGTGAACAATTT ATTTCAGACTCAATTTTG CCAATCTGTTAATGAGTT AATGTGTCTTCGTCAGAT CA | 48 |
| | | A.bau_552R 22 | ATTTCAGTTT AGAGCACTGT GC | 4 | | |
| *Citrobacter* spp. | ompA | C. spp_449F17 | GCTGCTTCTT CCTGCTG | 5 | GTCTGGAATACCAGTGG ACTAACAACATCGGCGA CGCAAACACCGTTGGCG GTCGTCCAGATAACGGC CTGCTGAGCGTTGGTGT TTCCTACCGTTTCGGCC AGCAGGAAGAAGCAGC | 49 |
| | | C. spp_443F18 S | CAGCTTSTTC CTGCTGAC | 6 | | |
| | | C. spp_551 Rl 9 | GTCTGGAATA CCAGTGGAC | 7 | | |
| *Enterobacter* spp. | rpoS | Enb.spp_21 6F17 | GGCCGAAGA AGAAGTCT | 8 | GCGGATGAGACCTAAGT TGCCCTCTTCAATCAGA TCCAGCAGAGCCAGACC ACGATTGCCGTAACGGC GGGCAATTTTCACGACC AGTCGCAGGTTACTTTC GATCATGCGACGACGCG AGGCAACATCACCACGC AAAGCACGACGTGCGAA ATAGACTTCTTCTTCGG CC | 50 |
| | | Enb.spp_36 9R19 | GCGGATGAG ACCTAARTTG | 9 | | |
| *Escherichia coli* | gyrB | E.coli_468F 19 | GGTTACCGG CGAGACTGA A | 10 | GACAACTCACGCAGACG TTTCGCCAGAATTTCATA TTCGAACTCGGTCACAT TGGTGAAGGTTTCGAGG CTGGGCCAGAAACGCAC CATGGTGCCGGTTTTTT CAGTCTCGCCGGTAACC | 51 |
| | | E.coli_567R 18 | GACAACTCAC GCAGACGT | 11 | | |
| *Klebsiella aerogenes* | gyrB | K.aer_2097 F18 | GCGTCGTCTT CGATAAGC | 12 | GAAGTTTGATATTCGTGA AAACAGCGAGCTGCAGC AGTTCGAGCCCATCGTG CGCGTGCGTACCCACGG CGTCGATACCGACTACC CGTTGGATAACGAGTTC ATCACCGGCGCGGAATA TCGCCGCATCTGCACCC TCGGCGAGAAGCTGCGT GGGCTTATCGAAGACGA CGC | 52 |
| | | K.aer_1941 R25 | GAAGTTTGAT ATTCGTGAAA ACAG | 13 | | |
| *Klebsiella pneumoniae* | gyrB | K.pne_1886 F19 | GGTGAACAC CCTGGTCTC | 14 | GATATTCCGGCCCCATA ATGAACTCGTTATCCAG CGGATAGTCGGTATCCA CGCCGTGGGTACGTACG CGAATAACCGGCTCAAA ATGCTGCAGTTCTTTGTT CTCGTGGAGATCGAATT TCCACTGGCTGCCGTGC TGTTCTTTCTCGTTCAGC TCGGAGACCAGGGTGTT CACC | 53 |
| | | K.pne_2043 R20 | GATATTCCGG CCCCATAATG | 15 | | |
| *Klebsiella oxytoca* | pehX | K.oxy_261F 17 | CCATTTCGGC TACCGTC | 16 | CGATGAAAGATATCGCC AAAGAGCCGTTCGTTTTT ACTATCAAATACAGCGC CGACGTGAATGATACGA CCCCTGCCGACGAGCCC GCTCAGTTCCGCGACGT TCAGGTGCAGGACGTCA CCGTCGATGGTACTTCG GCTAAGCACAGCATCCT GATTGACGGCATGACGG TAGCCGAAATGG | 54 |
| | | K.oxy_423R 21 | CGATGAAAGA TATCGCCAAA G | 17 | | |
| *Klebsiella variicola* | gyrB | K.var_1886 F18 | GGTGAACAC CCTGGTTTC | 18 | CGATATTCCGGCCCCAT GATGAACTCGTTATCCA GCGGATAGTCGGTATCC ACCCCGTGGGTACGGAC GCGGATAATCGGCTCAA ACTGCTGCAATTCACCG TTCTCGCGGACATCAAA TTTCCACTGGCTGCCGT GCTGCTCTTTCTCATTCA GCTCGGAAACCAGGGTG TTCACC | 55 |
| | | K.var_2046 R18 | CGATATTCCG GCCCCATG | 19 | | |
| *Pseudomonas aeruginosa* | oprL | Pse.aer_43 0F18 | GGTAAAGAG CGTCCGGTC | 20 | TTACTTCTTCAGCTCGAC GCGACGGTTCTGAGCCC AGGACTGCTCGTCGTGG CCGGTAGCGACCGGACG CTCTTTACC | 56 |
| | | Pse.aer_48 9R19 | TTACTTCTTC AGCTCGACG | 21 | | |
| *Proteus spp.* | rpoB | Pro.spp_23 56F20Y | CAACTCACTA YGGTCGTGTG | 22 | CATCTGTTACAGCGTTG TTTTCTACAACACGGTAA GGCGTTTCTAAGAATCC GTACTCGTTAGTCTGTG CATAAACAGATAATGAG TTAATCAGACCGATATTT GGACCTTCAGGTGTTTC GATTGGACACACACGAC CATAGTGAGTTG | 57 |
| | | Pro.spp_22 26R22 | CATCTGTTAC AGCGTTGTTT TC | 23 | | |
| *Serratia marcescens* | hasA | Ser.mar_34 9F17Y | GGCCTGAAC CTCAGYAG | 24 | CCGTAGTCGTCGAGGAT GCCGTTCAGCGCGGTTT CCAGCGCGCCGGTATCG CCGGACATCAGGCCGTA CACCACCTGGTGCACCA CGCCATCGTGGCCCTGC GCCTGCAGGCTGCTGAG GTTCAGGCC | 58 |
| | | Ser.mar_45 9R19 | CCGTAGTCGT CGAGGATG | 25 | | |
| *Enterococcus spp.* | rpoB | Enc.fae_12 1F23 | AGTCCCTCAT CTAAAAACCA TTG | 26 | AGCTTGGCTGGACACGT AGTAAAATACGGAAAGC ATCGCGAACGTAGAAGT TTCGCACGTATCAGTGA AGTATTGGAATTACCAA ATTTAATTGAA | 59 |
| | | Enc.spp_35 66F24Y | TCAATCAAAT TCGGTAATTC YAAT | 27 | | |
| | | Enc.spp_35 63F23Y | CGATCAAATT YGGTAATTCC AAC | 28 | | |
| | | Enc.spp_36 39R18R | ARCTTGGCTG GACACGTA | 29 | | |
| *Listeria spp.* | iap p60 | L.spp_444F 25 | ATGAATATGA AAAAAGCAAC TATCG | 30 | GCGATACCCCAAAGAGT ATCACCAGCTTCAACTA CTACAGTGCTTGCGGAT GCGATTGTTGGAGCAGC AAATGCTGTTACCGCAA TCCCAGCTGTAGCCGCG ATAGTTGCTTTTTTCATA TTCAT | 60 |
| | | L.spp_551R 20W | GCGATACCC CAAAGWGTA TC | 31 | | |
| *Staphylo*coccus *aureus* | nuc | Sta.aur_586 F21W | GTAGCGAAA WAGAAAAAC CTC | 32 | ATACTTATTAAGTGCTGG CATATGTATGGCAATTGT TTCAATATTACTTATAGG GATGGCTATCAGTAATG TTTCGAAAGAACAATAC GCAAAGAGGTTTTTCTTT TTCGCTAC | 61 |
| | | Sta.aur_656 R24 | ATACTTATTA AGTGCTGGC ATATG | 33 | | |
| | | Sta.sch_22 6F25Y | TTCAACTTCA ATTTTYTTAG CATTC | 34 | | |
| *Staphylo*coccus *argenteus* | nuc | Sta.arg_226 F25 | TTCCACTTCT ATTTTCTTAG CATTC | 35 | ATTAATTGATACGCCTGA AACGAAACATCCTAAAA AAGGCGTTGAAAAATAT GGCCCAGAAGCAAGTGC ATTTACGAAGAATATGGT AGAGAATGCTAAGAAAA TAGAAGTGGAA | 62 |
| | | Sta.arg_319 R22Y | ATTAATTGAT ACGCCYGAAA CG | 36 | | |
| *Staphylo*coccus *epidermidis* | atlE | Sta.epi_409 F20 | AGTTGAGACA GGAAATGGTC | | | 63 |
| | | Sta.epi_544 R19 | AGGAGTGGT CCACTTCTTC | | AGGAGTGGTCCACTTCT TCTGTTTAGCAAAACTCT TACCAGTCTTTACAGCA TCTTCATCAAAAGCACC AATACCAAAAAAGTTATA ATAGTGATGGTTACCTTC TTTAATTCCTTTTGATAA ATCTGATTGACCATTTCC TGTCTCAACT | |
| *Staphylo*coccus *spp.* | tuf | Sta.spp_59 5F20Y | TACATYCCAA CTCCAGAAC G | 39 | ACTTTGATTTGACCACGT TCAACACGGCCTGTAGC AACAGTACCACGACCAG TGATTGAGAATACGTCC TCAACTGGCATCATGAA TGGTTTGTCAGAATCAC GTTCTGGAGTTGGAATG TA | 64 |
| | | Sta.spp_69 7R20 | ACTTTGATTT GACCACGTTC | | | |
| *Strepto*coccus *spp* | rnpB | Str.spp_rnp B F20 | ATGAGGAAAG TCCATGCTAG | 41 | TTGGGTTGCTAGCTTGA GGGGTTTACCGCGTTCC ACTCTCTCTGTTTCCAGA AAGACTTCGTCACTGTG GCACTTTCAAGCCTACT CTGGCCTATCCAAGGAC TTAGCCATTTCAACTGC CGTAACGATTTCTCGTC CCTAGGCTTATGGTTTC GCCTAGCACAAACACTA CAGGCATCACAGCCTGT GCTAGCATGGACTTTCC TCATGAGAAGCAATTCT CCTCACGCGATTATCCA AAAAT | 65 |
| | | Str.spp_rnp B R18 | TTGGGTTGCT AGCTTGAG | 42 | | |
| *Strepto*coccus *pneumoniae* | xisco | Str.pneu_37 1F21 | CCTTTGATTT CATCCTGCTT G | 43 | CACAAAACTCAGCTCAA TCACAAGCTTCTAAGCA ATTAGCTACTGAAAAAG AATCAGCTAAAAATGCC ATTGAAAAAGCAGCCAA GAACAAGCAGGATGAAA TCAAAGG | 66 |
| | | Str.pneu_45 9R21 | CACAAAACTC AGCTCAATCA C | 44 | | |
| *Strepto*coccus *pyogenes* | gyrB | Str.pyo_162 3F22 | GCCACCTATT TATGGTGTTA AG | 45 | AACGATTTTCAGGATCC ATAGTAGTTTCCCAAAGT TGATGGTCATCCATTTC CCCAAGACCTTTATAAC GTTGAACAGTTGGTTTTG AACGACCAATACTATATT TTTCAAGAGCTGTTTTTA ATTGGTCTTCTTGATCAA TACCTGGCTGAATGTAC TCTTTAATCTCACTACCG ACCTTAACACCATAAATA GGTGGC | 67 |
| | | Str.pyo_ 180 0R23 | AACGATTTTC AGGATCCATA GTA | 46 | | |

Primer sets selected for detecting antimicrobial resistance genes of bacteria and the DNAs to be amplified by the primer sets are listed in Table 13.

**[Table 13]**

| Bacteria species | **Name of antimicrobial resistance gene** | Name of primer | Primer sequence (5' to 3') | SEQ ID NO: | DNA sequence to be amplified (biotinylated ReV chain notation) (5' to 3') | SEQ ID NO: |
|---|---|---|---|---|---|---|
| *Klebsiella pneumoniae* | *Beta-lactamase _KPC* | KPC_167 F20 | CTGTAAGTTA CCGCGCTGA G | 68 | GGCGTTATCACTGTAT TGCACGGCGGCCGCGG ACAGCTCCGCCACCGT CATGCCTGTTGTCAGA TATTTTTCCGAGATGG GTGACCACGGAACCAG CGCATTTTTGCCGTAA CGGATGGGTGTGTCCA GCAAGCCGGCCTGCTG CTGGCTGCGAGCCAGC ACAGCGGCAGCAAGAA AGCCCTTGAATGAGCT GCACAGTGGGAAGCGC TCCTCAGCGCGGTAAC TTACAG | 100 |
| | | KPC_376 R21_B | GGCGTTATCA CTGTATTGCA C | 69 | | |
| *Klebsiella pneumoniae* | *Beta-lactamase _NOM* | NDM_178 F19 | CAGCACACTT CCTATCTCG | 70 | CATTGGCATAAGTCGC AATCCCCGCCGCATGC AGCGCGTTCATACCGC CCATCTTGTCCTGATG CGCGTGAGTCACCACC GCCAGCGCGACCGGCA GGTTGATCTCCTGCTT GATCCAGTTGAGGATC TGGGCGGTCTGGTCAT CGGTCCAGGCGGTATC GACCACCAGCACGCGG CCGCCATCCCTGACGA TCAAACCGTTGGAAGC GACTGCCCCGAAACCC GGCATGTCGAGATAGG AAGTGTGCTG | 101 |
| | | NDM_408 R20 | CATTGGCATA AGTCGCAATC | 71 | | |
| *Pseudomonas aeruginosa* | *Beta-lactamase _IMP85* | IMP_307F 22 | GGAATAGRGT GGCTTAATTC TC | 72 | AGCCAAACCACTACGT TATCTGGAGTGTGCCC TGGACCAGGATAAAAA ACTTCAATCTTTTTCTT AACTAGCCAATAGCTA GCTCCGCTAAATGAAT TTTTAGCTTGTACTTTA CCGTCTTTTTTAAGAAG TTCATTTGTTAATTCAG ATGCATACGTGGGGAT AGATTGAGAATTAAGC CACTCTATTCC | 102 |
| | | IMP_478 R20 | ARCCAAACCA CTACGTTATC | 73 | | |
| *Citrobacter freundii* | *Beta-lactamase _VIM* | VIM_155F 18 | GTGTTTGGTC GCATATCG | 74 | CCCCACGCTGTATCAA TCAAAAGCAACTCATC ACCATCACGGACAATG AGACCATTGGACGGGT AGACCGCGCCATCAAA CGACTGCGTTGCGATA TGCGACCAAACAC | 103 |
| | | VIM_246 R18 | CCCCACGCT GTATCAATC | 75 | | |
| *Salmonella enterica* | *Beta-lactamase _CTX-M1* | CTX-M1_ 402F20 | TAACGTGGC GATGAATAAG C | 7β | TTGCCTTTCATCCATGT CACCAGCTGCGCCCGT TGGCTGTCACCCAATG CTTTACCCAGCGTCAG ATTACGCAGAGTTTGC GCCATTGCCCGAGGTG AAGTGGTATCACGCGG ATCGCCCGGAATGGCG GTGTTTAACGTCGGCT CGGTACGGTCGAGACG GAACGTTTCGTCTCCC AGCTGTCGGGCGAACG CGGTGACGCTAGCCGG GCCGCCAACGTGAGAA ATCAGCTTATTCATCG CCACGTTA | 104 |
| | | CTX-M1_ 631R20 | TTGCCTTTCA TCCAYGTCAC | 77 | | |
| *Salmonella enterica* | *Beta-lactamase _CTX-M2* | CTX-M2_ 414F20 | GAAYAAGCTG ATTGCCCATC | 78 | TTGCCCTTAAGCCACG TCACCAACTGTGCCCG CTGAGTTTCCGCCAGC GCTTTACCCAGCGTCA GATTTTTCAGGGTCTG CGCCATCGCGAGCGGC GTGGTGGTATCACGCG GGTCGCCTGGAATGGC GGTATTGAGCGTGGGC TCGGTTCTGTCCAGAC GGAAGGTCTCATCACC CAACGAGCGAGCAAAC GCCGTCACTTTATCGG GACCACCCAGATGGGC AATCAGCTTATTC | 105 |
| | | CTX-M2_ 633R18 | TTGCCCTTAA GCCACGTC | 79 | | |
| *Citrobacter amalonaticus* | *Beta-lactamase _CTX-M8* | CTX-M2_ 414F20 | GAAYAAGCTG ATTGCCCATC | 80 | GTGTTATCCCCAATCG CACGGGCAAACGCCGT CACTTTATCCGGCCCC CCAAGATGGGCAATCA GCTTGTTC | 106 |
| | | CTX_M8_ 485R19 | GTGTYATCCC CAATCGCA | 81 | | |
| *Escherichia coli* | *Beta-lactamase _CTX-M25* | CTX-M2_ 414F20 | GAAYAAGCTG ATTGCCCATC | 82 | GTGTCATCGCCAATCG TACGGGCAAATGCCGT CACTTTATCCGGCCCC CCGAGATGGGCAATCA GCTTATTC | 107 |
| | | CTX_M25 _485R21 | GTGTCATCGC CAATCGTACG | 83 | | |
| *Escherichia coli* | *Beta-lactamase _CTX-M9* | CTX-M9_ 291F18 | TGTCGAGATC AAGCCTG | 84 | GGGCAATCAATTTGTT CATGGCGGTATTGTCG CTGTACTGCAACGCGG CCGCGCTCAGCTCTGC CAGCGTCATTGTGCCG TTGACGTGTTTTTCGG CAATCGGATTGTAGTT AACCAGATCGGCAGGC TTGATCTCGACA | 108 |
| | | CTX-M9_ 411\R20 | GGGCAATCA ATTTGTTCAT G | 85 | | |
| *Acinetobacter baumannii* | *Beta-lactamase _OXA-23* | OXA23_2 62F18 | ATCGGATTGG AGAACCAG | 86 | CGCAAGTTCCTGATAG ACTGGGACTGCAGAAA GCTTCATGGCTTCTCC TAGTGTCATGTCTTTTT CCCAAGCGGTAAATGA CCTTTTCTCGCCCTTC CATTTAAATATTTCATT AATATCCGTTTTCTGGT TCTCCAATCCGAT | 109 |
| | | OXA23_3 86R20 | CGCAAGTTCC TGATAGACTG | 87 | | |
| *Acinetobacter baumannii* | *Beta-lactamase _OX4-40* | OXA40_1 31F22 | GCTATTTTGA TGAAGCTCAA AC | 88 | CTTAAATGTTGATGCA GGGACATATTCTTTATT TGCTCGTGCAAGAGCA TTACCATAGGTGCTAA GATTTTTACCCTCTTTA ATAATAATTACACCCT GTGTTTGAGCTTCATC AAAATAGC | 110 |
| | | OXA40_2 32R22 | CTTAAATGTT GATGCAGGG AC | 89 | | |
| *Klebsiella pneumoniae* | *Beta-lactamase _OXA-48* | OXA48_1 48F21 | AATAAGCAGC AAGGATTTAC C | 90 | GTGTTCATCCTTAACC ACGCCCAAATCGAGGG CGATCAAGCTATTGGG AATTTTAAAGGTAGATG CGGGTAAAAATGCTTG GTTCGCCCGTTTAAGA TTATTGGTAAATCCTTG CTGCTTATT | 111 |
| | | OXA48_2 52R19 | GTGTTCATCC TTAACCACG | 91 | | |
| *Acinetobacter haemolyticus* | *Beta-lactamase _OXA-58* | OXA58_5 16F23 | GCCTTTGACA ATTACACCTA TAC | 92 | CGCTCTACATACAACA TCTCTTTCACTTGTTGC TGAACTTCAGGTTTAAA AGGCAATTGCCCTTGG GCTAAATCATACACAA ACTTTACTTCTTGTATA GGTGTAATTGTCAAAG GC | 112 |
| | | OXA58 6 13R20 | CGCTCTACAT ACAACATCTC | 93 | | |
| *Staphylococcus aureus* | mecA | mecA_76 3F22 | GTTATGTTGG TCCCATTAAC TC | 94 | GATAGCCATCTTCATG TTGGAGCTTTTTATCGT AAAGTTTTTCGAGTCC CTTTTTACCAATAACTG CATCATCTTTATAGCCT TTATATTCTTTTTGTTT TAATTCTTCAGAGTTAA TGGGACCAACATAAC | 113 |
| | | mecA_87 5R20 | GATAGCCATC TTCATGTTGG | 95 | | |
| *Enterococcus faecalis* | vanA | vanA_808 F18 | GGACGGATA CAGGAAACG | 96 | ATTGACTTCGTTCAGTA CAATGCGGCCGTTATC TTGTAAAAACATATCCA CACGGGCTAGACCTCT ACAGCCGAGCGCTTTA TATATTTTTTTTGCCGT TTCCTGTATCCGTCC | 114 |
| | | vanA_900 R22 | ATTGACTTCG TTCAGTACAA TG | 97 | | |
| *Enterococcus faecium* | vanB | vanB_264 F19 | GCGTATTGAT GTGGCTTTC | 98 | CTTTGAATATCACAGC CCACATAGGGGATACC AGACAATACAAACAGC CCCTGTATCGCACCAT CCTCCCCGCATTTGCC ATGCAAAACCGGGAAA GCCACATCAATACGC | 115 |
| | | vanB_355 R20 | CTTTGAATAT CACAGCCCA C | 99 | | |

Probes selected for detecting target genes of bacteria are listed in Table 14.

**[Table 14]**

| Bacteria species | Name of target gene | Name of probe | Probe sequence (5' to 3') | SEQ ID NO: |
|---|---|---|---|---|
| *Acinetobacter spp* | rpoB | A.spp_P1 (rpoB) | TTTTTTTTTTTTCGCGGTTTTTTGG | 116 |
| *Acinetobacter spp.* | rpoB | A.spp_172P17 | TTTTTTTTATAGGAAAAACTGAACG | 117 |
| *Acinetobacter baumannii* | 16S-23S ITS | A.bau_P19 | TTTTTTGAATTTAGATTGAAGCTGT | 118 |
| *Citrobacter spp.* | ompA | C. spp_24P17R | TTTTTTTTGAAACGGTARGAAACAC | 119 |
| *Enterobacter spp.* | rpoS | Enb.spp_349P17 | TTTTTTTTCTGGATCTGATTGAAGA | 120 |
| *Escherichia coli.* | gyrb | E.coli_531P16 | TTTTTTTTTCAATGTGACCGAGTTC | 121 |
| *Klebsiella aerogenes* | gyrb | K.aer_2034P17 | TTTTTTTTGGTGATGAACTCGTTAT | 122 |
| *Klebsiella oxytoca* | pehX | K.oxy_392P17R | TTTTTTTTGCRCTGTATTTGATAGT | 123 |
| *Klebsiella pneumoniae* | gyrB | K.pne_P16 | TTTTTTTTTGAACTGCAGCATTTTG | 124 |
| *Klebsiella variicola* | syrB | K.var_1968P16 | TTTTTTTTTGTTGCAGCAGTTTGAG | 125 |
| *Pseudomonas aeruginosa* | oprL | Pse.aer_430P15 | TTTTTTTTTTGGTAAAGAGCGTCCG | 126 |
| *Proteus spp.* | rpoB | Pro.spp_P16 | TTTTTTTTTTAGAAACGCCTTACCG | 127 |
| *Serratia marcescens* | hasA | Ser.mar_424P14 | TTTTTTTTTTATGTCCGGCGATAC | 128 |
| *Enterococcus spp.* | rpoB | Enc.spp_3607P14 | TTTTTTTTTTCTTCTACGTTCGCG | 129 |
| *Enterococcus spp.* | rpoB | Enc.spp_3606P15 | TTTTTTTTTTGCTTCTACGTTCTCG | 130 |
| *Enterococcus spp.* | rpoB | Enc.spp_Probe A | TTTTTTTTGATGCTTTCCGTATTTT | 131 |
| *Listeria spp.* | iap p60 | L.spp_533P16_1 | TTTTTTTTTGTAGTAGTCGAAGCTG | 132 |
| *Listeria spp.* | iap p60 | L.spp_533P17_2 | TTTTTTTTTTGTAGTAGTTGAAGCTGG | 133 |
| *Staphylococcus aureus* | nuc | Sta.aur_625P21 | TTTTCCCTATAAGTAATATTGAAAC | 134 |
| *Staphylococcus argenteus* | nuc | Sta.arg_2.88P17 | TTTTTTTTTTCATATTTTTCAACGCCT | 135 |
| *Staphylococcus epidermidis* | atlE | Sta.epi_505P20 | TTTTTTTTTTGATGCTGTAAAGACTGGTAA | 136 |
| *Staphylococcus spp.* | tuf | Sta.spp_626P16 | TTTTTTTTTAACCATTCATGATGCC | 137 |
| *Streptococcus pneumoniae* | xisco | Str.pneu_440P17 | TTTTTTTTGCTAATTGCTTAGAAGC | 138 |
| *Streptococcus pyogenes* | gyrB | Str.pyo_1783P17 | TTTTTTTTCATCAACTTTGGGAAAC | 139 |
| *Streptococcus spp.* | rnpB | Str.spp_Probe B | TTTTTTTTTTTTTGCCACAGTGACG | 140 |

Probes selected for detecting antimicrobial resistance genes of bacteria are listed in Table 15.

**[Table 15]**

| Name of strain | Name ol antimicrobial resistance gene | Name of probe | Probe sequence (5' to 3') | SEQ ID NO: |
|---|---|---|---|---|
| *Klebsiella pneumoniae* | *Beta-lactamase _NDM* | NDM_365P16 | TTTTTTTTTATCAG GACAAGATGGG | 141 |
| *Klebsiella pneumoniae* | *Beta-lactamase _KPC* | KPC_330P18 | TTTTTTTATATCTG ACAACAGGCAT | 142 |
| *Citrobacter freundii* | *Beta-lactamase _VIM* | VIM_232P17 | TTTTTTTTGATGAG TTGCTTTTGAT | 143 |
| *Pseudomonas aeruginosa* | *Beta-lactamase _IMP85* | IMP_378P16K | TTTTTTTTTAGACG GTAAGGTKCAA | 144 |
| *Acinetobacter baumannii* | *Beta-lactamase _OXA-23* | OXA23_373P15 | TTTTTTTTTTCTTT CTGCAGTCCCA | 145 |
| *Acinetobacter baumannii* | *Beta-lactamase _OXA-40* | QXA40_217P19 | TTTTTTGCAAATAA AGAATATGTCC | 146 |
| *Klebsiella pneumoniae* | *Beta-lactamase _OXA-48* | OXA48_223P16 | TTTTTTTTTCCCAA TAGCTTGATCG | 147 |
| *Acinetobacter haemolyticus* | *Beta-lactamase _CXA-58* | OXA58_596P16 | TTTTTTTTTTCAG CAACAAGTGAA | 148 |
| *Salmonella enterica* | *Beta-lactamase _CTX-M1* | CTX-M1_594P15 | TTTTTTTTTTGGGT AAAGCATTGGG | 149 |
| *Salmonella enterica* | *Beta-lactamase _CTX-M2* | CTX-M2_465P14 | TTTTTTTTTTTTCG CTCGTTGGGTG | 150 |
| *Citrobacter amalonaticus* | *Beta-lactamase _CTX-M8* | CTX-M8_485P20 | TTTTTTTATAAAGT GACGGCGTTTG | 151 |
| *Escherichia coli* | *Beta-lactamase _CTX-M9* | CTX-M9_393P16 | TTTTTTTTTGTACA GCGACAATACC | 152 |
| *Escherichia coli* | *Beta-lactamase _CTX-M25* | CTX-M25_485P2 0 | TTTTTTTATAAAGT GACGGCATTTG | 153 |
| *Staphylococcus aureus* | mecA | mecA_822P17 | TTTTTTTTGATGAT GCAGTTATTGG | 154 |
| *Enterococcus faecalis* | vanA | vanA_867P1 6 | TTTTTTTTTCCGTG TGGATATGTTT | 155 |
| *Enterococcus faecium* | vanB | vanB_331P18 | TTTTTTTTTTGTAT TGTCTGGTATC | 156 |

Because the present invention is directed to a technique that enables multiplex assay of a plurality of bacterial species and antimicrobial resistance genes, it is necessary to evaluate the reactivity and specificity to individual templates in the evaluation using Multiplex PCR and a substrate having multiple probes immobilized thereon.

PCR products amplified by MultiplexPCR with representative species as templates were reacted in a system containing all probes, where multiplex assay was performed. PCR products amplified by MultiplexPCR with antimicrobial resistance genes as templates were reacted in a system containing all probes, where multiplex assay was performed. The measurement procedure is described in the following section, Overview of detection step.

### Overview of detection step

### (1) PCR amplification reaction

PCR amplification was performed using the genomic DNA of target bacterial species or antimicrobial resistance genes as templates.
Denaturation step: at 94°C for 30 seconds
Annealing step: at 60°C for 60 seconds → at 94°C for 30 seconds (Number of cycles: 30)
Extension step: at 72°C for 600 seconds

The composition of the PCR reaction solution is listed in the following Table.

**[Table 16]**

| Composition of the PCR reaction solution | | |
|---|---|---|
| PCR mix | Amount used/uL | Final concentration |
| Multiplex PCR Enzyme Mix | 0.1 | 1 U |
| 2×Multiplex PCR Buffer | 10 | 1x |
| PMBAC* or PMAMR** | 4.9 | - |
| Template | 5 | - |
| total | 20 | |

| | | |
|---|---|---|
| * Mixed solution of primers (composition is shown in Table 17) for detection of bacteria used in the identification of bacteria. ** Mixed solution of primers (composition is shown in Table 18) for detection of antimicrobial resistance genes used in the identification of antimicrobial resistance genes | | |

**[Table 17]**

| Table for PMBAC preparation | | | |
|---|---|---|---|
| Solution to be added (primer or ddH₂O) | Concentra- tion of stock solution (uM) | Amount added (uL) | Final concentration (uM) |
| A.bau_373F23 | 100 | 8.2 | 0.82 |
| A.bau_552R22_B | 100 | 8.2 | 0.82 |
| A.spp_3923F22 | 100 | 8.2 | 0.82 |
| A.spp_4056R25Y_B | 100 | 16.3 | 1.63 |
| C. spp_449F17 | 100 | 8.2 | 0.82 |
| C. spp_443F18S | 100 | 16.3 | 1.63 |
| C. spp_551R19_B | 100 | 8.2 | 0.82 |
| Enb.spp_216F17 | 100 | 8.2 | 0.82 |
| Enb.spp_369R19R_B | 100 | 16.3 | 1.63 |
| E.coli_468F19 | 100 | 8.2 | 0.82 |
| E.coli_567R18_B | 100 | 8.2 | 0.82 |
| K.aer_2097F18 | 100 | 8.2 | 0.82 |
| K.aer_1941R25_B | 100 | 8.2 | 0.82 |
| K.oxy_261F17 | 100 | 8.2 | 0.82 |
| K.oxy_423R21_B | 100 | 8.2 | 0.82 |
| K.pne_1886F19 | 100 | 8.2 | 0.82 |
| K.pne_2043R20_B | 100 | 8.2 | 0.82 |
| K.var_1886F18 | 100 | 8.2 | 0.82 |
| K.var_2046R18_B | 100 | 8.2 | 0.82 |
| Pse.aer_430F18 | 100 | 8.2 | 0.82 |
| Pse.aer_489R19_B | 100 | 20.4 | 2.04 |
| Pro.spp_2356F20Y | 100 | 8.2 | 0.82 |
| Pro.spp_2226R22_B | 100 | 8.2 | 0.82 |
| Ser.mar_349F17Y | 100 | 8.2 | 0.82 |
| Ser.mar_459R19_B | 100 | 8.2 | 0.82 |
| Enc.fae_121F23 | 100 | 8.2 | 0.82 |
| Enc.spp_3563F23Y | 100 | 16.3 | 1.63 |
| Enc.spp_3566F24Y | 100 | 16.3 | 1.63 |
| Enc.spp_3639R18R_B | 100 | 16.3 | 1.63 |
| Lspp_444F25 | 100 | 16.3 | 1.63 |
| L.spp_551R20_B | 100 | 16.3 | 1.63 |
| Sta.spp_595F20Y | 100 | 16.3 | 1.63 |
| Sta.spp_697R20_B | 100 | 40.8 | 4.08 |
| Sta_aur_566F21_W | 100 | 8.2 | 0.82 |
| Sta.aur_656R24 | 100 | 20.4 | 2.04 |
| Sta.arg_226F25 | 100 | 8.2 | 0.82 |
| Sta.sch_226F25Y | 100 | 16.3 | 1.63 |
| Sta.arg_319R22Y | 100 | 16.3 | 1.63 |
| Sta.epi_409F20 | 100 | 8.2 | 0.82 |
| Sta.epi_544R19 | 100 | 8.2 | 0.82 |
| Str.spp_rnpB F20 | 100 | 8.2 | 0.82 |
| Str.spp_rnpB R18 | 100 | 8.2 | 0.82 |
| Str.pneu_371F21 | 100 | 8.2 | 0.82 |
| Str.pneu_459R21 | 100 | 8.2 | 0.82 |
| Str.pyo_1623F22 | 100 | 8.2 | 0.82 |
| Str.pyo_1800R23 | 100 | 8.2 | 0.82 |
| ddH₂O | - | 478 | - |

**[Table 18]**

| Table for PMAMR preparation | | | |
|---|---|---|---|
| Solution to be added (primer or ddH₂O) | Concentration of stock solution (uM) | Amount added (uL) | Final concentration (uM) |
| KPC_167F20 | 100 | 8.2 | 0.82 |
| KPC_376R21_B | 100 | 8.2 | 0.82 |
| NDM_178F19 | 100 | 8.2 | 0.82 |
| NDM_408R20_B | 100 | 8.2 | 0.82 |
| IMP_307F22 | 100 | 16.3 | 1.63 |
| IMP_478R20_B | 100 | 16.3 | 1.63 |
| VIM_155F18 | 100 | 8.2 | 0.82 |
| vrM_246R18_B | 100 | 8.2 | 0.82 |
| CTX-M1_402F20 | 100 | 8.2 | 0.82 |
| CTX-M1_631R20_B | 100 | 8.2 | 0.82 |
| GTX-M2_414F20 | 100 | 8.2 | 0.82 |
| CTX-M2_633R18_B | 100 | 8.2 | 0.82 |
| CTX_M8_485R19_B | 100 | 8.2 | 0.82 |
| CTX_M25_485R21_B | 100 | 16.3 | 1.63 |
| CTX-M9_291F17 | 100 | 8.2 | 0.82 |
| CTX-M9_411R20_B | 100 | 8.2 | 0.82 |
| OXA23_262F18 | 100 | 8.2 | 0.82 |
| OXA23_386R20_B | 100 | 8.2 | 0.82 |
| OXA40_31F22 | 100 | 8.2 | 0.82 |
| OXA40_232R22_B | 100 | 8.2 | 0.82 |
| OXA48_148F21 | 100 | 8.2 | 0.82 |
| OXA48_252R19_B | 100 | 8.2 | 0.82 |
| OXA58_516F23 | 100 | 8.2 | 0.82 |
| OXA58_613R20_B | 100 | 8.2 | 0.82 |
| mecA_763F22 | 100 | 8.2 | 0.82 |
| mecA_875R20_B | 100 | 8.2 | 0.82 |
| vanA_808F18 | 100 | 8.2 | 0.82 |
| vanA_900R22_B | 100 | 8.2 | 0.82 |
| vanB_264F19 | 100 | 8.2 | 0.82 |
| vanB_355R20_B | 100 | 8.2 | 0.82 |
| ddH₂O | - | 730.6 | - |

### (2) Thermal denaturation of PCR product

PCR products were thermally denatured in the following conditions before they were exposed to probes immobilized on a substrate.

Thermal denaturation conditions: at 95°C for 5 minutes, followed by rapid cooling to 4°C.

### (3) Hybridization reaction of probes on a substrate with PCR product, and labeling

Hybridization reactions of individual probes immobilized onto a substrate with PCR products were performed. The reaction solution contains Saline-sodium-phosphate-EDTA buffer (SSPE-buffer). The reaction solution contains Saline-sodium-phosphate-EDTA buffer (SSPE-buffer). The PCR products captured by probes on a substrate were modified with biotin at the 5' end side and biotin was labeled with Streptavidin-Phycoerythrin (PlexBio Co., Ltd.). Hybridization reactions on a substrate and labeling were carried out by use of IntelliPlex 1000 πcode Processor, which is a device of PlexBio Co., Ltd., in the following conditions:
Hybridization conditions: incubation at 37°C for 20 minutes
Labeling conditions: incubation at 37°C for 10 minutes

### (4) Detection of labeled PCR product on substrate

Distinguishable IDs were provided onto each substrate and predetermined probes are immobilized to individual IDs. PCR products are captured by the corresponding probes on the substrate and labeled. The labeled PCR products on the substrate emit fluorescence, the values of which can be measured by a detector. Fluorescence values were measured by a fluorometer (100 Fluorescent Analyzer, PlexBio Co., Ltd.) in the same manner as in Example 1.

### 4. Results

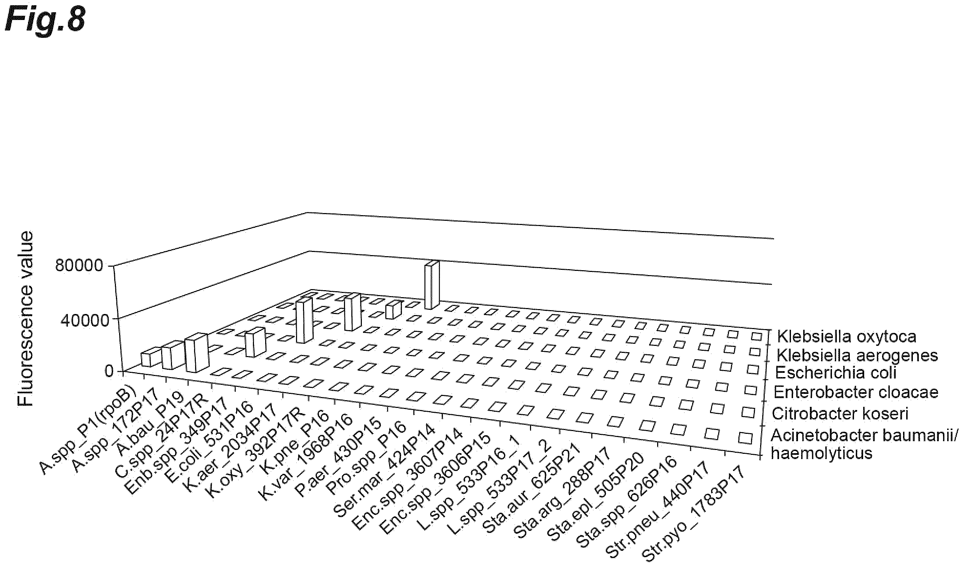

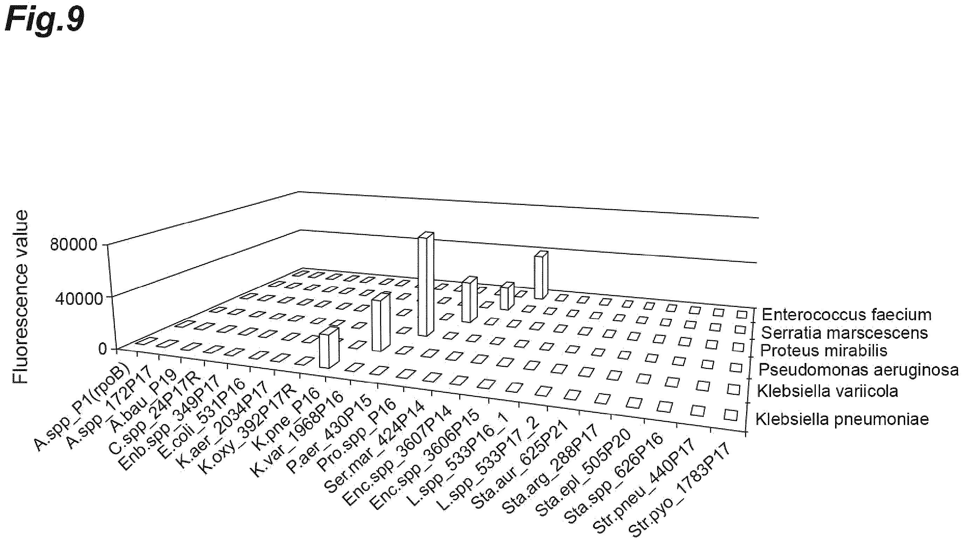

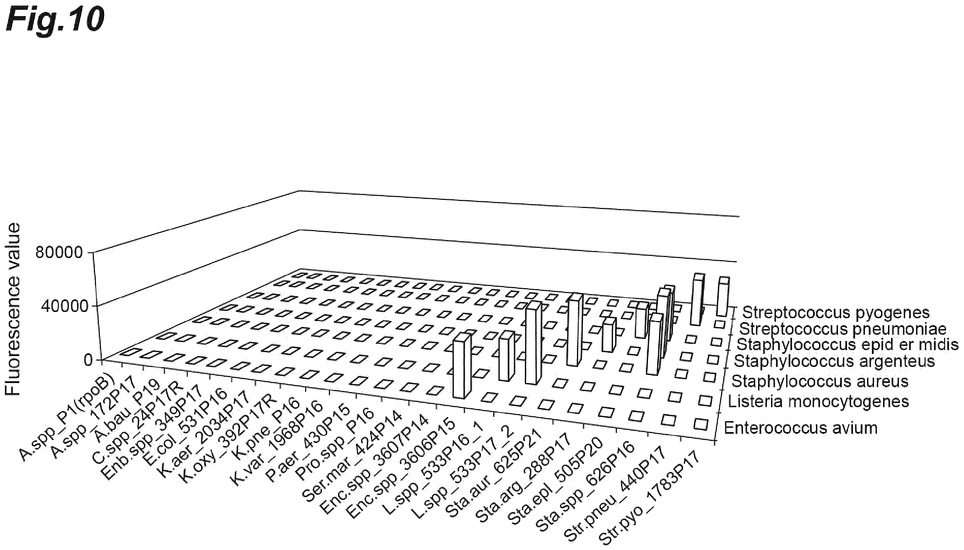

The reactivity and specificity of all probes to PCR products of individual bacterial species are evaluated and the evaluation results are shown in Table 19 and Figures 8 to 10. A cut-off value (fixed value: 3000 in all cases) was subtracted from the fluorescence values of individual probes, and then using the resultant values, graphs of Figures 8 to 10 were formed. A fluorescence signal from probes *E*. *coli*_531P16 was observed in samples of *Escherichia coli* but not observed in samples of the other bacterial species. From the result, the specific reaction to *Escherichia coli* was demonstrated. Accordingly, it was also demonstrated that, in the Multiplex system (multiplex assay system using all probes for Multiplex PCR), genomic DNA of *Escherichia coli* can be specifically detected by use of two primers (*E*. *coli_*468F19 and *E. coli_*567R18) for detection of *Escherichia coli* and a probe (*E. coli_*531P16). The probes corresponding to bacterial species other than *Escherichia coli* emitted fluorescence signals in response to PCR products of the respective bacterial genomes. The probes did not emit fluorescence signals in response to PCR products of the bacterial genomes to which they do not correspond. Accordingly, it was also demonstrated that, in the Multiplex system, primers and probes corresponding to individual bacterial species can function to specifically detect the bacterial species.

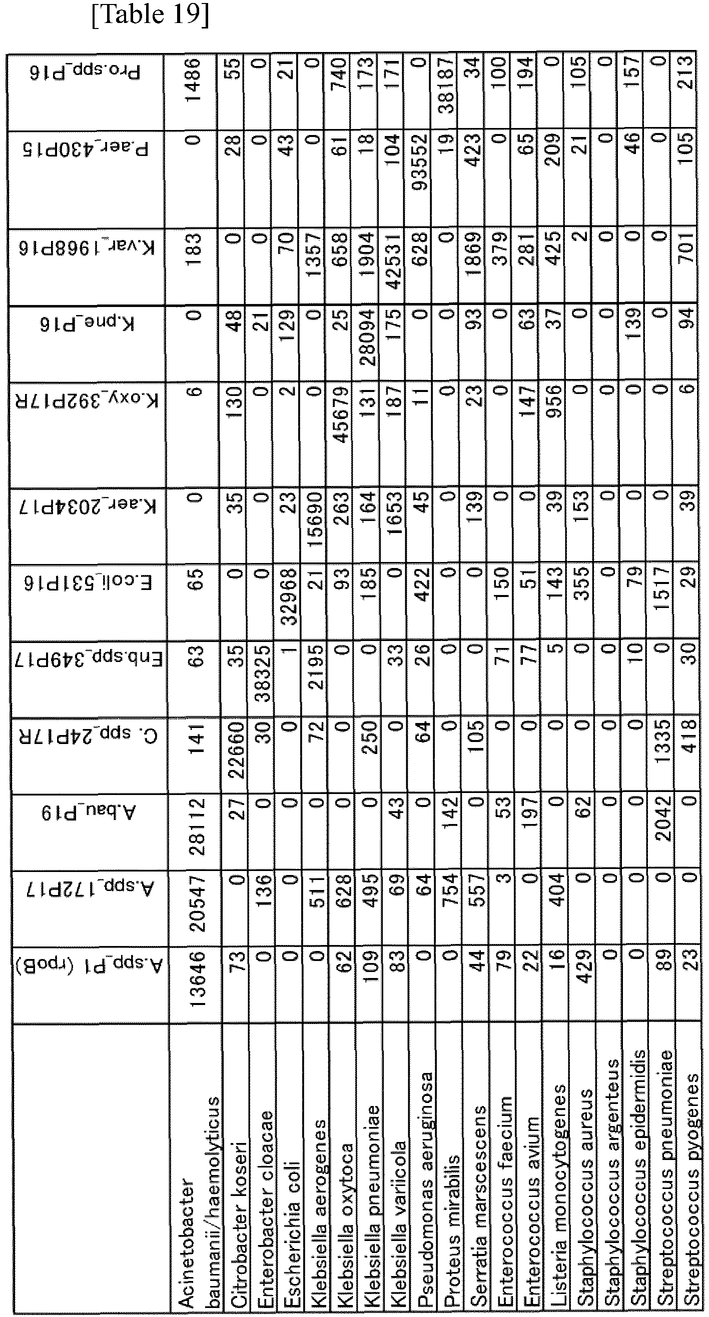

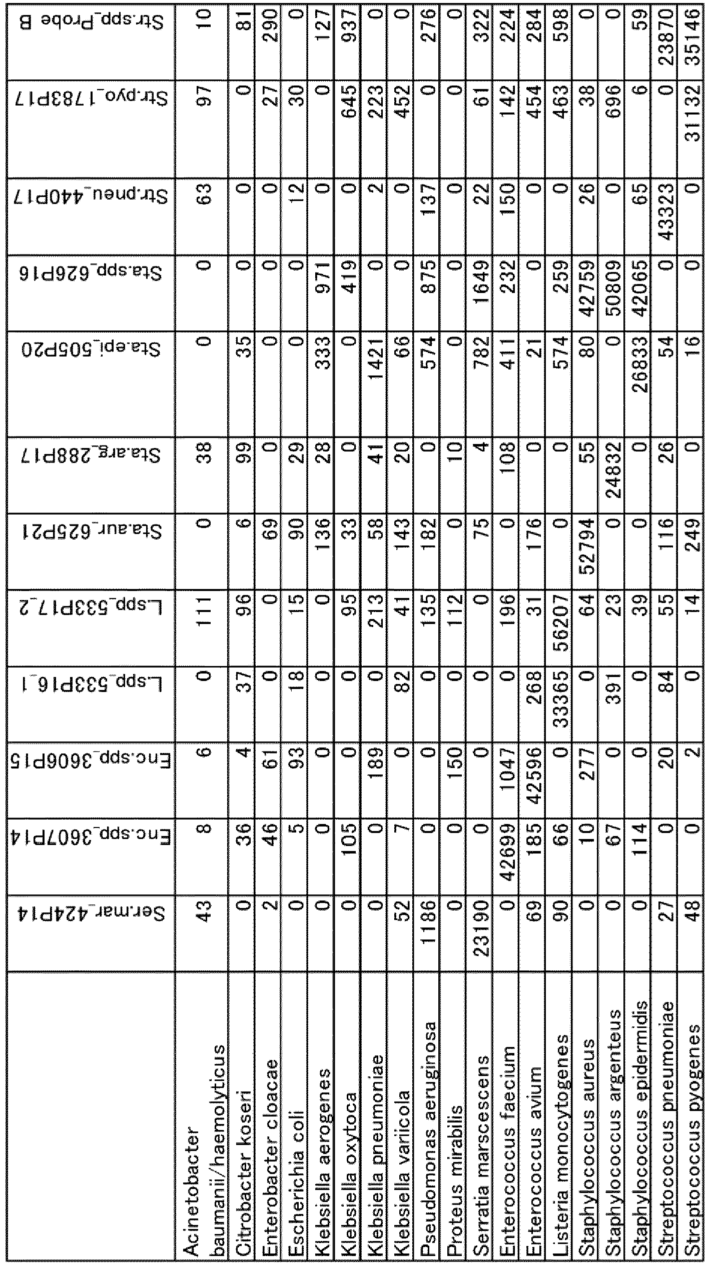

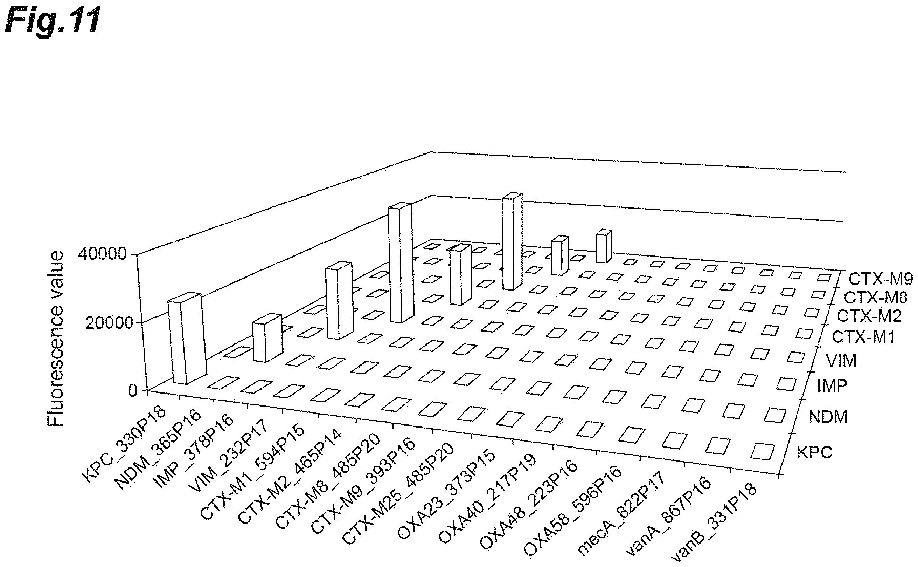

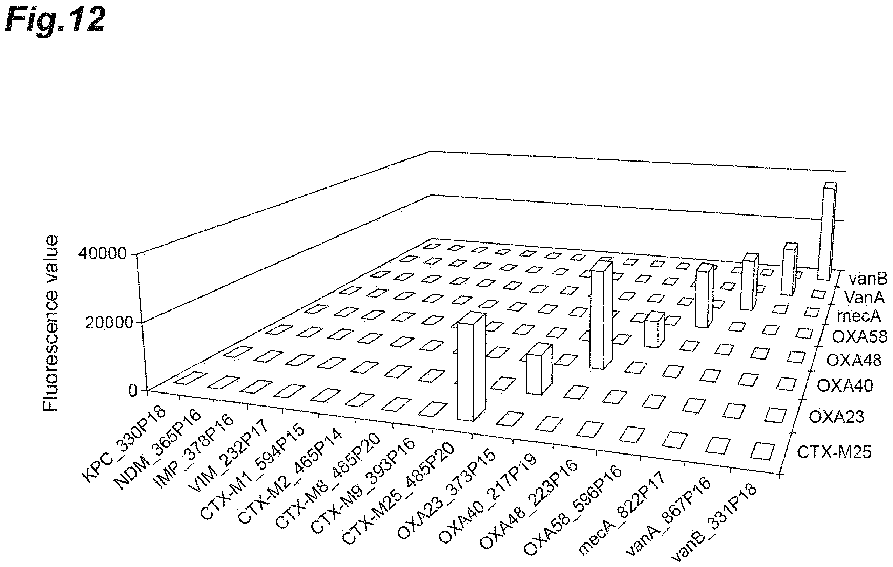

The evaluation results on the reactivity and specificity of all probes to PCR products of antimicrobial resistance genes are shown in Table 20, and Figures 11 and 12. A cut-off value (see Table 20) was subtracted from the fluorescence values of individual probes, and then using the obtained values, graphs of Figures 11 and 12 were formed. The probes for detecting antimicrobial resistance genes emitted fluorescence signals in response to the corresponding PCR products of antimicrobial resistance genes. No fluorescence signals were observed in response to the antimicrobial resistance genes to which the probes did not correspond. From this, it was also demonstrated that, in the Multiplex system, primers and probes corresponding to individual antimicrobial resistance genes can function to specifically detect the antimicrobial resistance genes.

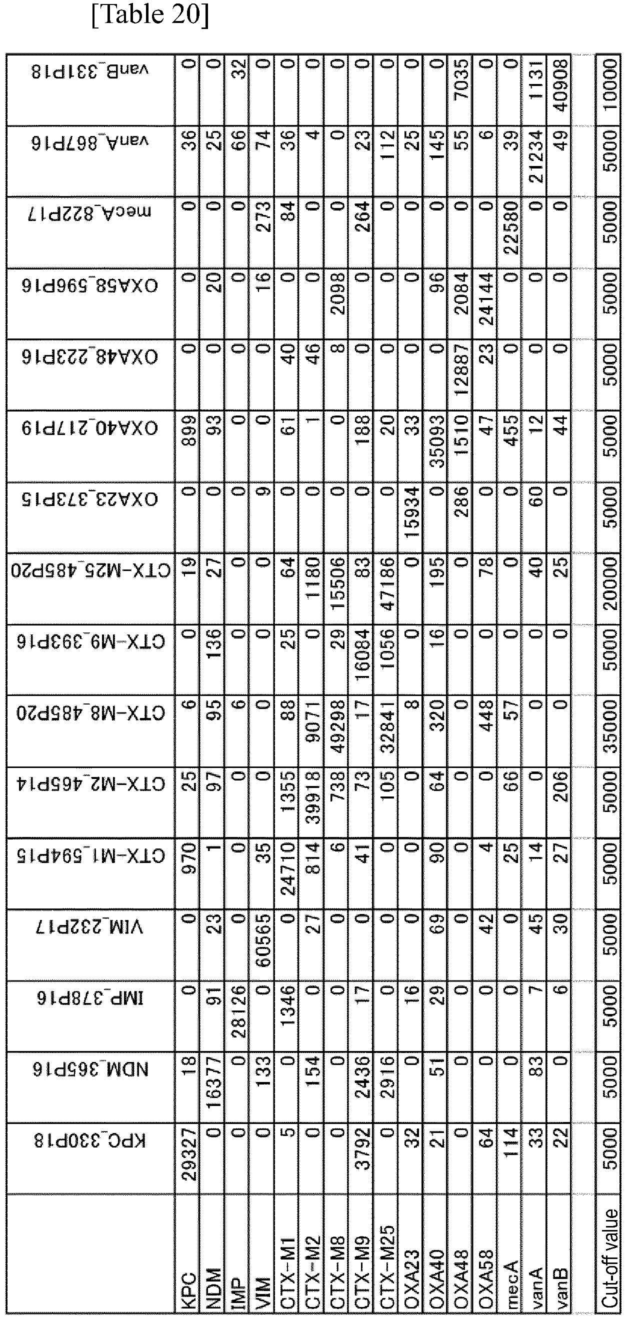

## Claims

1. A method for identifying causative bacteria of sepsis, the method comprising:
a step of performing a PCR method using a sample collected from a subject and a combination of sets of primers each specific to the full-length or a partial region of each of *gyrB* gene of *Escherichia coli, nuc* gene of *Staphylococcus aureus, atlE* gene of *Staphylococcus epidermidis, gyrB* gene of *Klebsiella pneumoniae* and *rpoB* gene of *Enterococcus* spp.; and
a step of detecting the presence or absence of amplification of the full-length or the partial region of each of the genes by using a combination of probes each specific to DNA of the full-length or the partial region.

2. The method according to claim 1, wherein
the set of primers specific to the full-length or a partial region of the *gyrB* gene of *Escherichia coli* comprises: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
the set of primers specific to the full-length or a partial region of the *nuc* gene of *Staphylococcus aureus* comprises: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
the set of primers specific to the full-length or a partial region of the *atlE* gene of *Staphylococcus epidermidis* comprises: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
the set of primers specific to the full-length or a partial region of the *gyrB* gene of *Klebsiella pneumoniae* comprises: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15,
the set of primers specific to the full-length or a partial region of the *rpoB* gene of *Enterococcus* spp. comprises: one or more primers selected from a primer containing the nucleotide sequence of SEQ ID NO: 26 or a nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29.

3. The method according to claim 1 or 2, wherein
the DNA of the full-length or the partial region of the *gyrB* gene of *Escherichia coli* comprises the nucleotide sequence of SEQ ID NO: 51 or a nucleotide sequence having a sequence identity of 90% or more with the nucleotide sequence of SEQ ID NO: 51,
the DNA of the full-length or the partial region of the *nuc* gene of *Staphylococcus aureus* comprises the nucleotide sequence of SEQ ID NO: 61 or a nucleotide sequence having a sequence identity of 90% or more with the nucleotide sequence of SEQ ID NO: 61,
the DNA of the full-length or the partial region of the *atlE* gene of *Staphylococcus epidermidis* comprises the nucleotide sequence of SEQ ID NO: 63 or a nucleotide sequence having a sequence identity of 90% or more with the nucleotide sequence of SEQ ID NO: 63,
the DNA of the full-length or the partial region of the *gyrB* gene of *Klebsiella pneumoniae* comprises the nucleotide sequence of SEQ ID NO: 53 or a nucleotide sequence having a sequence identity of 90% or more with the nucleotide sequence of SEQ ID NO: 53, and
the DNA of the full-length or the partial region of the *rpoB* gene of *Enterococcus* spp. comprises the nucleotide sequence of SEQ ID NO: 59 or a nucleotide sequence having a sequence identity of 90% or more with the nucleotide sequence of SEQ ID NO: 59.

4. The method according to claim 3, wherein the probes each hybridize with DNA of the full-length or the partial region under stringent conditions.

5. The method according to any one of claims 1 to 4, wherein
the probe specific to DNA of the full-length or the partial region of the *gyrB* gene of *Escherichia coli* comprises the nucleotide sequence of SEQ ID NO: 121 or a nucleotide sequence having a sequence identity of 90% or more with the nucleotide sequence of SEQ ID NO: 121,
the probe specific to DNA of the full-length or the partial region of the *nuc* gene of *Staphylococcus aureus* comprises the nucleotide sequence of SEQ ID NO: 134 or a nucleotide sequence having a sequence identity of 90% or more with the nucleotide sequence of SEQ ID NO: 134,
the probe specific to DNA of the full-length or the partial region of the *atlE* gene of *Staphylococcus epidermidis* comprises the nucleotide sequence of SEQ ID NO: 136 or a nucleotide sequence having a sequence identity of 90% or more with the nucleotide sequence of SEQ ID NO: 136,
the probe specific to DNA of the full-length or the partial region of the *gyrB* gene of *Klebsiella pneumoniae* comprises the nucleotide sequence of SEQ ID NO: 124 or a nucleotide sequence having a sequence identity of 90% or more with the nucleotide sequence of SEQ ID NO: 124, and
the probe specific to DNA of the full-length or the partial region of the *rpoB* gene of *Enterococcus* spp. is selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof.

6. The method according to any one of claims 1 to 5, wherein the probes contain different identifiers or are bound to the different identifiers on a solid support.

7. The method according to any one of claims 1 to 6, wherein the DNA amplified by the PCR method contains a label.

8. A kit for identifying causative bacteria of sepsis, the kit comprising:
a first reagent containing sets of primers for PCR each specific to the full-length or a partial region of each *of gyrB* gene of *Escherichia coli, nuc* gene of *Staphylococcus aureus, atlE* gene of *Staphylococcus epidermidis, gyrB* gene of *Klebsiella pneumoniae* and *rpoB* gene of *Enterococcus* spp.; and
a second reagent containing a combination of probes each specific to DNA of the full-length or the partial region of each of the genes.

9. The kit according to claim 8, wherein
the set of primers specific to the full-length or a partial region of the *gyrB* gene of *Escherichia coli* comprises: a primer containing the nucleotide sequence of SEQ ID NO: 10 or a nucleotide sequence having a sequence identity of 90% or more with the nucleotide sequence of SEQ ID NO: 10; and a primer containing the nucleotide sequence of SEQ ID NO: 11 or a nucleotide sequence having a sequence identity of 90% or more with the nucleotide sequence of SEQ ID NO: 11,
the set of primers specific to the full-length or a partial region of the *nuc* gene of *Staphylococcus aureus* comprises: one or more primers selected from a primer containing the nucleotide sequence of SEQ ID NO: 32 or a nucleotide sequence having a sequence identity of 90% or more with the nucleotide sequence of SEQ ID NO: 32, a primer containing the nucleotide sequence of SEQ ID NO: 34 or a nucleotide sequence having a sequence identity of 90% or more with the nucleotide sequence of SEQ ID NO: 34 and a combination thereof; and a primer containing the nucleotide sequence of SEQ ID NO: 33 or a nucleotide sequence having a sequence identity of 90% or more with the nucleotide sequence of SEQ ID NO: 33,
the set of primers specific to the full-length or a partial region of the *atlE* gene of *Staphylococcus epidermidis* comprises: a primer containing the nucleotide sequence of SEQ ID NO: 37 or a nucleotide sequence having a sequence identity of 90% or more with the nucleotide sequence of SEQ ID NO: 37; and a primer containing the nucleotide sequence of SEQ ID NO: 38 or a nucleotide sequence having a sequence identity of 90% or more with the nucleotide sequence of SEQ ID NO: 38,
the set of primers specific to the full-length or a partial region of the *gyrB* gene of *Klebsiella pneumoniae* comprises: a primer containing the nucleotide sequence of SEQ ID NO: 14 or a nucleotide sequence having a sequence identity of 90% or more with the nucleotide sequence of SEQ ID NO: 14; and a primer containing the nucleotide sequence of SEQ ID NO: 15 or a nucleotide sequence having a sequence identity of 90% or more with the nucleotide sequence of SEQ ID NO: 15, and
the set of primers specific to the full-length or a partial region of the *rpoB* gene of *Enterococcus* spp. comprises: one or more primers selected from a primer containing the nucleotide sequence of SEQ ID NO: 26 or a nucleotide sequence having a sequence identity of 90% or more with the nucleotide sequence of SEQ ID NO: 26, a primer containing the nucleotide sequence of SEQ ID NO: 27 or a nucleotide sequence having a sequence identity of 90% or more with the nucleotide sequence of SEQ ID NO: 27, a primer containing the nucleotide sequence of SEQ ID NO: 28 or a nucleotide sequence having a sequence identity of 90% or more with the nucleotide sequence of SEQ ID NO: 28 and a combination thereof; and a primer containing the nucleotide sequence of SEQ ID NO: 29 or a nucleotide sequence having a sequence identity of 90% or more with the nucleotide sequence of SEQ ID NO: 29.

10. The kit according to claim 8 or 9, wherein
the DNA of the full-length or the partial region of the *gyrB* gene of *Escherichia coli* comprises the nucleotide sequence of SEQ ID NO: 51 or a nucleotide sequence having a sequence identity of 90% or more with the nucleotide sequence of SEQ ID NO: 51,
the DNA of the full-length or the partial region of the *nuc* gene of *Staphylococcus aureus* comprises the nucleotide sequence of SEQ ID NO: 61 or a nucleotide sequence having a sequence identity of 90% or more with the nucleotide sequence of SEQ ID NO: 61,
the DNA of the full-length or the partial region of the *atlE* gene of *Staphylococcus epidermidis* comprises the nucleotide sequence of SEQ ID NO: 63 or a nucleotide sequence having a sequence identity of 90% or more with the nucleotide sequence of SEQ ID NO: 63,
the DNA of the full-length or the partial region of the *gyrB* gene of *Klebsiella pneumoniae* comprises the nucleotide sequence of SEQ ID NO: 53 or a nucleotide sequence having a sequence identity of 90% or more with the nucleotide sequence of SEQ ID NO: 53, and
the DNA of the full-length or the partial region of the *rpoB* gene of *Enterococcus* spp. comprises the nucleotide sequence of SEQ ID NO: 59 or a nucleotide sequence having a sequence identity of 90% or more with the nucleotide sequence of SEQ ID NO: 59.

11. The kit according to claim 10, wherein the probes each hybridize with DNA of the full-length or the partial region under stringent conditions.

12. The kit according to any one of claims 8 to 11, wherein
the probe specific to DNA of the full-length or the partial region of the *gyrB* gene of *Escherichia coli* comprises the nucleotide sequence of SEQ ID NO: 121 or a nucleotide sequence having a sequence identity of 90% or more with the nucleotide sequence of SEQ ID NO: 121,
the probe specific to DNA of the full-length or the partial region of the *nuc* gene of *Staphylococcus aureus* comprises the nucleotide sequence of SEQ ID NO: 134 or a nucleotide sequence having a sequence identity of 90% or more with the nucleotide sequence of SEQ ID NO: 134,
the probe specific to DNA of the full-length or the partial region of the *atlE* gene of *Staphylococcus epidermidis* comprises the nucleotide sequence of SEQ ID NO: 136 or a nucleotide sequence having a sequence identity of 90% or more with the nucleotide sequence of SEQ ID NO: 136,
the probe specific to DNA of the full-length or the partial region of the *gyrB* gene of *Klebsiella pneumoniae* comprises the nucleotide sequence of SEQ ID NO: 124 or a nucleotide sequence having a sequence identity of 90% or more with the nucleotide sequence of SEQ ID NO: 124, and
the probe specific to DNA of the full-length or the partial region of the *rpoB* gene of *Enterococcus* spp. is selected from a probe containing the nucleotide sequence of SEQ ID NO: 129 or a nucleotide sequence having a sequence identity of 90% or more with the nucleotide sequence of SEQ ID NO: 129, a probe containing the nucleotide sequence of SEQ ID NO: 130 or a nucleotide sequence having a sequence identity of 90% or more with the nucleotide sequence of SEQ ID NO: 130, a probe containing the nucleotide sequence of SEQ ID NO: 131 or a nucleotide sequence having a sequence identity of 90% or more with the nucleotide sequence of SEQ ID NO: 131 and a combination thereof.

13. The kit according to any one of claims 8 to 12, wherein the probes contained in the second reagent contain different identifiers or are bound to the different identifiers on a solid support.

14. The kit according to any one of claims 8 to 13, wherein the sets of primers are designed such that DNAs to be amplified by the PCR method using the sets of primers contain labels or the kit further comprises a third reagent for labeling the DNAs to be amplified.
